(19) Europäisches Patentamt / European Patent Office / Office européen des brevets

(11) **EP 4 578 939 A1**

(12) **EUROPEAN PATENT APPLICATION**
published in accordance with Art. 153(4) EPC

(43) Date of publication:
**02.07.2025 Bulletin 2025/27**

(21) Application number: **23854502.4**

(22) Date of filing: **17.08.2023**

(51) International Patent Classification (IPC):
**C12N 1/20** (2006.01)   **C12R 1/32** (2006.01)

(52) Cooperative Patent Classification (CPC):
**C12N 1/20;** C12R 2001/32

(86) International application number:
**PCT/CN2023/113637**

(87) International publication number:
**WO 2024/037604 (22.02.2024 Gazette 2024/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.08.2022 CN 202210989249**

(71) Applicant: **Peng, Guanghao**
**Nanjing, Jiangsu 210008 (CN)**

(72) Inventor: **Peng, Guanghao**
**Nanjing, Jiangsu 210008 (CN)**

(74) Representative: **Petraz, Gilberto Luigi et al**
**GLP S.r.l.**
**Viale Europa Unita, 171**
**33100 Udine (IT)**

Remarks:
The complete document including Reference Table(s) and the Sequence Listing(s) can be downloaded from the EPO website

(54) **MYCOBACTERIUM SP. BASED ON VARIOUS CARBON-NITROGEN METABOLIC PATHWAYS, METHOD FOR ISOLATING SAME, AND USE THEREOF**

(57)     Provided are a Mycobacterium sp., a method for isolating same, and use thereof. The preservation number of the Mycobacterium sp. is CGMCC No. 21272 or CGMCC No. 21273. The Mycobacteria sp. has significant nitrogen fixation, carbon fixation, and ammonia oxidation capacity, can directly oxidize ammonia nitrogen into nitrogen, and has an antagonistic effect on other microorganisms.

1: orf00266
2: orf00793
3: orf01696
4: orf04825
5: orf05051
6: orf04171

FIG. 6

## Description

### CROSS-REFERENCE TO RELATED APPLICATIONS

The application claims the benefit of the China patent application No. 202210989249.4, filed on August 17, 2022, the content of which is specifically and entirely incorporated herein by reference.

### TECHNICAL FIELD

The present invention relates to the technical field of wastewater treatment used in the fermentation industry, in particular to a *Mycobacterium sp.* based on various carbon-nitrogen metabolic pathways, a method for isolating same, and a use thereof.

### BACKGROUND

Nitrification in nature is generally considered to be accomplished by two types of nitrifying bacteria. The first stage is the oxidation of ammonia to nitrite by nitrifying bacteria, and the second stage is the oxidation of nitrite to nitrate by denitrifying bacteria. Research in 2015 proposed that the nitrification could be accomplished by bacteria single bacterium. Nitrifying bacteria have many applications in the aspects such as treatment of nitrogen-containing waste water and screening of the nitrification inhibitors, due to the characteristics of said nitrifying bacteria. However, nitrifying bacteria are considered in the prior art to be "chemoautotrophic" type bacteria, and the presence of organic matter is detrimental to the growth of nitrifying bacteria. Based on the awareness, the use of nitrifying bacteria suffers from significant limitations. For example, based on the awareness that nitrifying bacteria are chemoautotrophic, the microorganism base and process principle of the existing waste water biochemical treatment technology for removing pollutants containing C and N reside in a combination of the modes of "ammonification" reaction for removing carbon performed by heterotrophic bacteria, ammoxidation "nitrification" reaction with nitrous acid oxidation performed by chemoautotrophic bacteria, "denitrification" reaction for removing nitrogen performed by anaerobic (facultative) heterotrophic bacteria. However, the aforementioned combinations have the defects such as slow propagation rate of colony, low total nitrogen removal rate.

*Mycobacterium* is a slender and slightly curved bacterium, which is sometimes branched or exhibits protonema. The major characteristics include the large amount of lipids (mainly mycolic acids) contained in the cell wall, atrichosis, non-budding, do not generate endotoxin and exotoxins, are aerobic saprophytic bacteria, exhibit surface growth, and strong attachment to solid surfaces. Most of the research on *Mycobacterium* has been focused on the pathogenic bacteria: *Mycobacterium tuberculosis* and *Mycobacterium leprae.* There is no report with respect to nitrification activity of *Mycobacterium.*

Furthermore, there are limitations in existing methods for isolating nitrifying strains. For example, Chinese patent ZL 03118598.3 (Method for separating, identifying, purifying, heterotrophying and nitrifying microbe) discloses a method for separating and identifying heterotrophic microorganism with nitrification activity from different soils and selective cultivation process by means of nutrient agar plate separation and Griess reagent development. Although the method is general-purpose to screen many heterotrophic strains having nitrifying activity, it lacks specificity, and it is difficult to target a strain having high nitrifying activity.

### SUMMARY OF THE INVENTION

The present invention aims to overcome the defects in the prior art, and provides a strain of *Mycobacterium sp.* based on various carbon-nitrogen metabolic pathways, a method for isolating same and a use thereof. The strain or microbial agent of the present invention has desirable application prospect in the sewage treatment, screening of the nitrification inhibitors, and other aspects.

In order to achieve the above aims, the first aspect of the present invention provides a *Mycobacterium sp.,* and the deposit number of the *Mycobacterium sp.* is CGMCC No.21272 or CGMCC No. 21273.

The second aspect of the present invention provides a microbial agent comprising the aforementioned *Mycobacterium sp.*

The other aspects of the invention relate to the use of the *Mycobacterium sp.* or microbial agent.

The invention also provides a method for isolating the microorganism with nitrification activity.

The *Mycobacterium sp.* of the present invention, unlike the existing *Mycobacterium sp.,* has at least the following characteristics: 1) evident nitrogen fixation capacity, 2) strong ammoxidation, significant nitrification activity, ability to be heterotrophic, the removal of C (carbon capture) and N organic pollutant can be implemented without the need for coordination with other microorganisms, 3) capability to dehydrogenate hydrazine sulfate to yield hydroxylamine, 4) presence of at least five kinds of dehydrogenases, 5) presence of nitric oxide synthase gene, ability to oxidize arginine, wherein Fe is an important element affecting its nitrification activity, and 6) significant antagonistic effect on the other microorganisms.

The isolating method of the present invention enables to stably and specifically separate a strain with high nitrification activity from a sample (e.g., soil) with desirable repeatability.

Biological deposit

The *Mycobacterium sp.* provided by the present invention, has been deposited on December 1, 2020 in China General Microbiological Culture Collection Center (Address: No. 3, Courtyard 1, Beichen West Road, Chaoyang District, Beijing, China, Institute of Microbiology of the Chinese Academy of Sciences, Zip Code: 100101), with the abbreviation of the depositary institution unit as CGMCC, and the deposit number of the strain is CGMCC No.21272, abbreviated as YT.

The *Mycobacterium sp.* provided by the present invention, has been deposited on December 1, 2020 in China General Microbiological Culture Collection Center (Address: No. 3, Courtyard 1, Beichen West Road, Chaoyang District, Beijing, China, Institute of Microbiology of the Chinese Academy of Sciences, Zip Code: 100101), with the abbreviation of the depositary institution unit as CGMCC, and the deposit number of the strain is CGMCC No.21273, abbreviated as JD.

## BRIEF DESCRIPTION OF THE DRAWINGS

FIGS. 1a-1b illustrate the whole genome circle diagrams *of Mycobacterium sp.* of the invention;

FIGS. 1c-1d illustrate schematic diagrams of nitrogen metabolism pathways;

FIG. 2a illustrates the emergence of bacteria of LB-malachite green plates at a $10^{-7}$ dilution gradient.

FIG. 2b shows a transmission electron micrograph of target bacteria;

FIGS. 2c-2e illustrate an image of Griess reagent color development;

FIG. 2f illustrates a graph showing the distribution of target bacteria on the separation plate;

FIG. 3a is a photograph *of Mycobacterium sp.* of the invention cultured in a liquid medium;

FIGS. 3b-3c are photographs *of Mycobacterium sp.* of the invention cultured on a solid medium;

FIG. 3d shows the pattern of *Mycobacterium sp.* of the invention under Nikon's stereoscope;

FIG. 3e is a photograph of *Mycobacterium sp.* of the invention cultured on a solid medium;

FIG. 3f illustrates the result of PCR amplification of the nitrogenase ferritin gene;

FIG. 3g is a graph showing the changes of culture $OD_{600}$ and NH4$^+$-N concentrations during the cultivation process of *Mycobacterium sp.* by using ammonium sulfate as the nitrogen source and using pyruvic acid as the carbon source;

FIG.4a is a graph showing the changes of culture $OD_{600}$ and pH during the cultivation process;

FIG.4b is a graph showing the change in pyruvic acid concentration during the cultivation process;

FIG. 5 illustrates a photograph of the culture of *Mycobacterium sp.* of the invention in a medium containing hydrazine sulfate as the nitrogen source;

FIG. 6 shows a graph of transcriptome sequencing results for YT culture derived by using hydroxylamine as the nitrogen source;

FIG. 7a is a photograph illustrating the initial stages of the strain JD in pure culture by using the trans-sodium hyponitrite as the nitrogen source and the ammonium sulfate as the nitrogen source;

FIG. 7b shows the pattern of *Mycobacterium sp.* of the invention under Nikon's stereoscope;

FIG. 7c is a graph showing the results of a qualitative assay for the nitrification activity of a culture by using the Sodium trans-hyponitrite as the nitrogen source;

FIG. 8 is a photograph *of Mycobacterium sp.* of the invention cultured in iron-free liquid medium;

FIG. 9a is a graph comparing the pure culture of YT for 13 days with a control group;

FIG. 9b is a graph showing the NOS gene amplification *of Mycobacterium sp.* of the invention;

FIG. 10a is a graph showing the changes of culture $OD_{600}$ and pH during the cultivation process of *Mycobacterium sp.* by using sodium nitrite as the nitrogen source;

FIG. 10b is a graph showing the change in total nitrogen concentration of cultures during the cultivation process *of Mycobacterium sp.* by using sodium nitrite as the nitrogen source;

FIG. 11 illustrates a graph showing the change of $NO_2^-$-N after growth of strain JD for 6 days in anaerobic culture using sodium nitrite as the nitrogen source, and a photograph showing the culture;

FIG. 12a is a graph showing the changes of culture $OD_{600}$ and pH of strain JD during the cultivation process;

FIG. 12b is a graph showing the results of a qualitative assay for $NO_2^-$-N of the JD culture after cultivation for 11 days;

FIG. 12c is a graph illustrating the changes in $CO_2$ and $O_2$ concentrations in the JD culture system over time;

FIG. 12d is a graph illustrating the changes in $N_2$ and $N_2O$ concentrations in the JD culture system over time;

FIGS. 13a-13b show the influence of various nitrification inhibitors on YT growth;

FIG. 13c illustrates a graph comparing TN concentrations in cultures in which YT are cultured by using different nitrification inhibitors as the nitrogen source;

FIG. 14a is a graph showing the antagonistic effect of YT on other strains;

FIG. 14b shows the inhibition zones formed by the antagonistic effect of YT on three standard strains and an electron micrograph thereof;

FIG. 14c illustrates the inhibition zones formed by the antagonistic effect of YT on three standard strains and the pattern of YT under stereoscope.

## DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

In the present invention, "mM" refers to "mmol/L", "M" refers to "mol/L"; "ammonia nitrogen" is used interchangeably with "ammonium nitrogen".

### I. Strain and microbial agent

The first aspect of the present invention provides a strain *of Mycobacterium sp.,* characterized in that deposit number of the *Mycobacterium sp.* is CGMCC No.21272 or CGMCC No. 21273.

The present inventors have further verified through experiments that the strain CGMCC No. 21272 (abbreviated YT) or CGMCC No. 21273 (abbreviated JD) of the present invention are different from any of the existing *Mycobacterium sp.* , thus the strain of the present invention is a novel species of *Mycobacterium sp.,* which is named as *Mycobacterium guanghaopengii* sp. *nov.*

According to the invention, the cell morphology and physicochemical characteristics of *Mycobacterium sp.* with the deposit number CGMCC No.21272 are as shown in Table la.

Table la

| Experiment items | Results | Experiment items | Results | Experiment items | Results | Experiment items | Results |
|---|---|---|---|---|---|---|---|
| Cellular morphology | Rod-shape | Gram stain | Positive | Ligase | + | Oxidase | - |
| **BIOLOG GEN III** (growth experiments) | | | | | | | |
| Negative control | - | $\alpha$-D-glucose | + | Gelatin | - | D-raffinose | - |
| Glucuronamide | + | D-mannose | + | D-serine | - | $\alpha$-D-lactose | - |
| D-glucose-6-phosphoric acid | - | D-fructose | + | L-alanine | - | D-melibiose | + |
| D-fructose-6- phosphoric acid | + | D-galactose | + | L-arginine | + | L-lactic acid | - |
| D-glucuronic acid | + | Dextrin | - | L-histidine | - | Citric acid | - |
| p- hydroxyphenylacetic acid | - | D-fucose | + | L-pyroglutamic acid | - | $\alpha$-ketoglutarate | + |
| D- aspartic acid | - | L-fucose | + | L-serine | - | D-malic acid | - |
| D-methyl lactate | - | L-rhamnose | + | Pectin | - | L-malic acid | + |
| $\beta$-methyl-D-glucoside | - | Inosine | - | Sucrose | + | Bromosuccinic acid | + |
| $\beta$-hydroxy-D,L-butyric acid | + | D-sorbitol | - | D-gluconic acid | + | Tween 40 | + |
| N-acetyl-D-glucosamine | - | D-mannitol | + | Stachyose | - | $\gamma$-aminobutyric acid | - |

(continued)

| BIOLOG GEN III (growth experiments) | | | | | | |
|---|---|---|---|---|---|---|
| N-acetyl-β-D-manno-samine | - | D-arabitol | - | Mucic acid | - | A-hydroxybutyratic acid | - |
| N-acetyl-D-galacto-samine | - | Inositol | + | Quinic acid | - | Propionic acid | + |
| N-acetylneuraminic acid | - | Glycerol | + | Saccharic acid | + | Acetic acid | + |
| L-aspartic acid | - | D-maltose | - | D-salicin | - | Formic acid | + |
| L-glutamic acid | + | D-trehalose | + | α-ketobutyric acid | - | | |

| BIOLOG GEN II (chemically sensitive experiments; +, insensitive; -, sensitive) | | | | | | |
|---|---|---|---|---|---|---|
| Positive control | + | Potassium tellurite | + | pH 6.0 | + | Nalidixic acid | + |
| 1 % Sodium lactate | + | D-serine | + | pH 5.0 | - | Lithium chloride | + |
| Rifamycin SV | + | Guanidine hydrochloride | - | 1%NaCl | + | Aztreonam | + |
| Minocycline | + | Tetrazolium violet | + | 4%NaCl | + | Sodium butyrate | + |
| Vancomycin | + | Tetrazolium blue | - | 8%NaCl | + | Sodium Bro-mate | + |
| Sodium tetradecyl sulfate | + | | | | | | |

In the present invention, the 16S rRNA sequence of *Mycobacterium sp.* with deposit number of CGMCC No.21272 is shown as SEQ ID NO: 1.

SEQ ID NO:1:

```
CGGCTCCCTCCCACAAGGGGTTAGGCCACCGGCTTCGGGTGTTACCGACTTTCATGACGTGACGGGCGGTGTGTACAAGGCCC
GGGAACGTATTCACCGCAGCGTTGCTGATCTGCGATTACTAGCGACTCCGACTTCACGGGGTCGAGTTGCAGACCCCGATCCG
AACTGAGACCGGCTTTGAAAGGATTCGCTCCACCTCACGGCATCGCAGCCCTTTGTACCGGCCATTGTAGCATGTGTGAAGCC
CTGGACATAAGGGGCATGATGACTTGACGTCATCCCCACCTTCCTCCGAGTTGACCCCGGCAGTCTCTCACGAGTCCCCGCCA
TTACGCGCTGGCAACATAAGATAAGGGGTTGCGCTCGTTGCGGGACTTAACCCAACATCTCACGACACGAGCTGACGACAGCCA
TGCACCACCTGCACACAGGCCACAAGGGAATACCTATCTCTAGGCACGTCCTGTGCATGTCAAACCCAGGTAAGGTTCTTCGC
GTTGCATCGAATTAATCCACATGCTCCGCCGCTTGTGCGGGCCCCCGTCAATTTCTTTGAGTTTTAGCCTTGCGGCCGTACTC
CCCAGGCGGGTACTTAATGCGTTAGCTACGGCACGGATCCCAAGGAAGGAAACCCACACCTAGTACCCACCGTTTACGGCGT
GGACTACCAGGGTATCTAATCCTGTTCGCTCCCCACGCTTTCGCTCCTCAGCGTCAGTTACTGCCCAGAGACCCGCCTTCGCC
ACCGGTGTTCCTCCTGATATCTGCGCATTCCACCGCTACACCAGGAATTCCAGTCTCCCCTGCAGTACTCCAGTCTGCCCGTA
TCGCCCGCACGCCCACAGTTGAGCTGTGAGTTTTCACGAACAACGCGACAAACCACCTACGAGCTCTTTACGCCCAGTAATTC
CGGACAACGCTCGGACCCTACGTATTACCGCGGCTGCTGGCACGTAGTTGGCCGGTCCTTCTTCTCCAGGTACCGTCACTTGC
GCTTCGTCCCTGGCGAAAGAGGTTTACAACCCGAAGGCCGTCATCCCTCACGCGGCGTCGCTGCATCAGGCTTGCGCCCATTG
TGCAATATTCCCCACTGCTGCCTCCCGTAGGAGTCTGGGCCGTATCTCAGTCCCAGTGTGGCCGGTCACCCTCTCAGGCCGGC
TACCCGTCGTCGCCTTGGTGAGCCGTAACCTCACCAACAAGCTGATAGGCCGCGGGCCCATCCCACACCGCAAAAGCTTTCCC
CAACCAGACCATGCAGCCAGAAGGGTGTATTCGGTATTAGACCCAGTTTCCCAGGCTTATCCCAAAGTGCAGGGCAGATCACC
CACGTGTTACTCACCCGTTCGCCACTCGAGTACCCCGAAGGGCCTTTCCGTTCGACTGCATG
```

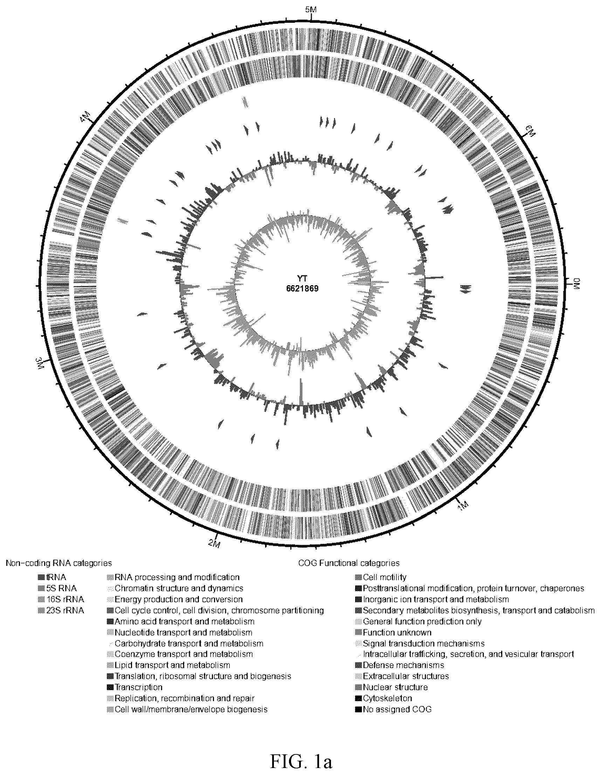

In the invention, the results of phylogenetic analysis based on the 16S rRNA gene sequence show that *Mycobacterium sp.* with deposit number of CGMCC No.21272 is a *Mycobacterium* genus strain, whose whole genome circle diagram is shown in FIG. 1a.

In the invention, the results of antibiotic susceptibility experiment *of Mycobacterium sp.* with deposit number of CGMCC No.21272 are shown in Table 1b.

Table 1b

| Antibiotic | Susceptibility degree | Antibiotic | Susceptibility degree | Antibiotic | Susceptibility degree |
|---|---|---|---|---|---|
| Fleroxacin | R | Tobramycin | S | Rifampin | R |
| Romifloxacin | S | Vancomycin | S | Ceftriaxone | R |
| Ciprofloxacin | S | Netilmicin | S | Cotrimoxazole | S |
| Penicillin | R | Tetracycline | S | Cefuroxime | R |
| Erythromycin | S | Cefaclor | R | Minocycline | S |
| Chloramphenicol | S | Cefazolin | R | Cephalothin | R |
| Azithromycin | S | Cefotaxime | R | Cefoperazone | R |
| Clindamycin | R | Ampicillin | R | Piperacillin | R |
| Doxycycline | S | Amikacin | S | Benzazolin | R |
| Clarithromycin | S | Ceftazidime | R | Furantoin | R |

Explanations: S: sensitive; M: moderately sensitive; R: resistant.

In the present invention, the cell morphology and physicochemical characteristics of *Mycobacterium sp.* with deposit number of CGMCC No.21273 are as shown in Table 2a.

Table 2a

| Experiment items | Results | Experiment items | Results | Experiment items | Results | Experiment items | Results |
|---|---|---|---|---|---|---|---|
| Cellular morphology | Rod-shape | Gram stain | Positive | Ligase | + | Oxidase | - |
| **BIOLOG GEN III** (growth experiments) | | | | | | | |
| Negative control | - | α-D-glucose | + | Gelatin | - | D-raffinose | - |
| Glucuronamide | + | D-mannose | + | D-serine | - | α-D-lactose | - |
| D-glucose-6-phosphoric acid | - | D-fructose | + | L-alanine | - | D-melibiose | + |
| D-fructose-6- phosphoric acid | + | D-galactose | + | L-arginine | + | L-lactic acid | - |
| D-glucuronic acid | + | Dextrin | - | L-histidine | - | Citric acid | - |
| p- hydroxyphenylacetic acid | - | D-fucose | + | L-pyroglutamic acid | - | α-ketoglutarate | + |
| D- aspartic acid | - | L-fucose | + | L-serine | - | D-malic acid | - |
| D-methyl lactate | - | L-rhamnose | + | Pectin | - | L-malic acid | + |
| β-methyl-D-glucoside | - | Inosine | - | Sucrose | + | Bromosuccinic acid | + |
| β-hydroxy-D,L-butyric acid | + | D-sorbitol | - | D-gluconic acid | + | Tween 40 | + |
| N-acetyl-D-glucosamine | - | D-mannitol | + | Stachyose | - | γ-aminobutyric acid | - |
| N-acetyl-β-D-mannosami ne | - | D-arabitol | - | Mucic acid | - | A-hydroxybutyr atic acid | - |
| N-acetyl-D-galactosamin e | - | Inositol | + | Quinic acid | - | Propionic acid | + |

(continued)

| BIOLOG GEN III (growth experiments) | | | | | | | |
| --- | --- | --- | --- | --- | --- | --- | --- |
| N-acetylneuraminic acid | - | Glycerol | + | Saccharic acid | + | Acetic acid | + |
| L-aspartic acid | - | D-maltose | - | D-salicin | - | Formic acid | + |
| L-glutamic acid | + | D-trehalose | + | α-ketobutyric acid | - | | |
| BIOLOG GEN II (chemically sensitive experiments; +, insensitive; -, sensitive) | | | | | | | |
| Positive control | + | Potassium tellurite | + | pH 6.0 | + | Nalidixic acid | + |
| 1 % Sodium lactate | + | D-serine | + | pH 5.0 | - | Lithium chloride | + |
| Rifamycin SV | + | Guanidine hydrochloride | - | 1%NaCl | + | Aztreonam | + |
| Minocycline | + | Tetrazolium violet | + | 4%NaCl | + | Sodium butyrate | + |
| Vancomycin | + | Tetrazolium blue | - | 8%NaCl | + | Sodium Bro-mate | + |
| Sodium tetradecyl sulfate | + | | | | | | |

In the invention, the 16S rRNA sequence of *Mycobacterium sp.* with deposit number of CGMCC No.21273 is shown as SEQ ID NO: 2.

SEQ ID NO:2:

```
GGCTCCCTCCCACAAGGGGTTAGGCCACCGGCTTCGGGTGTTACCGACTTTCATGACGTGACGGGCGGTGTGTACAAGGCCCG
GGAACGTATTCACCGCAGCGTTGCTGATCTGCGATTACTAGCGACTCCGACTTCACGGGGTCGAGTTGCAGACCCCGATCCGA
ACTGAGACCGGCTTTGAAAGGATTCGCTCCACCTCACGGCATCGCAGCCCTTTGTACCGGCCATTGTAGCATGTGTGAAGCCC
TGGACATAAGGGGCATGATGACTTGACGTCATCCCCACCTTCCTCCGAGTTGACCCCGGCAGTCTCTCACGAGTCCCCGCCAT
TACGCGCTGGCAACATAAGATAAGGGTTGCGCTCGTTGCGGGACTTAACCCAACATCTCACGACACGAGCTGACGACAGCCAT
GCACCACCTGCACACAGGCCACAAGGGAATACCTATCTCTAGGCACGTCCTGTGCATGTCAAACCCAGGTAAGGTTCTTCGCG
TTGCATCGAATTAATCCACATGCTCCGCCGCTTGTGCGGGCCCCCGTCAATTTCTTTGAGTTTTAGCCTTGCGGCCGTACTCC
CCAGGCGGGGTACTTAATGCGTTAGCTACGGCACGGATCCCAAGGAAGGAAACCCACACCTAGTACCCACCGTTTACGGCGTG
GACTACCAGGGTATCTAATCCTGTTCGCTCCCCACGCTTTCGCTCCTCAGCGTCAGTTACTGCCCAGAGACCCGCCTTCGCCA
CCGGTGTTCCTCCTGATATCTGCGCATTCCACCGCTACACCAGGAATTCCAGTCTCCCCTGCAGTACTCCAGTCTGCCCGTAT
CGCCCGCACGCCCACAGTTGAGCTGTGAGTTTTCACGAACAACGCGACAAACCACCTACGAGCTCTTTACGCCCAGTAATTCC
GGACAACGCTCGGACCCTACGTATTACCGCGGCTGCTGGCACGTAGTTGGCCGGTCCTTCTTCTCCAGGTACCGTCACTTGCG
CTTCGTCCCTGGCGAAAGAGGTTTACAACCCGAAGGCCGTCATCCCTCACGCGGCGTCGCTGCATCAGGCTTGCGCCCATTGT
GCAATATTCCCCACTGCTGCCTCCCGTAGGAGTCTGGGCCGTATCTCAGTCCCAGTGTGGCCGGTCACCCTCTCAGGCCGGCT
ACCCGTCGTCGCCTTGGTGAGCCGTAACCTCACCAACAAGCTGATAGGCCGCGGGCCCATCCCACACCGCAAAAGCTTTCCCC
AACCAGACCATGCAGCCAGAAGGGTGTATTCGGTATTAGACCCAGTTTCCCAGGCTTATCCCAAAGTGCAGGGCAGATCACCC
ACGTGTTACTCACCCGTTCGCCACTCGAGTACCCCGAAGGGCCTTTCCGTTCGACTTGCA
```

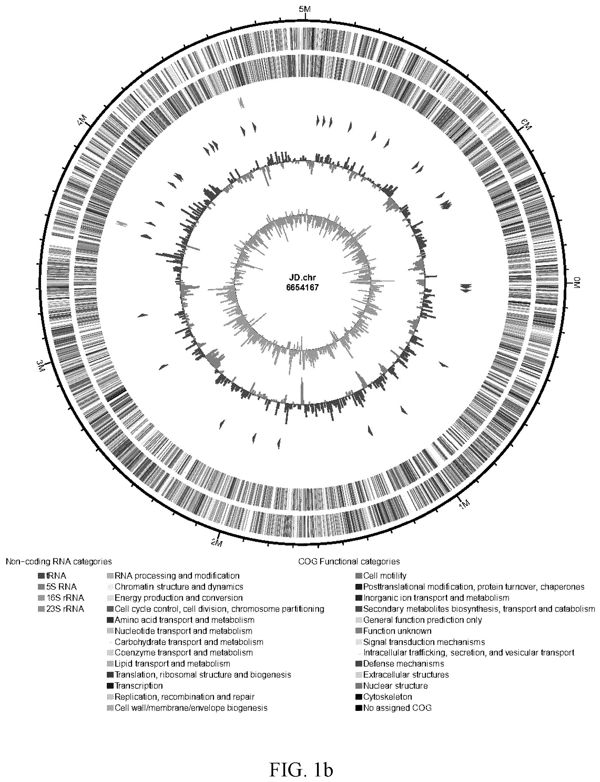

In the present invention, the results of phylogenetic analysis based on the 16S rRNA gene sequence show that *Mycobacterium sp.* with deposit number of CGMCC No.21273 is a *Mycobacterium* genus strain, whose whole genome circle diagram is shown in FIG. 1b.

In the invention, the results of antibiotic susceptibility experiment *of Mycobacterium sp.* with deposit number of CGMCC No.21273 are shown in Table 2b.

Table 2b

| Antibiotic | Susceptibility degree | Antibiotic | Susceptibility degree | Antibiotic | Susceptibility degree |
| --- | --- | --- | --- | --- | --- |
| Fleroxacin | M | Tobramycin | S | Rifampin | R |
| Romifloxacin | S | Vancomycin | S | Ceftriaxone | R |

(continued)

| Antibiotic | Susceptibility degree | Antibiotic | Susceptibility degree | Antibiotic | Susceptibility degree |
|---|---|---|---|---|---|
| Ciprofloxacin | S | Netilmicin | S | Cotrimoxazole | S |
| Penicillin | R | Tetracycline | S | Cefuroxime | R |
| Erythromycin | S | Cefaclor | R | Minocycline | S |
| Chloramphenicol | S | Cefazolin | R | Cephalothin | R |
| Azithromycin | S | Cefotaxime | M | Cefoperazone | R |
| Clindamvcin | R | Ampicillin | M | Piperacillin | R |
| Doxycycline | S | Amikacin | S | Benzazolin | R |
| Clarithromycin | S | Ceftazidime | R | Furantoin | R |

Explanations: S: sensitive: M: moderately sensitive: R: resistant.

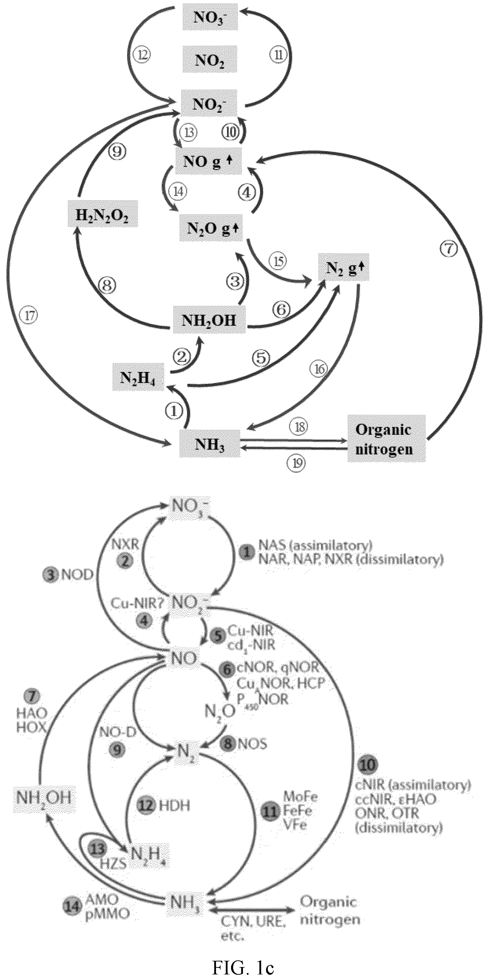

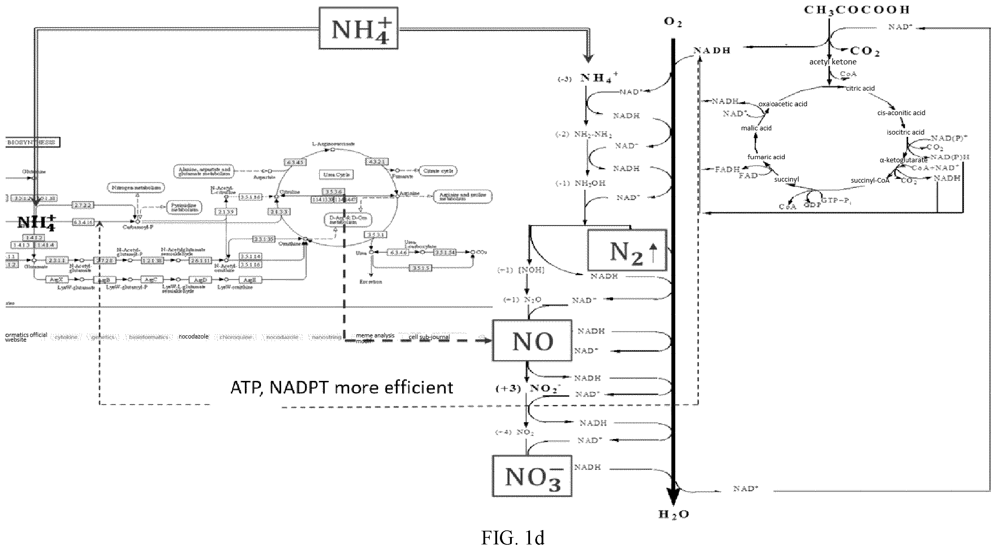

The nitrogen metabolic pathway of the *Mycobacterium sp.* of the invention is shown in FIG. 1c (the upper figure illustrates a schematic diagram of the nitrogen metabolic pathway of the *Mycobacterium sp.* of the invention, and the lower figure is a schematic diagram of the currently known nitrogen metabolic pathway) and FIG. 1d, as can be seen, the *Mycobacterium sp.* of the invention includes an additional direct ammonia oxidation pathway and an organic nitrogen (e.g. arginine) oxidation pathway, in addition to the conventional metabolic pathway.

The second aspect of the present invention provides a microbial agent, is characterized in that the microbial agent comprises the aforementioned *Mycobacterium sp.* The microbial agent can be a solid microbial agent or a liquid microbial agent, or a microbial agent in a proper form prepared by using conventional auxiliary materials or carriers in the field. For example, for the sake of preparing the solid microbial agent, the auxiliary materials can include but are not limited to at least one of non-water-soluble carbonate (e.g., calcium carbonate), rice bran, sawdust, cake meal powder, straw powder and the like, and the viable count of *Mycobacterium sp.* in the solid microbial agent may be within the range of $10^5$-$10^{18}$ CFU/g.

## II. Use in the environmental protection field

The strains of the present invention have nitrogen fixation capability, nitrification capability and denitrification capability, as a result, the third aspect of the present invention provides a method for nitrogen removal, and the method comprises: contacting the aforementioned *Mycobacterium sp.* or the aforementioned microbial agent with a system for nitrogen removal. The system for nitrogen removal refers to various commonly used systems requiring removal of N, wherein N may be present in the conventional form of organic nitrogen and/or inorganic nitrogen, preferably in the form of molecular nitrogen ($N_2$), ammoniacal nitrogen ($NH_3$ and/or $NH_4'$), nitrate nitrogen ($NO_3^-$) or nitrite nitrogen ($NO_2^-$), thus the systems to be denitrified may refer to various systems containing ammonia nitrogen (e.g., waste containing ammonia nitrogen such as $NH_3$ and/or ammonium salts ($NH_4'$)), various systems containing nitrate nitrogen (e.g., waste containing $NO_3^-$), various systems containing nitrite nitrogen (e.g., waste containing $NO_2^-$), various systems containing $N_2$. The system for nitrogen removal can be a system comprising nitrogen-containing pollutants, or any system requiring denitrification, such as wastewater, waste gas or waste residue. The contact enables the *Mycobacterium sp.* to grow by using N in the system for nitrogen removal as a nitrogen source, thereby changing the existence form of N and facilitating the removal of N. A suitable carbon source (e.g. at least one of glucose, pyruvic acid and pyruvate) can also be added into the system depending on the characteristics of *Mycobacterium sp.* When N in the system for nitrogen removal is molecular nitrogen, the contacting can convert the molecular nitrogen into ammoniacal nitrogen, nitrate nitrogen and/or nitrite nitrogen (performing a function of nitrogen fixation), besides carbon source and nitrogen source, inorganic salts also can be added into the contact system, such as 0.1-1g/L of calcium chloride, 0.05-0.5g/L of magnesium sulfate, 0.5-5g/L of dipotassium hydrogen phosphate, 0.005-0.05g/L of sodium molybdate, 0.01-0.1 g/L of ferrous sulfate. When N in the system for nitrogen removal is ammoniacal nitrogen, the contact allows the ammoniacal nitrogen being converted into nitrate nitrogen and/or nitrite nitrogen (performing a function of nitrification). When N in the system for nitrogen removal is nitrate nitrogen and/or nitrite nitrogen, the contacting is carried out under anaerobic conditions, and enables that the nitrate nitrogen and/or the nitrite nitrogen are converted into $N_2$ and/or $N_2O$ (performing a function of denitrification).

The invention also provides a use of the aforementioned *Mycobacterium sp.* or the aforementioned microbial agent in treatment of nitrogen pollution (nitrogen-containing pollutants, particularly ammonia nitrogen contamination). The fourth aspect of the present invention provides a method for treating nitrogen pollution, which is characterized in that the method comprises: contacting the aforementioned *Mycobacterium sp.* or the aforementioned microbial agent with a nitrogen-containing pollutant. The nitrogen-containing pollutant may be any of a variety of common pollutants including ammoniacal nitrogen, and the contacting allows the *Mycobacterium sp.* to grow by using N in the nitrogen-containing pollutant as a nitrogen source, thereby altering the existence form of N to facilitate removal of N. The contacting converts the ammoniacal nitrogen into nitrate nitrogen, nitrite nitrogen and $N_2$. In the method for treating nitrogen pollution, in order to remove ammoniacal nitrogen, the contact system may be initially placed under conditions that facilitate the *Mycobacterium sp.* to exert nitrification (conversion of ammoniacal nitrogen into nitrate nitrogen and/or nitrite nitrogen), and then placed under conditions that are favorable for the *Mycobacterium sp.* to perform denitrification (conversion of nitrate nitrogen and/or nitrite nitrogen into $N_2$) so as to convert environmentally unfriendly ammoniacal nitrogen into colorless and odorless nitrogen gas.

The *Mycobacterium sp.* of the invention is capable of performing one-way nitrogen removal (direct oxidation of an ammonia nitrogen such as hydrazine and/or hydroxylamine into a nitrogen-containing gas (direct ammoxidation), and particularly, under aerobic conditions in the presence of organic matter, the *Mycobacterium sp.* may oxidize the ammonia nitrogen via the pathway "ammonia nitrogen via the ly, under aerobic conditions ingas and nitrous oxide", such a process may be accomplished before occurrence of the nitrification, and the *Mycobacterium sp.* may accomplish whole-course nitrogen removal under aerobic conditions), which overcomes the defect in the conventional biological nitrogen removal technology that the two processes of aerobic nitrification and anaerobic denitrification are required to be performed in stages, thereby greatly simplifying the nitrogen removal process flow, and saving processing space and cost. Therefore, the process for the oxidation of ammonia by using the aforementioned *Mycobacterium sp.* is applicable to a wide variety of ammonia nitrogen-containing waste water (particularly the waste water containing hydroxylamine), and includes single pass nitrogen removal (i.e. direct oxidation of ammonia nitrogen to nitrogen-containing body by controlling pH) and nitrification. In some embodiments of the invention, the ammonia nitrogen-containing waste water is propellant wastewater and/or hydroxylamine wastewater. Preferably, pyruvic acid is used as a carbon source in order to desirably achieve the single-path nitrogen removal.

Various types of hydrazine and/or derivatives thereof (e.g., hydrazine, hydrazine sulfate, unsymmetrical dimethylhydrazine) may be removed by using the aforementioned *Mycobacterium sp.* Hydroxylamine and/or derivatives thereof (e.g., oximes) can also be removed by using the aforementioned *Mycobacterium sp.*

Preferably, the nitrogen-containing pollutant is at least one of hydroxylamine, hydroxylamine derivatives, hydrazine and hydrazine derivatives.

More preferably, the hydroxylamine derivative is oxime.

More preferably, the hydrazine derivative is hydrazine sulfate and/or unsymmetrical dimethylhydrazine.

The *Mycobacterium sp.* of the invention is capable of performing a reverse oxidation tricarboxylic acid cycle to convert $CO_2$ to amino acids, carbohydrates and lipids, thus the invention also provides a use of the aforementioned *Mycobacterium sp.* or the aforementioned microbial agent for carbon capture. The fifth aspect of the present invention provides a method for capturing $CO_2$, which is characterized in that the method comprises: contacting the aforementioned *Mycobacterium sp.* or the aforementioned microbial agent with a $CO_2$-containing system. The contacting enables the growth of *Mycobacterium sp.* by using $CO_2$ to be captured as a carbon source, thereby achieving the carbon capture. A suitable nitrogen source (e.g., an ammonium salt) can also be added into the system depending on the characteristics of the *Mycobacterium sp.* The $CO_2$-containing system may be at least one of a coal fired power plant effluent gas, a natural gas production effluent gas, a synthetic fuel plant effluent gas, a fossil fuel based hydrogen plant effluent gas, and the like.

For the contacting process, unless otherwise specified in the invention, the carbon source is used in such an amount that the content of C in the contact system is preferably 0.5-5g/L; the nitrogen source is used in such an amount that the content of N in the contact system is preferably 0.2-2 g/L. Inorganic salts can also be added into the contact system, such as 0.1-1g/L of sodium dihydrogen phosphate , 0.5-5g/L of dipotassium hydrogen phosphate, 0.01-0.1g/L of magnesium sulfate solution, 0.005-0.05g/L of manganese sulfate , and 0.005-0.05g/L of ferrous sulfate. The contacting process may be carried out at a temperature suitable for growth of *Mycobacterium sp.* (for example 15-35°C, preferably 25-32°C). The pH conditioning agent may be used such that the initial pH of the contacting system is within the range of 6-9, preferably neutral (e.g., within the range of 7-7.2). The contacting time can be determined as desired, for example, from 10h to 50d (e.g., 20h, 1d, 3d, 4d, 5d, 6d, 7d, 8d, 9d, 10d, 11d, 13d, 15d, 17d, 19d, 21d, 23d, 25d, 27d, 29d, 31d, 35d, 40d, 45d, 50d, or any range therebetween). In the case where no reverse explanation is made, the contact (culture/growth) is carried out under aerobic conditions.

WO2009018686A1 (A METHOD FOR REMOVING THE CONTAMINATION OF C, N UTILIZING HETEROTROPHIC AMMONIA-OXIDIZING BACTERIA) still belongs to a mixed bacteria treatment process, and cannot improve the ammonia oxidation rate to the maximum, while the *Mycobacterium sp.* of the invention can be applied in a pure culture mode, the absolute number of bacteria is initially increased to the maximum, and the bacteria are then added, such that the ammonia oxidation rate is improved to the maximum.

### III. The use in the agricultural fields

The inventors of the present invention have discovered that: based on the traditional ammonium nitrogen metabolic pathway, the pathway for the strains of the invention to metabolize ammonium nitrogen further comprises: the arginine oxidation pathway; as a result, in addition to screening nitrification inhibitors based on the conventional route, the substances that can act on any node in the above pathway to inhibit conversion of ammonia nitrogen to other forms and the loss of ammonia nitrogen may be used as the targeted agricultural chemicals. Therefore, the invention also provides a use of the aforementioned *Mycobacterium sp.* or the aforementioned microbial agent for screening agricultural chemicals. The sixth aspect of the invention provides a method for screening agricultural chemicals, which is characterized in that the method comprises: inoculating the aforementioned *Mycobacterium sp.* into an ammonia nitrogen medium containing agricultural chemicals to be screened, and culturing the *Mycobacterium sp.,* and determining whether the agricultural chemicals to be screened have a function of inhibiting the ammonia nitrogen loss based on the change of N during the culturing process and/or the growth condition of the *Mycobacterium sp.* Monitoring the change of N during the cultivation process may include: measuring the amount of ammonia nitrogen in the culture, measuring the amount of nitrate nitrogen and/or nitrite nitrogen in the culture, or detecting the generation of $N_2$ and/or $N_2O$ in the culture system. The agricultural chemicals to be screened can be considered as the products having the effect of inhibiting the loss of ammonium nitrogen, if the ammonium nitrogen content is substantially unchanged after the cultivation process; nitrate nitrogen, nitrite nitrogen, $N_2$ or $N_2O$ is not detected during the cultivation process, or the growth condition of *Mycobacterium sp.* is not good during the cultivation process. The concentration of the agricultural chemicals to be screened in the ammonium nitrogen culture medium containing the agricultural chemicals to be screened may be within the range of 1-20mM, and the medium may be a modified Stephenson's medium added with the agricultural chemicals to be screened, and the specific composition can be as follows: 1-20mM of agricultural chemicals to be screened, 0.5-5g/L of carbon source (calculated in terms of C), 0.2-2g/L of nitrogen source (calculated in terms of N), 0.1-1g/L of sodium dihydrogen phosphate, 0.5-5g/L of dipotassium hydrogen phosphate, 0.01-0.1g/L of magnesium sulfate, 0.005-0.05g/L of manganese sulfate, and 0.005-0.05g/L of ferrous sulfate. The carbon source is preferably at least one of glucose, pyruvic acid and pyruvate. The nitrogen source may be ammoniacal nitrogen ($NH_3$ and/or $NH_4^+$), preferably at least one of ammonia gas, ammonium salts, and amino acids, more preferably ammonium sulfate and/or arginine. The culture temperature may be within the range of 15-35°C, preferably within the range of 25-32°C. The culture time can be from 10h to 50d (e.g., 20h, 1d, 3d, 4d, 5d, 6d, 7d, 8d, 9d, 10d, 11d, 13d, 15d, 17d, 19d, 21d, 23d, 25d, 27d, 29d, 31d, 35d, 40d, 45d, 50d or any range therebetween).

In the invention, the agricultural chemicals mainly refer to potential products capable of inhibiting the loss of ammonia nitrogen, especially the products capable of reducing the loss of nitrogen fertilizer, improving the utilization rate of nitrogen fertilizer and reducing the content of nitrite in crops, thus the agricultural chemicals can be called ammonis nitrogen loss inhibitors, including nitrification inhibitors and/or nitric oxide synthase inhibitors. Since the strains of the invention have more aforementioned metabolic pathways of ammonia nitrogen, its use in screening agricultural chemicals facilitates screening for more potent inhibitors. The nitrification inhibitor can inhibit the activity of enzymes or the growth of nitrifying bacteria in the nitrification process to inhibit nitrification. Specific examples of the ammonia nitrogen loss inhibitors may include non-heterocyclic inhibitors (e.g., aminoguanidines, ethanamidines, cycloalkylamidines, thiocarbamides) and heterocyclic inhibitors (e.g., pyridines, imidazoles, indazoles, triazoles, coumarins, piperazines, piperidines, pteridines, tetrahydropteridines, sulfur-azepine rings, thiazoles) may include, but are not limited to: ammonium sulfate, ammonium nitrate, potassium nitrate, calcium sulfate, copper sulfate, acetylene, ethynylpyridine, phenylacetylene, 4-amino-1,2,4-triazole (ATC), aminoguanidine, aminoguanidine hydrochloride, 2-amino-4-chloro-9-methylpyridine, potassium azide, sodium azide, 2-chloro-6-(trichloromethyl) pyridine (also known as Nitrapyrin, CP), 3,4-dimethylpyrazole phosphate (DMPP), amidinothiourea (ASU), 2-methyl-4,6-bis(trichlorotoluene) s-triazine (MDCT), thiourea-N-2,5-dichlorobutanediamide, allylthiourea, 4-amino-1,2,3-triazole hydrochloride, and 2-sulphathiazole (ST).

The inventors of the present invention have found that: non-amino acid nitrification inhibitors have a strong inhibitory effect on nitric oxide synthase, thus the invention also provides a method of inhibiting (*in vivo, in vitro,* non-therapeutic purposes) the activity of nitric oxide synthase (especially the activity of nitric oxide synthase of *Mycobacterium sp.* in the invention), which is characterized in that the method comprises: contacting at least one of the non-amino acid nitrification inhibitors with nitric oxide synthase. The non-amino acid nitrification inhibitors may be selected from at least one of the group consisting of triazoles, aminoguanidines and thioureas, and preferably at least one selected from the group consisting of 4-amino-1,2,4-triazole, aminoguanidine, aminoguanidine hydrochloride, and amidinothiourea. When the non-amino acid nitrification inhibitor is used for inhibiting the nitric oxide synthase activity of the *Mycobacterium sp.,* the non-amino acid nitrification inhibitor may be directly added into the *Mycobacterium sp.* culture system with an addition amount of 2-10mM.

### IV. Use in the medicament fields

The seventh aspect of the invention provides a use of the aforementioned *Mycobacterium sp.* or the aforementioned microbial agent in treatment and/or prevention of skin diseases, the treatment and/or prevention of diseases associated with low nitrite level, the treatment and/or prevention of body odour, the treatment of supplying nitric oxide to a subject, or inhibiting growth of microorganisms.

In some embodiments, the Mycobacterium sp. or microbial agent may be used to treat at least one of the following diseases: HIV, dermatitis, infection (e.g., an infection in a diabetic foot ulcer) in an ulcer (e.g., a venous ulcer, e.g., an ulcer of lower limb, e.g., a lower limb venous ulcer), allergic dermatitis, acne (e.g., acne vulgaris), eczema, contact dermatitis, anaphylaxis, psoriasis, urticaria, rosacea, skin infections, vascular diseases, vaginal yeast infections, sexually transmitted diseases, cardiopathy, atherosclerosis, alopecia, ulcer of lower limb secondary to diabetes or bed rest, angina (particularly chronic stable angina), ischemic diseases, congestive heart failure, myocardial infarction, ischemia reperfusion injury, laminitis, hypertension, hypertrophic organ degeneration, Raynaud's phenomenon, fibrosis, fibrotic organ degeneration, allergy, autoimmune sensitization, end-stage renal disease, obesity, impotence, pneumonia, primary immunodeficiency disease, epidermolysis bullosa or cancer.

In some embodiments, the *Mycobacterium sp.* or microbial agent may be used to treat at least one of the following diseases: infections in diabetic foot ulcers, allergic dermatitis, acne (e.g. acne vulgaris), eczema, psoriasis, urticaria, rosacea and skin infections.

In some embodiments of the invention, the use is a use in manufacture of a medicament or formulation for treatment and/or prevention of skin diseases, treatment and/or prevention of diseases associated with low nitrite level, treatment and/or prevention of body odour, treatment of supplying nitric oxide to a subject, and inhibiting growth of microorganisms.

In some embodiments of the invention, the inhibition is *in vitro* inhibition.

In some embodiments of the invention, the microorganisms are *Mycobacterium* genus microorganisms, given that the strains of the invention are capable of producing nitric oxide, the strains of the invention are capable of inhibiting various nitric oxide-sensitive microorganisms (including fungi, bacteria and viruses), in particular various nitric oxide-sensitive microorganisms that cause skin diseases and/or various nitric oxide-sensitive microorganisms that grow well *in vitro,* more preferably at least one of *Mycobacterium vanbaalenii, Mycobacterium vaccae and Mycobacterium austroafricanum.*

In the invention, the medicament or formulation can further contain pharmaceutically common auxiliary materials so as to prepare a specific dosage form for use. The dosage forms of the medicament may be such as tincture, ointment, cream, paste, aerosol, spray, powder, otic formulation, lotion, rinse, liniment, smear, coating, gel, patch, and the like.

## V. Isolation method

The eighth aspect of the invention provides a method for isolating the *Mycobacterium sp.* with nitrification activity, which is characterized in that the method comprises: (1) enriching the *Mycobacterium sp.* with a nitrification activity; (2) using a medium added with antibiotics for screening the *Mycobacterium sp.* with a high nitrification activity.

In some embodiments of the invention, the medium used for enrichment may be a medium commonly used in the field for isolating *Mycobacterium tuberculosis* In other embodiments of the invention, the medium used for enrichment is preferably a modified Stephenson's medium, more preferably containing: optionally 0.5-5g/L (calculated in terms of C) of organic carbon source (e.g., glucose), 2-10g/L (calculated in terms of carbonate ion) of carbonate (e.g., calcium carbonate), 0.2-2g/L (calculated in terms of N) of ammonium sulfate, 0.1-1g/L of sodium dihydrogen phosphate, 0.5-5g/L of dipotassium hydrogen phosphate, 0.01-0.1g/L of magnesium sulfate, 0.005-0.05g/L of manganese sulfate, and 0.005-0.05g/L of ferrous sulfate. In step (1), the culture temperature for the enrichment may be within the range of 15-35°C, preferably 25-32°C. The culture time for enrichment can be determined according to the qualitative detection results of $NH_4^+$-N, $NO_2^-$-N and $NO_3^-$-N, and may be 4-30 days.

In the invention, if the target strains are isolated after the enrichment of the modified Stephenson's medium, the strains can be selected for performing the screening process in step (2), if the target strains are not isolated after the enrichment of the modified Stephenson's medium, the strains can be further treated with acid (impurity removal) and then subjected to the operation of step (2). Therefore, in a preferred embodiment of the invention, step (1) comprises: inoculating the sample into the modified Stephenson's medium for culturing, then mixing the culture with acid and subjecting to the acid treatment, and neutralizing the acid in the treated system. The amount of acid in the acid-treatment may be 0.2-2mol (calculated in terms of hydrogen ion), preferably 0.3-0.5mol (calculated in terms of hydrogen ion) relative to 1L of the culture. The acid used for the acid treatment may be any of commonly used inorganic acids, preferably HCl. The temperature of acid treatment is preferably within the range of 3-8°C. The time of acid treatment is preferably within the range of 1-5 min. The alkali used for neutralization may be a commonly used inorganic base such as an alkali metal hydroxide (e.g., sodium hydroxide).

In some embodiments of the present invention, the content of the antibiotic in the culture medium supplemented with an antibiotic in step (2) is preferably within the range of 0.1-0.5 mg/L. The antibiotic may be any of various antibiotics commonly used in the field, preferably malachite green, penicillin and the like. Preferably, the culture medium supplemented with an antibiotic is a LB culture medium supplemented with an antibiotic, more preferably comprising: 5-15g/L of peptone, 5-15g/L of yeast extract, 5-15g/L of sodium chloride and 0.1-0.5mg/L of antibiotic. The culture temperature for the enrichment may be within the range of 15-35°C, preferably within the range of 25-32°C. The culture time for the enrichment may be 4-30 days. After the enrichment in step (2), the dominant bacteria are the target bacteria having a high nitrification activity. The inventors of the present invention have discovered that the time of the acid treatment and the concentration of the antibiotic within the above preferred ranges enable the desirable isolation of the target strain, otherwise may cause failure of the isolation. The method of the invention is particularly advantageous for the isolation of *Mycobacterium sp.*

The present invention will be described in detail below with reference to examples. In the following preparation examples and examples, a portion of the culture media and test methods used are as follows:

## 1. Preparation of Basal medium

### 1.1 Preparation of modified Stephenson's medium (ST medium)

2.0g of $(NH_4)_2SO_4$, 0.25g of $NaH_2PO_4$, 0.75g of $K_2HPO_4$, 0.03g of $MgSO_4 \cdot 7H_2O$, 0.01g of $MnSO_4 \cdot H_2O$ and 0.01g of $FeSO_4 \cdot 7H_2O$ were weighed, and dissolved in 1,000ml of deionized water, the pH was adjusted to 7.0-7.2, and sterilized with high pressure steam for use.

### 1.2 Ordinary nutrient agar plate (PM plate)

3g of beef extract, 5g of peptone, 20g of agar and 1,000ml of distilled water were mixed, pH 7.0-7.2. Before sterilization, the pH of each culture medium was adjusted to the desired pH range by using NaOH solution having a concentration of 1M or HCl solution having a concentration of 25% (V/V).

### 1.3 Medium with sodium pyruvate as a carbon source

According to the concentration requirements of each experiment, a sodium pyruvate solution with a certain concentration was prepared, the solution was independently sterilized, a certain volume (the volume was determined according to the concentration required by each experiment) of sodium pyruvate solution was added into a modified Stephenson's medium before use, such that the designed concentration of the sodium pyruvate in the culture medium met the requirements of each experiment. The actual concentration would be lower than the designed concentration due to partial decomposition of sodium pyruvate during the preparation process. The desired pH was adjusted by using 1M NaOH solution.

## 2. Qualitative determination of nitrite nitrogen ($NO_2^-$-N)

### 2.1 Preparation of Griess reagent

> **(1) Sulfanilic acid reagent (solution A):** 0.5g of sulfanilic acid was dissolved in 150ml of dilute acetic acid solution with a concentration of 20%, stored in a brown bottle and refrigerated for use.
> **(2) $\alpha$-naphthylamine reagent (solution B):** 0.5g of $\alpha$-naphthylamine was added into 20ml of distilled water and 150ml of diluted acetic acid solution with a concentration of 20%, and stored in a brown bottle and refrigerated for use.

**2.2 Qualitative determination of $NO_2^-$-N**: a plurality of droplets of the culture solution were put on a white porcelain colorimetric plate, two drops of Griess reagents A and B were added respectively, if nitrite existed, the red color would be displayed.

Color and its corresponding concentration range: "+-" exhibited trace pink, the concentration was lower than 0.5mg/L; "+" exhibited light pink, the concentration was about 0.5mg/L; "++" exhibited pink, the concentration was about 1mg/L; "+++" exhibited deep carmine, the concentration was about 5mg/L; "++++" exhibited light brown, the concentration was about 10mg/L; ">++++" exhibited brown, the concentration was higher than 20mg/L.

## 3. Qualitative determination of ammonium nitrogen ($NH_4^+$-N)

### 3.1 Preparation of Nessler's reagent

17g of potassium mercuric iodide was weighed and dissolved in 10ml of water, 24.4g of KOH was dissolved in 100ml volumetric flask containing 70ml of water, cooled to room temperature, the potassium mercuric iodide was slowly injected into the volumetric flask while shaking, water was added until the scale and the solution was uniformly mixed, and subjected to standing still for two days for use. The solution was placed in a brown bottle and stored in a dark place.

3.2 Qualitative determination of $NH_4^+$-N: several droplets of culture solution were taken and put on a white porcelain colorimetric plate, two drops of Nessler reagent were added, if ammonium nitrogen existed, the yellow color would be displayed.

Color and its corresponding concentration range: "+" indicated yellowish, the concentration was less than 0.5mg/L; "+++" indicated a concentration of about 5mg/L; "++++" indicated a concentration of about 10mg/L; ">++++" indicated a dark yellow color, the concentration was higher than 20mg/L.

### 4. Qualitative determination of nitrate nitrogen ($NO_3^-$-N)

### 4.1 Preparation of diphenylamine reagent

0.5g of diphenylamine, 100ml of concentrated sulfuric acid and 20ml of distilled water were used. Diphenylamine was initially dissolved in distilled water, concentrated sulfuric acid was slowly added, then stored in brown bottle for use. During qualitative determination, several droplets of culture solution were taken and put on a white porcelain colorimetric plate, two droplets of diphenylamine reagent were added, if nitrate existed, the blue color would be displayed.

4.2 Qualitative determination of $NO_3^-$-N: several droplets of culture solution were placed on a white porcelain colorimetric plate, two droplets of diphenylamine reagent were added, and if nitrate existed, the blue color would be displayed. "-" indicated a negative result after the diphenylamine droplets were added, and "+" indicated a positive result after the diphenylamine droplets were added.

### 5. Quantitative determination of various forms of nitrogen

When the quantitative determination of various indicators was carried out, 5,000g the culture solution was centrifuged at 4°C for 15min, and the supernatant was extracted for testing.

The quantitative determination of $NH_4^+$-N was carried out by indophenol blue colorimetric method, and the quantitative determination of $NO_2^-$-N was performed by Griess reagent colorimetric method. TN was measured by using potassium peroxysulfate oxidation-flow analyzer. The quantitative determination of $NO_x^-$-N ($NO_2^-$-N + $NO_3^-$-N) included reduction with hydrazine sulfate, and measurement with flow analysis-spectrometry, firstly, the contents of $NO_x^-$-N ($NO_2^-$-N + $NO_3^-$-N) and the content of $NO_2^-$-N were measured respectively, wherein the content of $NO_3^-$-N was the difference between the contents of $NO_x^-$-N and the content of $NO_2^-$-N. The basic principle was that pump pipes with different pipe diameters were compressed by a peristaltic pump, a reaction reagent and a sample to be detected were injected into a closed continuous flowing current carrier in proportion, a color reaction was carried out in a chemical reaction unit, a signal value of the color reaction was measured in a detector, and the concentration of the sample to be detected was measured according to the standard curve method.

### 6. Determination of gas in culture system

The contents of $N_2$, $CO_2$, $O_2$, $N_2O$ and other gas in the culture system were measured by the gas chromatograph (Agilent).

**7. Measurement of cell growth amount:** turbidimetry, expressed by OD value at a wavelength of 600nm.

**8. Determination of pH value:** precision pH test paper was used in conjunction with a pH meter.

**9. Determination of pyruvic acid concentration:** the culture medium supernatant was taken (4°C, high speed centrifugation at 5,000rpm for 15 min) and measured by an Agilent 1260 liquid phase chromatograph (ultraviolet detector), wherein the basic conditions were as follows: a wavelength of 214nm; mobile phase; the rate of phosphate buffer solution: methanol =98:2, the flow rate was 1.0 ml/min.

**10. Treatment and analysis of culture:** when the cultured thallus was analyzed, the thallus was firstly obtained by centrifuging under the conditions including a temperature of 4°C and a high-speed centrifuge with the rotating speed of 10,000rpm for 5min, the thallus was stored at the temperature of 4°C for use.

When the dry weight of the culture and the contents of carbon and nitrogen were measured, the centrifuged thallus shall be frozen and vacuum-dried and then weighed.

**11. Experimental replication and layout:** each experiment used non-inoculated medium as control group (CK), the experiment was laid out according to the standard that each test was performed in 3 replicates.

**Preparation Example 1 Isolation of *Mycobacterium sp.***

**1. The isolation and identification method of target bacteria**

**1.1 Medium**

Required medium: modified Stephenson's medium, LB-malachite green solid culture medium, PM plate.

**Preparation of a LB-malachite green solid culture medium**

(1) **Preparation of malachite green mother liquor:** 0.125g of malachite green solid was weighed and dissolved in 500ml of deionized water, pH was adjusted to 7.0 after complete dissolution, and sterilized by high-pressure steam for use.

(2) **Preparation of a LB solid culture medium:** 10g of peptone, 10g of yeast extract and 10g of sodium chloride were weighed, dissolved in 1,000ml of deionized water, pH was adjusted to 7-7.2, and sterilized with high-pressure steam for use. Every 100ml of LB solid medium was added with 100μl of malachite green having a concentration of 0.25g/L, such that the concentration of malachite green in LB solid medium was 0.25 mg/L.

**1.2 Isolation method**

(1) Enrichment culture: 2g of the sample (original sample or enrichment culture sample) was inoculated into 100ml of modified Stephenson's medium containing calcium carbonate, the content of calcium carbonate in the culture medium was 0.5g/100ml; or the sample was inoculated into the modified Stephenson's medium containing calcium carbonate and added with organic carbon source (the concentration was 0.01M); shake cultivation was performed at 28°C and a rotating speed of 180rpm to increase abundance of the target bacteria in the culture medium. Generally, the isolation was implemented when the enrichment culture was carried out until the $NH_4^+$-N concentration in a culture medium indicated a negative result after the Nessler reagent were dripped, the $NO_2^-$-N concentration in a culture medium indicated a positive result after the Griess reagent were dripped; or both the $NH_4^+$-N and $NO_2^-$-N in a culture medium indicated a negative result, the $NO_3^-$-N concentration in a culture medium indicated a positive result after the diphenylamine droplets were added.

(2) Removing impurities: 3ml of cold concentrated hydrochloric acid (4°C, the addition amount was 3% by volume of the culture solution) was added into the culture system, vibrated uniformly to remove other hybrid bacteria; the reaction mixture was then rapidly neutralized with a cold sodium hydroxide solution (4°C) within 3 minutes to adjust pH of the system to about 7.0.

(3) Isolation of target bacteria by selective (positive) plates: the bacterial liquid expressing nitrification activity after the impurity removal treatment was diluted according to a ten-fold gradient and coated on a LB-malachite green solid plate, thereby isolating the target bacteria in a targeted manner.

**1.3 Purification**

After culturing at 28°C for 7 days, a single colony was picked up on the PM plate and lineated for purification. The microscopic examination was carried out to verify the purity.

**1.4 Activity identification**

The obtained separated and purified target bacteria was inoculated on a PM plate, cultured at 28°C for 10 days, the Griess reagent was directly dropwise added on the plate, the nitrification activity was confirmed, and the plate without inoculation of bacteria as a blank control. Within 1min, the Griess reagent displayed red color, indicated the generation of nitrite. The verification was repeated, the chromogenic reaction still displayed the positive result, it was preliminarily determined that the bacteria were nitrifying active bacteria. The PM culture medium was subjected to the high pressure liquid chromatography analysis, it indicated that the contents of nitrite and nitrate were trace amounts, wherein the nitrite was not detected, and the $NO_3^-$-N content was lower than 0.2mg/L, so that the result was not interfered.

**1.5 nitrification activity verification under liquid pure culture**

1 ring of the pure bacterial lawn grown on the PM plate was scraped and added into 100mL of PBS solution, subjected to ultrasonic treatment for 2min, and fully and uniformly dispersed to obtain a PBS-target bacterial suspension. 1ml of the bacterial suspension was inoculated into 50ml of modified Stephenson culture medium (the container was 250ml conical flask) with different organic substances as a carbon sources, and each group had 3 replicates. The culture medium without inoculation of bacterial suspension was used as a blank control. The bacterial suspension was subjected to shake cultivation at 28°C and a rotational speed of 180rpm for more than 14 days, the supernatant of the culture solution was extracted to determine $NO_2^-$-N content. The $NO_2^-$-N content was 5mg/L higher than the control group, it was determined that the nitrification activity was obviously high.

**2. Isolation, purification and identification of soil target bacteria in hydro-fluctuation belt of Three Gorges Reservoir of Yangtze River in Kaizhou District of Chongqing Municipality in China.**

The soil sample to be tested was taken from the hydro-fluctuation belt (with the latitude 31°07'56-57" and longitude 108°28'34-35") of the Three Gorges Reservoir of Yangtze River in Kaizhou District of Chongqing Municipality.

5ml of 0.1M glucose (concentration in the medium was 0.01M) was added into 45ml of modified Stephenson's medium (supplemented with calcium carbonate), 1g of a soil sample after screened with 20-mesh sieve was added, then subjected to a shake cultivation at 28°C and the rotational speed of 180rpm for 14 days.

About 200μl of samples were taken every 2 or 3 days, the samples were placed on a porcelain colorimetric plate, the Griess reagent was dripped (qualitative determination) to observe the change of $NO_2^-$-N content, the samples were cultured for about 7 days, and showed the obvious nitrification activity, the content was about 1.0ppm, and the $NO_2^-$-N content exceeded 20ppm after the samples are cultured for 13 days. The results were shown in the following table:

Table 3a

| Culture days | Color change after dripped with Griess reagent | | |
|---|---|---|---|
| | 1 replicate | 2 replicates | 3 replicates |
| 1 day | +- | +- | +- |
| 4 days | +- | +- | +- |
| 7 days | ++ | ++ | ++ |
| 9 days | +++ | +++ | +++ |
| 11 days | >++++ | >++++ | >++++ |
| 13 days | >++++ | >++++ | >++++ |

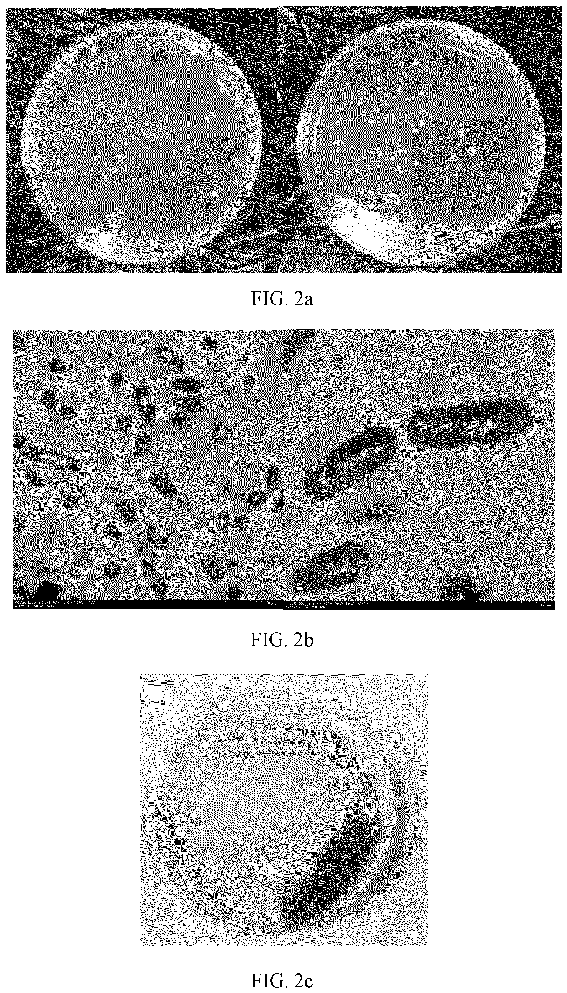

After 14 days of culture, concentrated HCl solution (the addition amount was 3% by volume of the culture solution) was added into the culture solution, after vibration for 3min, the culture solution was diluted according to 10 times of gradient and then coated on a LB-malachite green plate, each dilution gradient had 3 replicates. The results showed that the number of target bacteria averaged 21 under the $10^{-7}$ dilution gradient (FIG. 2a, two replicates were illustrated in the left figure and right figure). In the meanwhile, the target microorganism was isolated.

A single colony was picked and placed on a PM plate, and subjected to lineation and purification. After cultivation at 28°C, the microscopic examination was performed, it was confirmed that the external morphology of thallus was single, it was pure bacterium (FIG. 2b).

The obtained separated and purified bacteria was inoculated on the PM plate, cultured at 28°C for 10 days, the Griess reagent was directly dripped on the plate, the color was immediately changed into brown (FIG. 2c), $NO_2^-$-N content was higher than 10mg/L, indicated a strong nitrification activity.

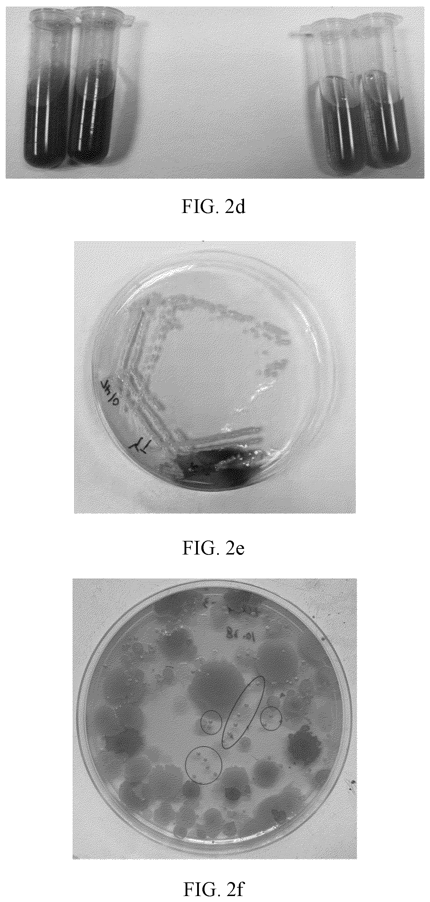

The pure bacterial lawn grown on the PM plate was scraped to prepare a PBS-target bacterial suspension, 1ml bacterial suspension was inoculated into 100ml of modified Stephenson culture medium (the container was 250ml conical flask) with 0.06M pyruvic acid as a carbon source, each group was repeated three times. The culture medium without inoculation of bacterial suspension was used as a blank control. The bacterial suspension was subjected to shake cultivation at 28°C and a rotational speed of 180rpm for 24 days, the supernatant of the culture solution was extracted to determine that $NO_2^-$-N content exceeded 100 mg/L. FIG. 2d showed the color development conditions of Griess reagent after the supernatant of the target bacterium culture solution was diluted by 10-fold and 100-fold, it indicated that the target bacterium had a high nitrification activity.

### 3. Isolation of target bacteria from North China moisture soil

1g of North China soil screened with 100 mesh sieve was inoculated into 50ml of modified Stephenson culture medium added with calcium carbonate for culture, the change of $NO_2^-$-N concentration was detected regularly by using the Griess reagent to express the nitrification activity; after culturing for 14 days, concentrated HCl solution was added into the culture system, the addition amount was 3% by volume of the culture solution, the culture solution was fully vibrated; the pH was rapidly neutralized with sodium hydroxide solution within 3min to about 7.0. 1ml of the culture solution after neutralization with alkali was added into 9ml of sterilized sterile water, diluted according to 10 times of gradient and coated on a LB-malachite green plate, and each dilution gradient was repeated for 3 times. Target colonies appeared on the plate. The statistics of the bacteria appearance of the target bacteria were shown in the following table:

Table 3b

|  | 1 replicate (number of bacteria) | 2 replicates (number of bacteria) | 3 replicates (number of bacteria) |
|---|---|---|---|
| $10^{-1}$ | 36 | 44 | 47 |
| $10^{-3}$ | 89 | 7 | 27 |
| $10^{-4}$ | 371 | 71 | 9 |

A single colony was picked and placed on a PM plate, and subjected to lineation and purification. The microscopic examination was performed. After cultivation at 28°C for 14 days, the color of said colony was changed to brown color after dripped with Griess reagent (FIG. 2e), indicated a strong nitrification activity.

The pure bacterial lawn grown on the PM plate was scraped to prepare a PBS-target bacterial suspension, 1ml of bacterial suspension was inoculated into 100ml of modified Stephenson culture medium (the container was 250ml conical flask) with 0.06M pyruvic acid as a carbon source, each group was repeated three times. The culture medium without inoculation of bacterial suspension was used as a blank control. The bacterial suspension was subjected to shake cultivation at 28°C and a rotational speed of 180rpm for 28 days, the supernatant of the culture solution was extracted to determine that $NO_2^-$-N content exceeded 100 mg/L. It indicated that the target bacterium had a high nitrification activity.

### 4. Isolation of target bacteria from other various soils

By using the isolation scheme of the target bacteria developed in the present invention, the target bacteria can also be isolated from the soil in farmland in Zhaogang Township, Fengqiu County, Xinxiang City, Henan Province (longitude and latitude were 35°02' and 114°5'), Wushan soil (longitude and latitude were 31°32'58.98" and 120°41'55.37") and yellow soil (longitude and latitude were 31°48.87" and 120°38'32.22") in Changshu City, Jiangsu Province; and red soil in Yingtan City, Jiangxi Province.

It was discovered from isolation that the hetero-bacteria were generally present at 24h, while the target bacteria were present at 5-7 days. FIG. 2f showed the emergence of the target bacteria from the soil or soil suspension (modified Stephenson's medium), the target bacteria were indicated by the circle, and were clearly distinguishable from other hetero-bacteria.

### Example 1

### Authigenic nitrogen fixation performance of *Mycobacterium sp.* of the invention

### 1. Materials and methods

**1.1 Preparation of experimental strains and PBS bacterial liquid:** strains JD and YT were obtained according to the same method in Preparation Example 1 and deposit in CGMCC. The strain JD was isolated from soil in the hydro-fluctuation belt (with the latitude 31°07'56-57" and longitude 108°28'34-35") of the Three Gorges Reservoir of Yangtze River in Kaizhou District of Chongqing Municipality; the strain YT was isolated from soil in farmland in Zhaogang Township, Fengqiu County, Xinxiang City, Henan Province (longitude and latitude were 35°02' and 114°5').

1-ring of pured bacteria of strain JD or YT was placed in a 100mL PBS solution, subjected to ultrasonic treatment for 2min, uniformly dispersed to about $1\times10^7$ CFU/mL, and prepared into PBS-JD/YT bacterial liquid for use.

### 1.2 Authigenic nitrogen fixation in the absence of nitrogen in the form of chemistry

1ml of PBS-YT/JD bacterial liquid was inoculated in a liquid medium for pure culture, and the nitrogen fixation capability of the *Mycobacterium sp.* was preliminarily judged according to the concentration change condition of $NH_4^+$-N, $NO_2^-$-N and $NO_3^-$-N. The fixation plate was then used for carrying out the lineation culture, the growth condition of the plate was observed, if the bacteria grew normally, the colonies were scraped to detect the nitrogen fixation gene; if the nitrogen fixation gene was detected, it was determined that the nitrogen fixation function existed in the colonies.

### 1.2.1 Liquid medium and cultivation method

### A: Liquid medium

The medium contained glucose, $CaCl_2$, $MgSO_4 \cdot 7H_2O$, $K_2HPO_4 \cdot 3H_2O$, $Na_2MoO_4 \cdot 2H_2O$, HCl and $FeSO_4 \cdot 7H_2O$, and all the reagents were prepared separately.

Glucose: 11.25g +45ml water
$CaCl_2$: 0.625g +25ml water
$MgSO_4 \cdot 7H_2O$: 1.25g +25ml water
$K_2HPO_4 \cdot 3H_2O$: 2.62g +25ml water
$Na_2MoO_4 \cdot 2H_2O$: 0.15g +25ml of water
HCl: 100ml HCl +900ml water
$FeSO_4 \cdot 7H_2O$: 0.5g +12.5ml water (prepared ready to use on-site, filtered and sterilized)

During preparation, 100ml of deionized water was placed in a 250ml triangular flask and separately sterilized, all reagents were separately prepared and then placed in a high-pressure steam kettle and sterilized for use.

Under aseptic condition, 2ml of glucose, 0.6ml of $CaCl_2$, 0.4ml of $MgSO_4 \cdot 7H_2O$, 0.1ml of $FeSO_4 \cdot 7H_2O$, 0.1ml of $Na_2MoO_4 \cdot 2H_2O$, 1ml of $K_2HPO_4 \cdot 3H_2O$ and 0.16ml of HCl were added into each 100ml of aseptic water to obtain the liquid culture medium for the authigenic nitrogen fixation experiment.

### B: Culture system and culture conditions

1ml of PBS-JD/YT bacterial liquid was inoculated into the liquid medium, and cultivated at a temperature of 28°C and a shaking table with a rotational speed of 180rpm. The change conditions of $NH_4^+$-N, $NO_2^-$-N, $NO_3^-$-N contents in the culture medium were qualitatively determined every three days, and the nitrogen fixation capability was preliminarily determined.

1.2.2 Nitrogen fixation plate method

The experimental pure strains were lineated and inoculated in a nitrogen fixation medium, and cultivated in an incubator at 28°C, wherein the humidity of the culture condition was ensured to be constant during the cultivation process, and the evaporation of moisture in the plate was prevented. The growth condition of the bacterial colony was observed for a certain time, if the bacterial colony can grow normally, all bacterial colonies were scraped by using a sterilized stainless steel medicine spoon, and suspended in phosphate buffer solution PBS, centrifuged to remove supernatant, collected the centrifuged solid matter, cryo-preserved by using liquid nitrogen, the nitrogen fixation gene and the nitrogen fixation capability of the bacterial colony were detected.

### 1.2.3 Detection of nitrogen fixation gene

Three pairs of nitrogen fixation primers were used for performing PCR amplification, each pair of primers was provided with 4 replicates. The three pairs of primers were able to amplify nitrogenase ferritin from nitrogen fixation bacteria. Letters (e.g., Y, S, R, B, W) other than A, T, C, G in the primer sequence indicated degenerate bases which enabled the primers to amplify the target gene more comprehensively.

The amplification length of the first pair of primers was 360bp, and the primer sequences were as follows:

PolyF: 5'-TGCGAYCCSAARGCBGACTC-3' (SEQ ID NO: 3)
PolyR: 5'-ATSGCCATCATYTCRCCGGA-3' (SEQ ID NO: 4)

The amplification length of the second pair of primers was 280bp, and the sequences of the primers were as follows:

nifH F: 5'-ACCCGCCTGATCCTGCACGCCAAGG-3' (SEQ ID NO: 5)

nifH R: 5'-ACGATGTAGATTTCCTGGGCCTTGTT-3' (SEQ ID NO: 6)

The amplification length of the third pair of primers was 450bp, and the sequences of the primers were as follows:

nifH-F: 5'-AAAGGYGGWATCGGYAARTCCACCAC-3' (SEQ ID NO: 7)
nifH-R: 5'-TTGTTSGCSGCRTACATSGCCATCAT-3' (SEQ ID NO: 8)

**1.3 Authigenic nitrogen fixation in the presence of nitrogen in the form of chemistry**

The PBS-JD/YT bacterial liquid was inoculated into a medium with pyruvic acid as a carbon source and ammonium sulfate as a nitrogen source for pure culture, the growth of the bacteria and the change condition of $NH_4^+$-N concentration of the culture medium were periodically measured in the cultivation process; if the bacteria grew normally and the $NH_4^+$-N concentration suddenly increased at the same time, the existence of nitrogen fixation phenomenon can be judged. The authigenic nitrogen fixation of *Mycobacterium sp.* of the present invention in the presence of nitrogen in the form of chemistry only took ammonia secretion by the strain into consideration, and the nitrogen fixation during cell synthesis process was not considered. In addition, the authigenic nitrogen fixation energy consumption of the *Mycobacterium sp.* was calculated according to the consumption of the pyruvic acid corresponding to the stage.

**1.3.1 Medium with pyruvic acid as a carbon source and ammonium sulfate as a nitrogen source**

A: Modified Stephenson's medium: the nitrogen source was ammonium sulfate with a concentration of 2.0 g/L.
B. Medium with pyruvic acid as a carbon source: the prepared and separately sterilized pyruvic acid solution was added into the modified Stephenson's medium, such that the designed concentration of pyruvic acid in the culture medium was 0.02M. The desired pH was finely adjusted by using NaOH solution with a concentration of 1M.

1ml of PBS-JD/YT bacterial liquid was inoculated in 100ml of culture medium with pyruvic acid as a carbon source and ammonium sulfate as a nitrogen source for pure culture.
The culture conditions: temperature of 28°C, rotating speed of 180rpm, $CO_2$ concentration of 0.5%, and the relative humidity (RH) of 50%.

**1.3.2 Analysis of indicators**

After 5 days of culture, the growth amount of the thallus was measured every 2 days. The contents of $NH_4^+$-N, $NO_2^-$-N and $NO_x^-$-N in supernatant of the culture solution were measured.

**1.4 Quantification determination of nitrogen fixation activity**

The nitrogen fixation activity of the experimental strain was determined by acetylene reduction method. 10mL of the bacterial liquid at the middle logarithmic growth phase (OD600=0.4-0.8) was added into 100mL glass bottle, and after sealing, 10% of the gas volume in the bottle was replaced by a syringe with purified acetylene. The culture was carried out at 28°C for 24 hours in the dark condition, the gas in the flask was collected, and the ethylene concentration was analyzed by a gas chromatograph (Shimadzu, GC-2014C) equipped with a hydrogen flame ionization detector (FID), wherein the chromatographic column was Porapak T 4 mm×2 m packed column. The chromatographic column temperature was set at 60°C, the injector temperature was set at 120°C, the FID detector temperature was set at 220°C, and the carrier gas was high purity nitrogen gas. The biomass of bacterial liquid was characterized by measuring the protein content of the bacterial liquid by Bradford method (Bradford, 1976), and Bovine Serum Albumin (BSA) was used as protein standard. The ethylene production rate per unit mass of protein per unit time (nmol $C_2H_4$/mg protein/h) was calculated.
The calculation formulas were as follows:

(1) Calculation of headspace volume ($cm^3$) in the bottle: $V_{headspace}=V_{total}-V_{liquid}$. $V_{total}$ was 100$cm^3$, and $V_{liquid}$ was the volume occupied by 10ml of bacterial liquid.
(2) According to the ethylene standard gas concentration, the ethylene concentration (ppm) of a sample was

$$C_{C_2H_4} = \frac{P_{C_2H_4} \times C_{s\tan dard C_2H_4}}{P_{s\tan dard C_2H_4}}$$

calculated by an external standard method: $P_{C2H4}$ denoted the measured peak height of sample, and $C_{standard\ C2H4}/P_{standard\ C2H4}$ was the ethylene concentration corresponding to the unit ethylene peak height.

$$E_{total} = \frac{C_{C_2H_4} \times V_{headspace}}{22.4}$$

(3) Calculation of ethylene production (nmol $C_2H_4$) during the cultivation time:

(4) Calculation of the ethylene production rate per unit mass of protein per unit time (nmol $C_2H_4$/mg/h):

$$Rate = \frac{(E_{total})_{t_i} - (E_{total})_{t_0}}{\Delta t \times W_{sample}}$$

## 1.5 Comparison of nitrogen fixation energy consumption

The nitrogen fixation capacity of *Mycobacterium sp.* in the invention was analyzed and investigated by selecting typical nitrogen fixation bacteria genera/species such as *Azotobocter (Azotobacter chroococcum* and the like), *Clostridium pasteurianum, Klebsiella pneumoniae,* and *Streptomyces thermoautotrophicus,* and taking the artificial nitrogen fixation into consideration, using the number of milligrams of nitrogen fixed per 1 milligram of pyruvic acid consumed, based on the published nitrogen fixation energy consumption data of the relevant bacteria genera/species and artificial nitrogen fixation, and comparing it with the experimental results of nitrogen fixation capacity *of Mycobacterium sp.* strains JD and YT in the invention.

## 2. Results

## 2.1 Authigenic nitrogen fixation in the absence of nitrogen in the form of chemistry

## 2.1.1 Growth condition in the liquid medium

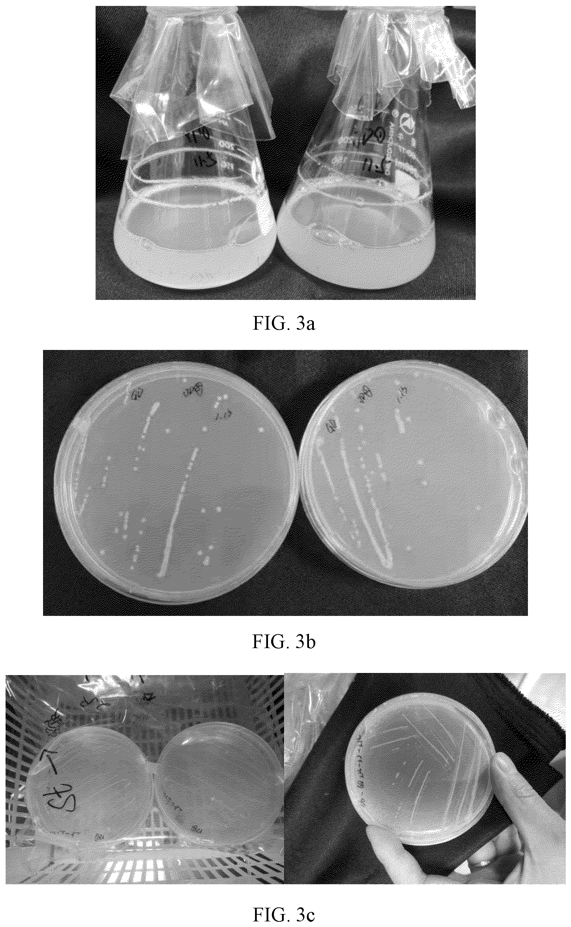

The bacterial strains JD and YT were inoculated in a liquid medium respectively, both of the JD strain and the YT strain could grow, but grew slowly; it was observed that a ring of bacteria was adhered to the wall of the culture bottle, it indicated that the two strains were strongly adhered to the wall, such that the continuous growth of the bacteria was affected, as shown in FIG. 3a, the strain YT was on the left side, and the strain JD was on the right side.

Table 4a illustrated the pH changes of the liquid medium and the accumulation conditions of $NH_4^+$-N, $NO_2^-$-N, $NO_3^-$-N during pure culture of the *Mycobacterium sp.* strains JD and YT. In the cultivation process, strains JD and YT would produce acid, so that the pH of the liquid medium was decreased, however, the bacterial strain stopped growing when the pH of the culture medium was too low, thus the pH was timely adjusted to 7.0-7.2 during the cultivation process. By qualitative analysis, there was accumulation of $NH_4^+$-N in the liquid medium, indicated that the strains JD and YT had a certain nitrogen fixation capability.

**Table 4a Changes in pH and ammoniacal nitrogen during pure culture of *Mycobacterium sp.* strains JD and YT in the liquid nitrogen fixation medium**

| Growth time (d) | Strain | pH | $NH_4^+$-N | $NO_2^-$-N | $NO_3^-$-N |
|---|---|---|---|---|---|
| 4 | CK | 7.0-7.2 | - | - | - |
| | JD | 6.4-6.7 | + | - | - |
| | YT | 6.4-6.7 | + | - | - |
| 7 | CK | 7.0-7.2 | - | - | - |
| | JD | 6.4 | ++ | - | - |
| | YT | 6.4 | ++ | - | - |

(continued)

| Growth time (d) | Strain | pH | NH$_4^+$-N | NO$_2^-$-N | NO$_3^-$-N |
|---|---|---|---|---|---|
| 19 | CK | 7.0-7.2 | - | - | - |
| | JD | 7.0-7.2 | + | - | + |
| | YT | 7.0-7.2 | + | - | + |
| 25 | CK | 7.0-7.2 | - | - | - |
| | JD | 6.7-7.0 | + | - | - |
| | YT | 6.7-7.0 | + | - | - |

### 2.1.2 Growth conditions on nitrogen fixation plate

As can be seen from FIG. 3a and Table 4a, *Mycobacterium sp.* strains YT and JD grew slowly in liquid medium, both strains had accumulation of NH$_4^+$-N, but the accumulation amount was very low. Since the bacterium had surface growth characteristics, it was not suitable to obtain a large number of cells by liquid culture. The invention further developed a nitrogen fixation plate experiment.

FIG. 3b (strain YT on the left side, and strain JD on the right side) showed that two strains YT and JD cultured in the liquid medium were lineated and inoculated on a nitrogen-free agar medium plate, after 11 days of culture, the two strains grew normally on the plate with single colony morphology, which indicated on the one hand that the liquid medium and the inoculated strains were not contaminated, and on the other hand that the turbidity phenomenon (FIG. 3a) in the growth experiment of the strains in the liquid medium was caused by normal growth performance of the strains, rather than apoptosis of microorganisms.

FIG. 3c showed conditions of strains YT (left) and JD (right) grown on nitrogen fixation plates for 7 days, which indicated that both strains grew on the plates, and a milky-white stain was visible to the naked eye. After growth for 29 days, the two strains further grew, the single colony was clearly visible, but the bacterial quantity was small, and the growth was still slow.

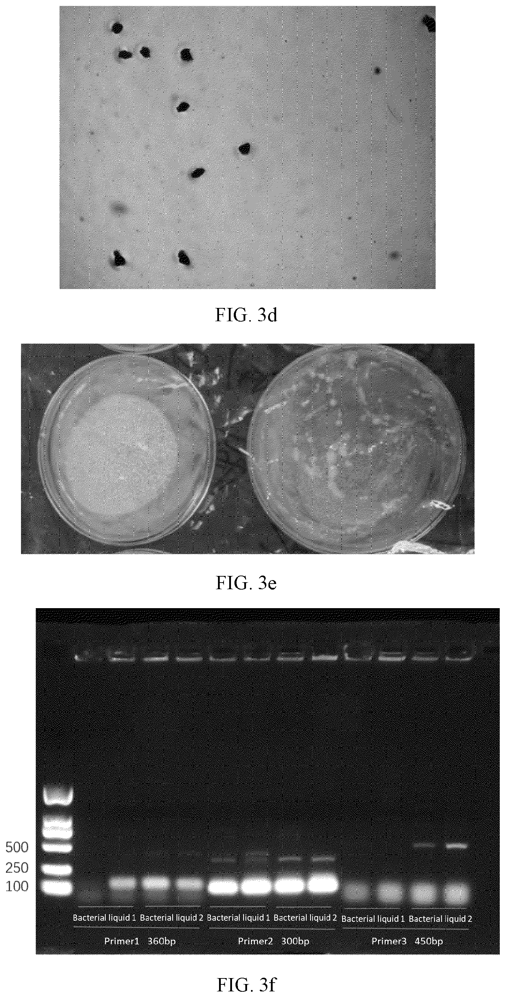

FIG. 3d showed JD thallus cell morphology after cultivation on the nitrogen fixation plate for more than 40 days (Nikon stereoscope, 100-fold magnification).

In order to promote growth of the bacterial strains on the nitrogen fixation plate and obtain a large amount of growths to meet the requirements of research and detection (monoclone was not needed), the bacteria on the nitrogen fixation plate shown in FIG. 3c were scraped by a coating rod and coated on the nitrogen fixation plate again in the research, but the growth was still not ideal; resting cells was then adopted and centrifuged, a supernatant was removed, the bacterial colonies were directly scraped and coated on a LB plate, and the bacterial colonies were picked as many as possible during coating. FIG. 3e showed the culture condition of bacteria after centrifugation of resting cells, a supernatant was removed, the bacterial colonies were directly scraped and coated on the nitrogen fixation plate (strain YT on the left side, and strain JD on the right side), and it showed that both strains grew obviously on the nitrogen fixation plate after the above treatments.

### 2.1.3 Detection of nitrogen fixation gene

Three pairs of primers with nitrogen fixation ferritin were used for PCR amplification, all three primers can be amplified, and particularly, the amplification result of the second primer was more obvious, the result was shown in FIG. 3f, it indicated that both of the strain YT (bacterial liquid 1) and strain JD (bacterial liquid 2) had the nitrogen fixation capability on a molecular level.

### 2.2 Authigenic nitrogen fixation in the presence of nitrogen in the form of chemistry

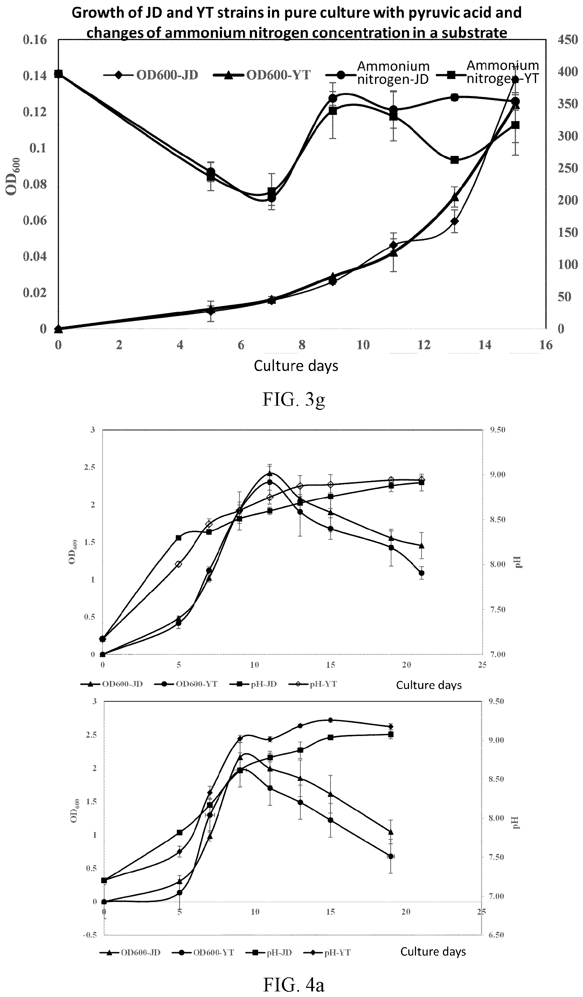

In the presence of nitrogen in the form of chemistry (2.0g/L ammonium sulfate), and pyruvic acid used as a carbon source, the strains JD and YT of *Mycobacterium sp.* of the invention grew normally; when the two strains grew, the concentration of ammonia nitrogen in the medium initially had a rapid decrease, after 7 days of growth, the concentration of ammonia nitrogen fell to a lower level, it rapidly exhibited an increase, after 9 days of growth, a decrease in the concentration of ammonia nitrogen again was observed, refer to FIG. 3g. As can be seen, the distinct nitrogen fixation phenomenon exhibited after 7-9 days of growth.

As shown in the following table, during the nitrogen fixation process of *Mycobacterium sp.* of the present invention, the culture system inoculated with strain JD had a decrease in pyruvic acid of 6.05mg and an increase in ammonia nitrogen of 15.49mg, i.e., 2.56mg of nitrogen was fixed (ammonia was secreted) per 1mg of pyruvic acid consumed by the strain JD; the culture system inoculated with the strain YT had a decrease in pyruvic acid of 7.06mg and an increase in ammonia nitrogen of 15.098mg, i.e., 2.13mg of nitrogen was fixed (ammonia was secreted) per 1mg of pyruvic acid consumed by the strain YT. An average 2.13mg of nitrogen was fixed (ammonia was secreted) per 1mg of pyruvic acid consumed by the strains JD and YT.

**Table 4b Change in the concentrations of $NH_4^+$-N and pyruvic acid in the culture medium <u>between 7 days and 9 days of growth of *Mycobacterium sp.*</u>**

| Tested strains | Pyruvic acid concentration decrease (mg/L) | Ammonia nitrogen concentration increase (mg/L) | Culture medium volume (ml) |
|---|---|---|---|
| JD | 60.5 | 154.9 | 100 |
| YT | 70.6 | 150.98 | 100 |

### 2.3 Nitrogen fixation activity

An average of the ethylene generation rates of strains YT and JD were 0.827±0.552 nmol/mg protein/h, 0.502 ±0.337 nmol/mg protein/h, respectively, as analyzed by the experimental results for the nitrogen fixation activity of strains YT and JD.

### 2.4 Comparison of the nitrogen fixation capability of *Mycobacterium sp.* of the invention, typical nitrogen fixation bacterium and artificial nitrogen fixation

### 2.4.1 Energy consumption of nitrogen fixation of typical bacterium genera/species

(1) Nitrogen fixation bacterium genus *(Azotobocter):* the nitrogen fixation bacteria, represented by round brown nitrogen fixation bacterium *(Azotobocter chroococcum),* fixed 10-20mg of nitrogen per 1g of glucose consumed (ZHU Zhao-liang *et al.,* 1992). In the tricarboxylic acid cycle process, a total of 15 ATP molecules were produced by 1 molecule of pyruvic acid if calculated from the beginning of dehydrogenation, and 2 molecules of pyruvic acid were produced per glucose molecule, a total of 36-38 ATP molecules were produced per glucose molecule in 3 stages of glycolysis, tricarboxylic acid cycle and oxidative phosphorylation (SHEN Tong, *et al.,* 2000), therefore, with respect to ATP produced in the tricarboxylic acid cycle alone: 1mol of glucose corresponded to 2mol of pyruvic acid and corresponded to 30mol of ATP. As a result, for nitrogen fixation bacterium genus, 10-20mg of nitrogen were fixed per 1g of glucose consumed, it was corresponding to 10-20mg of nitrogen fixed per 0.978g of pyruvic acid, i.e., 0.0102-0.0204mg of N was fixed per 1mg of pyruvic acid consumed.

(2) *Clostridium pasteurianum: Clostridium pasteurianum* fixed 1.5-7.0mg of Nitrogen per 1g of glucose consumed (ZHU Zhao-liang *et al.,* 1992), based on the above analysis, in terms of ATP produced from the tricarboxylic acid cycle, 1g of glucose corresponded to 0.978g of pyruvic acid, it was corresponding to 1.5-7.0mg of nitrogen fixed per 0.978g of pyruvic acid, i.e., 0.001534-0.007157mg of nitrogen was fixed per 1mg of pyruvic acid consumed.

(3) *Klebsiella pneumoniae:* the basic enzymatic reactions of nitrogen fixation enzymes represented by *Klebsiella pneumoniae* were as follows: $N_2 + 8H^+ + 8e^- + 16$ Mg ATP $\rightarrow 2NH_3 + H_2 + 16$MgADP + 16Pi (HAN Bin, *et al.,* 2009), 1mol of $N_2$ was corresponding to 16mol of ATP. Since 1 molecule of pyruvic acid generated a total of 15 molecules of ATP in the tricarboxylic acid cycle (SHEN Tong, *et al.,* 2000), 16mol of produced ATP shall be corresponding to 1.07mol of pyruvic acid. That is, at least 1.07mol of pyruvic acid was required for 1mol of $N_2$ fixed by *Klebsiella pneumoniae* to generate 16mol of ATP, and based on conversion, 0.298g of Nitrogen can be fixed per 1g of pyruvic acid consumed by *Klebsiella pneumoniae,* that is, 0.298mg of Nitrogen can be fixed per 1mg of pyruvic acid consumed.

(4) *Streptomyces thermoautotrophicus:* the basic enzymatic reaction of the *Streptomyces thermoautotrophicus* nitrogen fixation enzyme was as follows: $N_2 + 8H^+ + 8e^- + 4$-12 MgATP $\rightarrow 2NH_3 + H_2 + 4$-12MgADP + 4-12Pi (Markus Ribbe *et al.,* 1997). Since 1 molecule of pyruvic acid generated a total of 15 molecules of ATP in the tricarboxylic acid cycle (SHEN Tong, *et al.,* 2000), for *Streptomyces thermoautotrophicus,* at least 4-12mol of ATP produced from 0.27-0.8mol of pyruvic acid was required for fixing 1mol of $N_2$, and based on conversion, 0.40-1.18g of Nitrogen can be fixed per 1g of pyruvic acid consumed by *Streptomyces thermoautotrophicus,* that is, 0.4-1.18mg of Nitrogen can be fixed per 1mg of pyruvic acid consumed.

(5) *Mycobacterium sp.* of the invention: the section "Authigenic nitrogen fixation in the presence of compound nitrogen" of the invention investigated and calculated the nitrogen fixation energy consumption of strains JD and YT, i.e., 2.56mg of nitrogen was fixed (ammonia was secreted) per 1mg of pyruvic acid consumed by the strain JD, 2.13mg of nitrogen was fixed (ammonia was secreted) per 1mg of pyruvic acid consumed by the strain YT, and an average 2.13mg of nitrogen was fixed (ammonia was secreted) per 1mg of pyruvic acid consumed by the strains JD and YT.

**2.4.2 Energy consumption of artificial nitrogen fixation**

According to the data in the artificially synthetic ammonia industry at present, the energy consumption for synthesizing 1 ton of ammonia ($NH_3$, molecular weight 17.03) was $48.2 \times 10^6$ kJ, and the energy consumption for synthesizing 1 ton of urea ($CO(NH_2)_2$, molecular weight 60.06) was $33.2 \times 10^6$ kJ. When calculated based on the amount of fixed nitrogen, the energy consumption for fixing 0.822g of nitrogen in the artificially synthetic ammonia industry was 48.2kJ, which was corresponding to the energy released by hydrolysis of 1.58 molecules of ATP (30.54kJ/mol of heat was released by ATP hydrolysis to yield ADP), which was corresponding to the amount of ATP generated by 9.28g pyruvic acid (calculated according to 1 molecule of pyruvic acid generated 15 molecules of ATP) through the tricarboxylic acid cycle, as a result, the energy consumption in the artificially synthetic ammonia industry can be converted into that 0.08858mg of nitrogen was fixed per 1mg of consumed pyruvic acid.

During the synthesis process of urea, the energy of 33.2kJ was required for fixing 0.466g of Nitrogen, based on the conversion result of nitrogen in urea, it was equivalent to the energy generated by hydrolysis of 1.087 molecules of ATP, which was equivalent to the amount of ATP generated by 6.38g of pyruvic acid through the tricarboxylic acid cycle, such that the energy consumption in the artificial synthesis of urea can be converted that 0.073mg of nitrogen was fixed per 1mg of consumed pyruvic acid.

**2.4.3 Comparison of the nitrogen fixation capabilities of different bacterium species/genera and artificial nitrogen fixation**

Based on the energy consumption analysis of several typical nitrogen fixation bacterium species/genera and artificial nitrogen fixation, the sequences of nitrogen fixation capacities were as follows: *Mycobacterium sp.* of the invention > *Streptomyces thermoautotrophicus* > *Klebsiella pneumoniae* > Artificial nitrogen fixation (artificially synthetic ammonia and artificially synthetic urea) > *Azotobocter* represented by *Azotobocter chroococcum* > *Clostridium pasteurianum,* based on the amount of nitrogen fixation per unit weight of consumed pyruvic acid. The nitrogen fixation capability of *Mycobacterium sp.* in the invention was 2-6 times that of *Streptomyces thermoautotrophicus,* 8 times that of *Klebsiella pneumoniae,* 115-230 times that of the nitrogen fixation bacterium genera, and 328-1,532 times that of *Clostridium pasteurianum.* In terms of artificial nitrogen fixation (artificially synthetic ammonia and artificially synthetic ammonia), the nitrogen fixation capacity of *Mycobacterium sp.* of the invention was about 29 times its nitrogen fixation capacity, as shown in the following Table.

**Table 4c Comparison of the nitrogen fixation capabilities of *Mycobacterium sp.* of the invention with typical nitrogen fixation bacterium genera/species and artificial nitrogen fixation**

| Typical bacterium species/genera/mode | Latin name/mode | Nitrogen fixation capability (amount of nitrogen fixation in mg per 1mg of pyruvic acid consumed: mg nitrogen/mg pyruvic acid) |
|---|---|---|
| *Azotobocter* represented by *Azotobocter chroococcum* | *Azotobocter chroococcum* | 0.0102-0.0204 |
| *Clostridium pasteurianum* | *Clostridium pasteurianum* | 0.001534-0.007157 |
| *Klebsiella pneumoniae* | *Klebsiella pneumoniae* | 0.298 |
| *Streptomyces thermoautotrophicus* | *Streptomyces thermoautotrophicus* | 0.4-1.18 |
| *Mycobacterium sp.* of the invention | *Mycobibacterium guanghaopengii* sp.nov. | 2.13-2.56 |

(continued)

| Typical bacterium species/genera/mode | Latin name/mode | Nitrogen fixation capability (amount of nitrogen fixation in mg per 1mg of pyruvic acid consumed: mg nitrogen/mg pyruvic acid) |
|---|---|---|
| Artificial nitrogen fixation | Artificially synthetic ammonia | 0.08858 |
| | Artificially synthetic urea | 0.073 |

### 3. Conclusion

(1) When the strains JD and YT were cultivated in a liquid medium, which allowed normal growth of the two strains and accumulation of ammonia nitrogen in the culture medium; the two strains grew obviously when the strains JD and YT were cultivated by using a nitrogen fixation plate culture medium; the existence of the gene with nitrogen fixation capacity was verified by PCR amplification of both strains using 3 pairs of primers with nitrogenase ferritin. The *Mycobacterium sp.* of the invention had a nitrogen fixation effect in the absence of compound nitrogen.
(2) The *Mycobacterium sp.* of the invention exhibited a strong nitrogen fixation effect in the presence of a nitrogen in chemistry (ammonium sulfate), and an average of 2.35mg of nitrogen was fixed (ammonia was secreted) per 1mg of consumed pyruvic acid.
(3) The average values of the ethylene production rates of the two strains were 0.827 nmol/mg protein/h and 0.502 nmol/mg protein/h, respectively. The *Mycobacterium sp.* of the invention exhibited obvious nitrogen fixation activity.
(4) The *Mycobacterium sp.* of the invention has strong nitrogen fixation capability, which was 2-6 times that of *Streptomyces thermoautotrophicus,* 8 times that of *Klebsiella pneumoniae,* 115-230 times that of *Azotobocter,* and 328-1,532 times that of *Clostridium pasteurianum.* In terms of artificial nitrogen fixation (artificially synthetic ammonia and artificially synthetic ammonia), the nitrogen fixation capacity of *Mycobacterium sp.* of the invention was about 29 times its nitrogen fixation capacity.

### Example 2

**Growth and nitrification activity of *Mycobacterium sp.* of the invention in pure culture based on pyruvic acid as a carbon source**

#### 1. Materials and methods

**1.1 Tested bacteria:** PBS-JD/YT bacterial liquid.

**1.2 Culture medium and indicator determination**

**(1) Medium**

The modified Stephenson's medium with pyruvic acid as a carbon source, wherein the concentration of pyruvic acid in the medium was 0.06M and 0.1M respectively. 1M NaOH solution was used to fine-tune the desired pH.

**(2) Method and indicators**

1ml of PBS-JD/YT bacterial liquid was inoculated in a prepared and sterilized liquid culture medium, shake cultivation was carried out at the temperature of 28°C and the rotating speed of 180rpm, each of the growth amount, the pH and the $NH_4^+$-N, $NO_2^-$-N, $NO_x^-$-N contents of the thallus was periodically measured.

#### 2. Results

As shown in FIG. 4a (upper figure illustrated 0.06M pyruvic acid, lower figure illustrated 0.1M pyruvic acid) and the following table, when pure culture was performed with 0.06M pyruvic acid as a carbon source, after 24d of culture, the $OD_{600}$ average values of the strains JD and YT were 1.84 and 1.62, respectively, in the meanwhile, the $NH_4^+$-N

concentration in the culture solution was reduced from an initial value of 424mg/L to 93.08mg/L and 66.87mg/L, respectively; accumulation of $NO_2^-$-N occurred in culture of strains JD and YT, the average concentration reached 139.90mg/L and 96.31mg/L respectively. Experiments showed that when 0.06M pyruvic acid was used as a carbon source for pure culture, both YT and JD strains can grow and generate strong nitrification. When pure culture was carried out by taking 0.1M pyruvic acid as a carbon source, after 33d of culture, both of the $OD_{600}$ average values of the strains JD and YT were 2.04, at the moment, the $NH_4^+$-N content in the culture solution almost disappeared, the average concentrations were only 4.52mg/L and 3.88mg/L, and $NO_2^-$-N concentration was accumulated to 177.92mg/L and 161.14mg/L. Experiments showed that when 0.1M pyruvic acid was used as a carbon source for pure culture, both YT and JD strains can grow and generate strong nitrification.

**Table 5 Growth and nitrification activity of strains YT and JD in pure culture with different concentrations of pyruvic acid as a carbon sources**

| Pyruvic acid concentration (M) | Strain | $OD_{600}$(nm) | | $NH_4^+$-N (mg/L) | | $NO_2^-$-N (mg/L) | | $NO_x^-$-N (mg/L) | |
|---|---|---|---|---|---|---|---|---|---|
| | | Average | SD | Average | SD | Average | SD | Average | SD |
| CK | | 0 | | 424.0 | | 0 | | 0 | |
| 0.06 | JD | 1.84 | 0.07 | 93.08 | 19.76 | 139.90 | 36.31 | 219.04 | 57.54 |
| | YT | 1.62 | 0.13 | 66.87 | 16.30 | 96.31 | 23.30 | 152.39 | 36.67 |
| 0.1 | JD | 2.04 | 0.17 | 4.52 | 2.40 | 177.92 | 19.62 | 287.88 | 31.95 |
| | YT | 2.04 | 0.30 | 3.88 | 3.74 | 161.14 | 30.69 | 260.24 | 50.88 |

**3. Conclusion:** when pure culture was carried out by taking pyruvic acid with different concentrations as a carbon sources, the test strains can grow normally and generate strong ammoxidation effect, and the nitrification activity was obvious.

**Example 3**

**Fixation of $CO_2$ by *Mycobacterium sp.* of the invention**

**1. Materials and methods**

**1.1 Test bacteria:** PBS-JD/YT bacterial liquid.

**1.2 Medium and culture system:**

A. Modified Stephenson's medium
B. Pyruvic acid was used as a carbon source. Firstly, a 2M pyruvic acid solution (88.0701 g of pyruvic acid was weighed, 400mL of distilled water was added and stirred, 40g of solid NaOH was further added, the solution was cooled, the volume was then determined to be 500mL, the pH value was adjusted to 7.17) was prepared, and the solution was separately sterilized for later use.

Before use, 5ml of 2M pyruvic acid solution was initially added into 94ml of modified Stephenson culture medium.
1ml of PBS-JD/YT bacterial liquid was inoculated into the culture medium, and the total volume of the culture system was 100 ml. The concentration of pyruvic acid in the culture medium was designed at approximately 0.1M.
**1.3 Culture conditions:** shake cultivation was carried out at the temperature of 28°C, the rotating speed of 180rpm, and $CO_2$ concentration of 5%. The cultivation was continued for 21 days.
**1.4 Indicator determination:** the growth amount and pH of the thallus were periodically measured; the supernatant was extracted to measure the contents of $NH_4^+$-N, $NO_2^-$-N, $NO_x^-$-N; the content of pyruvic acid in the culture medium was periodically measured, and the content of pyruvic acid was detected by using High Performance Liquid Chromatography (HPLC).
After the culture was finished, centrifuged for 5min by using a high-speed centrifuge with a rotating speed of 10,000 rpm at 4°C, the obtained thallus was freezed and then dried in vacuum, the dry weight and the carbon and nitrogen contents of the thallus were measured. The fixed amount of $CO_2$ was estimated by analyzing the change of carbon and nitrogen contents in the culture system (cmedium supernatant, thallus).

## 2. Results

### 2.1 Change of pyruvic acid concentration in the culture system

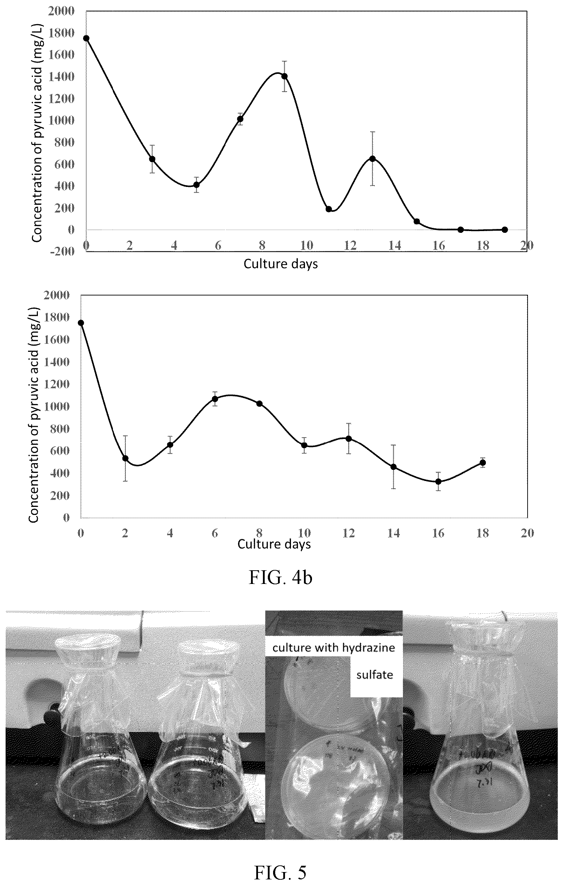

As shown in FIG. 4b (upper figure illustrated the strain JD, lower figure illustrated the strain YT), under the high concentration $CO_2$ culture condition, pure culture of strains JD and YT was performed with pyruvic acid as a carbon source, both of the concentrations of pyruvic acid in the culture medium of both strains showed the changes of initially decreasing, then increasing and decreasing again, which reflected that the organism independently completed the metabolic processes of forward TCA cycle and reverse TCA cycle. In the first stage, the strain performed forward TCA cycle, consumed pyruvic acid, generated energy for accumulating arginine metabolic intermediate product and prepared for ammonia oxidation; in the second stage, the bacterial strain started ammonia oxidation to carry out arginine metabolism, energy generated by metabolism was used for pushing the reverse TCA cycle, pyruvic acid was accumulated to prepare the anabolism; in the third stage, the bacterial strain carried out anabolism, consumed pyruvic acid, completed gluconeogenesis and realized growth. Both strains fixed $CO_2$ by the reverse TCA cycle pathway.

### 2.2 Analysis on consumption of carbon and nitrogen in the metabolic process and the carbon fixation quantity thereof

(1) Carbon/nitrogen ratio of thallus

The experimental results showed that the carbon/nitrogen ratio of the *Mycobacterium sp.* thallus in the invention was 5.65:1, which was not significantly different from the carbon/nitrogen ratio (empirical value was 5: 1) of the ordinary heterotrophic bacteria thallus.

(2) Carbon and nitrogen consumption in thallus anabolism

The consumption of carbon and nitrogen during the anabolism of bacterial thallus was classified into two types, one type is the consumption of substances for anabolism, and the other type is the consumption of energy for anabolism. The substances and energy required for the anabolism of heterotrophic bacteria were generally derived from organic carbon sources and nitrogen sources in the environment (the main sources were usually derived from decomposition of other life). The total carbon/nitrogen mass ratio was generally around 25:1 as a matter of experience, wherein the substance of carbon source for anabolism contributes 20% of the total carbon source, and the energy for generating anabolism contributed 80% of the total carbon source.

(3) The $CO_2$ emission reduction effect of the *Mycobacterium sp.* of the invention

The difference between the *Mycobacterium sp.* of the invention and the ordinary heterotrophic bacteria was that the ordinary heterotrophic bacteria used organic carbon sources for obtaining energy through glycolysis and TCA cycle (simultaneously release $CO_2$), while the *Mycobacterium sp.* of the invention can obtain energy through glycolysis and TCA cycle of the organic carbon sources, it can also obtain energy required for assimilation through nitrogen metabolic pathways such as ammonia oxidation and nitrification, so as to promote reverse TCA cycle and assimilate $CO_2$. Therefore, when the *Mycobacterium sp.* of the invention was used for completing the anabolism of one part of nitrogen and five parts of carbon, it can reduce twenty parts of organic carbon consumption required for completing the TCA cycle, it can also utilize the energy generated by nitrogen metabolism to reversely drive the TCA cycle to fix five parts of $CO_2$ so as to obtain a substance required by anabolism (i.e., organic carbon source). Therefore, the consumption of one part of nitrogen for assimilation by the *Mycobacterium sp.* of the invention was corresponding to reduced emissions of 25 parts of $CO_2$ compared to the ordinary heterotrophic bacteria.

(4) The equivalent of fixed $CO_2$ was estimated from substrate changes in the experiment

In a matrix solution consisting of 424mg of $NH_4^+$-N and 0.1mol of pyruvic acid, after 21 days of continuous culture of strain JD, 4.52mg $NH_4^+$-N, 287.8mg $NO_x^-$-N and less than 0.1mg organic carbon were remained. Therefore, the mass of nitrogen element used for assimilation growth was 131.68mg. When calculated according to the molar ratio of 25:1, the mass of carbon element consumption reduced by assimilating 131.68mg of nitrogen element was 2,822mg, which was converted into 10.35g by mass of $CO_2$ emission reduction. Similarly, after 21 days of continuous culture of strain YT, 3.88mg $NH_4^+$-N, 260.6mg $NO_x^-$-N and less than 0.1mg organic carbon were remained. Therefore, the mass of nitrogen element used for assimilation growth was 159.52mg. When calculated according to the molar ratio of 25:1, the mass of carbon element consumption reduced by assimilating 159.52mg of nitrogen element was 3,418mg, which was converted into 12.53g by mass of $CO_2$ emission reduction.

## 3. Conclusion

(1) The *Mycobacterium sp.* of the invention presented a reversed TCA cycle pathway.

(2) The *Mycobacterium sp.* of the invention fixed $CO_2$ through reverse TCA cycle, and has the function of $CO_2$ emission reduction.

**Example 4**

**Growth and nitrification activity of *Mycobacterium sp.* of the invention cultivated with hydrazine sulfate as a nitrogen source**

**1. Materials and methods**

**1.1 Tested bacteria:** resting cells of strains JD and YT obtained from Preparation Example 1. That is, the cultured bacteria were centrifuged to remove the supernatant, washed repeatedly with physiological saline, placed in a refrigerator at 4°C until color was not displayed for $NO_2^-$-N after the dripping with Griess reagent, and then lineated to confirm that the bacteria were pure. During inoculation, the cultured bacteria were centrifuged to remove the supernatant and suspended with a culture medium, and then cultured in a shaking flask.

**1.2 Culture system**

**(1) Medium**

A. Modified Stephenson medium with hydrazine sulfate as nitrogen source
2.0g of $(NH_4)_2SO_4$ in the modified Stephenson medium was replaced with equal nitrogen amount of $N_2H_4 \cdot H_2SO_4$, i.e. 1.97g of $N_2H_4 \cdot H_2SO_4$, with the other ingredients and treatments unchanged.
B. Pyruvic acid was used as a carbon source: 6ml of a separately sterilized pyruvic acid solution with a concentration of 1.0M was added to 100ml of culture medium A, such that the designed concentration of pyruvic acid in the culture medium was about 0.06M. The pH was adjusted by using NaOH solution with a concentration of 1M.

**(2) Culture system**

1ml of the resting cell broth was inoculated into the above culture medium, shake cultivation was carried out at the temperature of 28°C and the rotating speed of 180rpm.
**(3) Indicator measurement:** the growth amount $OD_{600}$, the pH and the $NH_4^+$-N, $NO_2^-$-N, $NO_x^-$-N contents of the thallus in the culture solution were measured.

**2. Results**

The tested strain was inoculated in a liquid culture medium with hydrazine sulfate as a nitrogen source, and the culture solution was clarified at the moment (a left picture of FIG. 5); after 7 days of growth, the bacterial liquid growing in hydrazine sulfate was inoculated on a solid plate, after 14 days of growth on the solid plate, it was obviously discovered that the test strain normally grew on the plate with hydrazine sulfate as a nitrogen source, however, when the qualitative determination of $NO_2^-$-N was carried out, the plate did not display color (a middle picture of FIG. 5); after 38d growth in the liquid medium, the culture medium became turbid and the strain grew when observed with naked eye (a right picture of FIG. 5), the OD values of two strains YT and JD at the moment were 1.09 and 1.12 respectively (Table 6b).
During the culture period, the change of $NH_4^+$-N, $NO_2^-$-N, $NO_3^-$-N contents in the culture solution was periodically analyzed, as can be seen by combining the qualitative analysis result (Table 6a) and the quantitative determination result (Table 6b), the formation of $NH_4^+$-N and the accumulation of $NO_2^-$-N, $NO_3^-$-N, $NO_x^-$-N were not implemented in the culture solution; at the same time, the pH increased significantly, i.e., the pH was 6.4 at the beginning, and reached 7.7-8.0 after 25 days of growth. Therefore, the bacterium can grow when hydrazine sulfate was used as a substrate, but nitrite and nitrate were not generated.

**Table 6a Content change of $NH_4^+$-N, $NO_2^-$-N, $NO_3^-$-N in culture solution when strains YT and JD were in pure culture with hydrazine sulfate as nitrogen source**

| Strain | Growth days (d) | $NH_4^+$-N | $NO_2^-$-N | $NO_3^-$-N |
|--------|-----------------|------------|------------|------------|
| YT/JD | 0 | - | - | - |
| YT/JD | 11 | - | - | - |
| YT/JD | 25 | - | - | - |
| YT/JD | 32 | - | - | - |
| YT/JD | 39 | - | - | - |

**Table 6b Growth and nitrification activity of strains YT and JD cultured using hydrazine sulfate as nitrogen source**

| Strain | $OD_{600}$ (600nm) | | $NH_4^+$-N (mg/L) | $NO_2^-$-N (mg/L) | $NO_x^-$-N (mg/L) |
|--------|---------|------|-------------------|-------------------|-------------------|
| | Average | SD | | | |
| YT | 1.09 | 0.03 | $-0.135\pm0.033$ | $0.004\pm0.001$ | $-0.013\pm0.028$ |
| JD | 1.12 | 0.08 | $-0.156\pm0.005$ | $0.006\pm0.002$ | $-0.021\pm0.021$ |

3. Conclusion: the *Mycobacterium sp.* of the invention can grow by using hydrazine sulfate as nitrogen source, but did not convert into ammonium nitrogen, and did not produce nitrite and nitrate, thus it was completely short of nitrification activity; the strain was possible to generate hydroxylamine by utilizing a metabolic pathway of hydrazine sulfate dehydrogenation.

**Example 5**

**Growth and nitrification activity of *Mycobacterium sp.* of the invention cultured with hydroxylamine as nitrogen source**

**1. Materials and methods**

**1.1 Medium**

(1) Fe ion-containing culture medium (g/L): $NaH_2PO_4$ 0.25, $K_2HPO_4$ 0.75, $MgSO_4 \cdot 7H_2O$ 0.03, $MnSO_4 \cdot H_2O$ 0.01, $FeSO_4 \cdot 7H_2O$ 0.01, before sterilization, the pH of culture medium was adjusted to 7.0-7.2 by using sulfuric acid and sodium hydroxide.
(2) Fe ion-free culture medium (g/L): $NaH_2PO_4$ 0.25, $K_2HPO_4$ 0.75, $MgSO_4 \cdot 7H_2O$ 0.03, $MnSO_4 \cdot H_2O$ 0.01, before sterilization, the pH of culture medium was adjusted to 7.0-7.2 by using sulfuric acid and sodium hydroxide.

Two nitrogen sources were set, namely 0.2mM and 1.8mM of hydroxylamine hydrochloride respectively; carbon sources were 0 and 0.06M pyruvic acid.

**1.2 Culture**

The resting cells of strains JD and YT, that is, the cultured strains JD and YT were centrifuged to remove the supernatant, washed repeatedly with physiological saline, placed in a refrigerator at 4°C until color was not displayed for $NO_2^-$-N after the dripping with Griess reagent, and then lineated to confirm that the bacteria were pure. During inoculation, the cultured bacteria were suspended with a culture medium and then cultured in a shaking flask.

### 1.3 Culture conditions

The cultivation was performed at a shaking table with a rotational speed of 180rpm at a temperature condition of 28°C for 21 days.

### 1.4 Culture System and indicator determination

The non-inoculated strains were used as a control group, two types of non-carbon source and 0.06M pyruvic acid were set according to the following Table at two nitrogen source concentrations of 0.2mM hydroxylamine and 1.8mM hydroxylamine, and the culture was performed by using a Fe-containing medium and a Fe-free medium, respectively.

**Table 7a Culture system setup**

| Strain | 0.2mM hydroxylamine | | | | 1.8mM hydroxylamine | | | |
|---|---|---|---|---|---|---|---|---|
| | 0 pyruvic acid | | 0.06M pyruvic acid | | 0 pyruvic acid | | 0.06M pyruvic acid | |
| CK | +Fe | -Fe | +Fe | -Fe | +Fe | -Fe | +Fe | -Fe |
| JD | +Fe | -Fe | +Fe | -Fe | +Fe | -Fe | +Fe | -Fe |
| YT | +Fe | -Fe | +Fe | -Fe | +Fe | -Fe | +Fe | -Fe |

After the cultivation was finished, the growth amount ($OD_{600}$) and pH of the thallus in the culture solution were measured respectively, the thallus were freezed and centrifuged (4°C, 10,000rpm), the contents of $NH_4^+$-N, $NO_2^-$-N, $NO_3^-$-N in the supernatant were measured respectively, the thallus after centrifugation was quickly freezed by using liquid nitrogen, and then storing at an environment of -80°C, the transcriptome sequencing was performed. Each measurement had 3 replicates.

### 1.5 Culture transcriptome sequencing

The sequencing was classified as follows according to the following Table: (1) w-0: resting cells subjected to starvation treatment; (2) w-1: 0.2mM hydroxylamine hydrochloride + 0M pyruvic acid; (3) w-2: 0.2mM hydroxylamine hydrochloride + 0.06M pyruvic acid.

**Table 7b Culture transcriptome sequencing**

| Genes | W-0 | W-1 | W-2 | type | Func |
|---|---|---|---|---|---|
| orf00266 | 34.013 | 460.507 | 232.566 | up | short-chain dehydrogenase/reductase SDR |
| orf00793 | 213.975 | 2419.729 | 5252.699 | up | short-chain dehydrogenase |
| orf01696 | 12.538 | 118.403 | 99.649 | up | short chain dehydrogenase |
| orf04825 | 74.438 | 2394.107 | 252.975 | up | Dehydrogenase |
| orf05051 | 0 | 21.956 | 8.983 | up | short-chain dehydrogenase |

### 2. Results

### 2.1 Growth and nitrification activity of *Mycobacterium sp.* of the invention in pure culture with hydroxylamine as nitrogen source

As can be seen from Table 7c, the strains JD and YT can grow in both the iron-containing medium and the iron-free medium under pure culture conditions by using hydroxylamine hydrochloride as a nitrogen source (0.2mM and 1.8mM) and pyruvic acid as a carbon source (with a concentration of 0.06M), both of the strains did not exhibit nitrification activity based on the analysis on changes in $NH_4^+$-N, $NO_2^-$-N, $NO_3^-$-N concentration.

**Table 7c Comparison of growth and nitrification activity of strains in the pure culture with hydroxylamine +0.06M pyruvic acid**

| Hydroxylamine concentration (mM) | Strain | Iron-containing/-free medium | $OD_{600}$ | $NH_4^+$-N (mg/L) | $NO_2^-$-N (mg/L) | $NO_3^-$-N (mg/L) |
|---|---|---|---|---|---|---|
| 0.2 | CK | Iron-containing | 0 | 1.26 | 0.04 | 0.02 |
| | | Iron-free | 0 | 1.44 | 0.04 | 0.04 |
| | JD | Iron-containing | 1.76 | 0 | 0.005 | 0 |
| | | Iron-free | 1.82 | 0 | 0.010 | 0 |
| | YT | Iron-containing | 1.85 | 0.01 | 0.010 | 0 |
| | | Iron-free | 1.41 | 0.01 | 0.008 | 0 |
| 1.8 | CK | Iron-containing | 0 | 1.23 | 0.038 | 0.02 |
| | | Iron-free | 0 | 1.41 | 0.039 | 0.02 |
| | JD | Iron-containing | 1.24 | 0.02 | 0.008 | 0 |
| | | Iron-free | 1.5 | 0.23 | 0.006 | 0 |
| | YT | Iron-containing | 1.54 | 0.01 | 0.005 | 0 |
| | | Iron-free | 1.34 | 0.01 | 0.006 | 0 |

Table 7d showed the growth and nitrification activity of two strains JD and YT in pure culture using hydroxylamine hydrochloride as a nitrogen source without the addition of pyruvic acid as carbon source into the culture medium, the strains JD and YT grew in both iron-containing culture medium and iron-free culture media under the hydroxylamine concentrations of 0.2mM and 1.8 mM, and exhibited nitrification activity.

**Table 7d Comparison of growth and nitrification activity of strains in pure culture with hydroxylamine + 0 pyruvic acid**

| Hydroxylamine concentration (mM) | Strain | Iron-containing/-free medium | $OD_{600}$ | $NH_4^+$-N (mg/L) | $NO_2^-$-N (mg/L) | $NO_x^-$-N (mg/L) |
|---|---|---|---|---|---|---|
| 0.2 | CK | With Fe | 0 | 1.29 | 0.04 | 0.04 |
| | | Fe free | 0 | 1.44 | 0.04 | 0.04 |
| | JD | With Fe | 1.66 | 1.28 | 2.55 | 5.57 |
| | | Fe free | 1.79 | 1.45 | 2.48 | 5.13 |
| | YT | With Fe | 1.86 | 1.05 | 2.7 | 5.71 |
| | | Fe free | 1.89 | 1.3 | 2.6 | 5.45 |
| 1.8 | CK | With Fe | 0 | 1.23 | 0.04 | 0.04 |
| | | Fe free | 0 | 1.41 | 0.04 | 0.04 |
| | JD | With Fe | 1.82 | 1.85 | 3.85 | 16.75 |
| | | Fe free | 1.67 | 1.61 | 1.95 | 16.29 |
| | YT | With Fe | 1.93 | 1.8 | 1.10 | 19.48 |
| | | Fe free | 1.79 | 1.91 | 1.88 | 20.86 |

## 2.3 Culture transcriptome sequencing

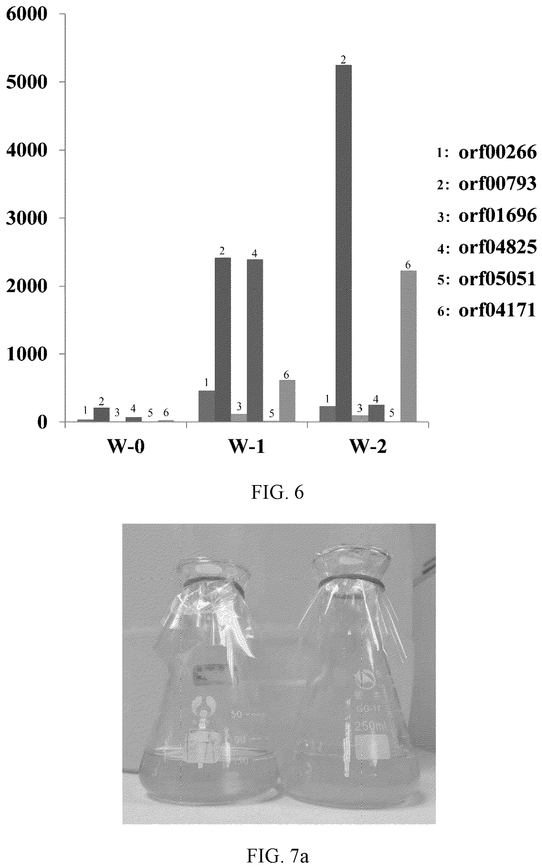

Analysis on the YT culture transcriptome sequencing results clearly indicated that at least 5 dehydrogenases existed in the *Mycobacterium sp.* of the invention, and the expressions of hydroxylamine dehydrogenase in strains were obviously different under the various treatment conditions such as the presence or absence of added pyruvic acid as the carbon source (FIG. 6).

For the five dehydrogenases, the enzyme that grew twice could be associated with the pathway from hydroxylamine to nitrogen and the pathway from hydroxylamine to ammonia, if "hydrogen dehydrogenase" was determined to be the pathway to nitrogen, the other pathway was then determined as the pathway to ammonia; the expressions of other four dehydrogenases may be associated with subsequent oxidation products of hydroxylamine (to nitrous oxide, nitrous oxide to nitric oxide, nitric oxide to nitrous acid, hydroxylamine to hyponitrous acid, nitrous acid to nitric acid).

## 3. Conclusion

(1) Hydroxylamine was used as a nitrogen source, and regardless of the existence or nonexistence of an organic carbon source or iron ions in the culture medium, the *Mycobacterium sp.* of the invention can grow, but the expressions of the nitrification activity exhibited obvious difference.
(2) The results of adding $Fe^{2+}$ and not adding $Fe^{2+}$ are consistent, which indicated that $Fe^{2+}$ did not produce influence and the reaction did not perform along the arginine pathway.
(3) Whether a carbon source was added imposed great influence on the reaction result, after the carbon source was added, nitrogen runaway was most probably generated, other products can be generated, but the nitrous acid was not generated; nitrous acid can be generated without adding a carbon source, and the nitrification activity was shown.
(4) The same results were obtained for two concentrations of hydroxylamine and two different bacterial samples, the above results were enhanced.
(5) Through the culture transcriptome sequencing analysis, at least 5 dehydrogenases existed in the *Mycobacterium sp.* of the invention, and the expressions of hydroxylamine dehydrogenase in strains were obviously different under different treatment conditions such as the presence or absence of added pyruvic acid as the carbon source.

### Example 6

### Hyponitrous acid (sodium trans-hyponitrite) culture experiment

### 1. Materials and methods

### 1.1 Culture

Resting cells of strains JD and YT.

### 1.2 Medium

Ammonium sulfate in the modified Stephenson medium was removed and replaced by the sodium trans-hyponitrite with equal nitrogen content, and other ingredients were unchanged and the pH was natural. The carbon source was pyruvic acid, and the concentration of pyruvic acid in the culture medium was set to 0.06M.

### 1.3 Culture conditions

The cultivation was performed at a shaking table with a rotational speed of 180rpm at the conditions of 28°C and 5% $CO_2$ for about 24 days.

### 1.4 Observation indicators

The growth of thallus was observed, and the contents of $NH_4^+$-N, $NO_2^-$-N, $NO_3^-$-N in the culture solution was qualitatively determined.

### 2. Results

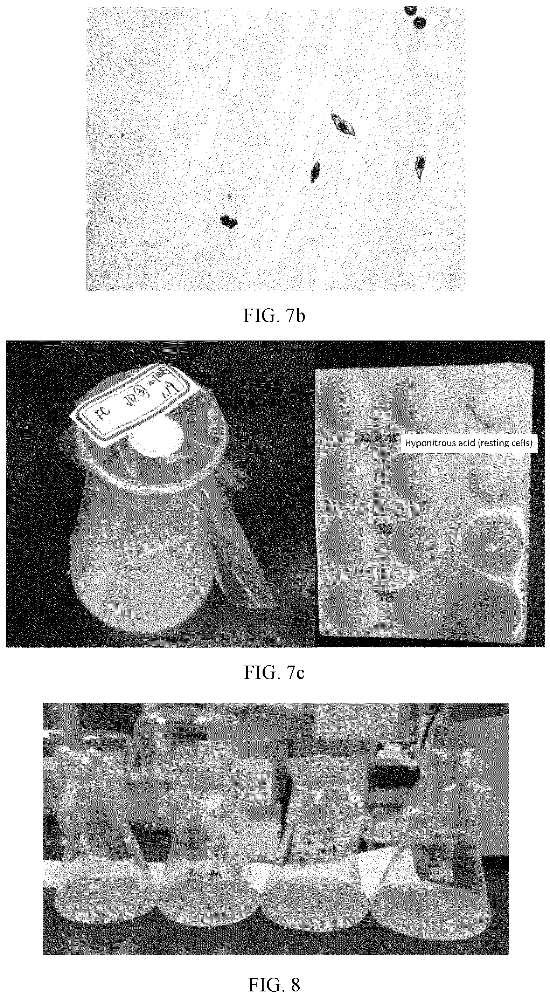

As shown in FIG. 7a, the film-formation was seen indistinctly on the initial surface of the pure culture system using sodium trans-hyponitrite as a nitrogen source, and a small amount of filamentous bacteria was visible to the naked eye, which indicated the growth of thallus, but the growth was not maximized in the continuous culture, probably because that the sodium trans-hyponitrite was strong base and weak acid salt (the pH was stable about 12), if the pH was adjusted to about 7, a large amount of bubbles were generated in the solution, and the sodium trans-hyponitrite was decomposed. The stereoscope observation results of the cell morphology were shown in FIG. 7b.
Through chromogenic titration of relevant indicators in a liquid culture medium in the resting cell culture experiment with sodium trans-hyponitrite as a nitrogen source, the strains YT and JD had slow nitration reaction, and diphenylamine participated reaction and exhibited weakly positive results (FIG. 7c, the $NH_4^+$-N, $NO_2^-$-N, $NO_3^-$-N were sequentially illustrated from left to right in the right picture of FIG. 7c).

## 3. Conclusion

The *Mycobacterium sp.* of the invention was capable of growing by using hyponitrous acid (sodium trans-hyponitrite) and exhibiting nitrification activity.

**Example 7**

**Influence of iron ions on the growth and nitrification activity of *Mycobacterium sp.* of the invention**

1. **Materials and methods**

**1.1 Medium:** PBS-JD and PBS-YT bacterial liquid.

**1.2 Medium**

    A. Modified Stephenson medium
    B. Fe-free modified Stephenson medium: $FeSO_4 \cdot 7H_2O$ in the modified Stephenson medium was removed, and the other ingredients and treatments were unchanged.
    C. modified Stephenson medium without iron and manganese: both $FeSO_4 \cdot 7H_2O$ and $MnSO_4 \cdot H_2O$ were removed from the modified Stephenson medium, and the other ingredients and treatments were unchanged.

Each culture medium used pyruvic acid as carbon source, and the concentration of pyruvic acid in the culture medium was designed to be 0.06M.

**1.3 Influence of iron ions on growth and nitrification performance of strains**

1ml of PBS-JD/YT bacterial liquid was respectively inoculated into the three culture media, namely A + pyruvic acid, B + pyruvic acid and C + pyruvic acid, and was placed in a shaking table with the rotating speed of 180rpm and the constant temperature of 28°C. the continuous culture was performed for 24 days, the $OD_{600}$ value of the bacterial liquid was periodically measured; meanwhile, the culture solution was centrifuged at 4°C for 15min (5,000g), the contents of $NO_2^-$-N, $NH_4^+$-N and $NO_x$-N ($NO_2^-$-N + $NO_3^-$-N) in the supernatant were measured, the ammonia oxidation and nitrification capacity of the test strains were analyzed through calculation of the ammonia oxidation rate and the total nitrification rate.

Each of the three treatment culture media used the culture medium without inoculation of bacterium as the control group (CK), and the experiment was arranged according to the standard that each measurement had 3 replicates.

Calculation of the nitration rates:

$$\text{Total nitrification rate} = [NO_x^-\text{-N}] \times 100/[NH_4^+\text{-N(CK)}]$$

$$\text{Nitrosification rate} = [NO_2^-\text{-N}] \times 100/[NH_4^+\text{-N (CK)}]$$

$$\text{Short-cut nitrification rate} = [NO_2^-\text{-N}] \times 100/[NO_x^-\text{-N}]$$

$$\text{Ammoxidation rate/}NH_4^+\text{-N conversion rate} = (NH_4^+\text{-N(CK)}] - [NH_4^+\text{-N (residue)}]) \times 100/[NH_4^+\text{-N(CK)}]$$

**1.4 Treatment of laboratory ware:** all glassware used in the above experiments was soaked in a nitric acid solution with a nitric acid/water ratio of 1: 1 for 24 hours, fully washed with natural water, and then sufficiently washed with deionized water to eliminate the influence of heavy metal adsorption of experimental apparatus on the experimental results.

## 2. Results

Table 8 illustrated the growth and nitrification activity of the test strains in the iron and manganese-containing basal medium, the iron-free medium, and the iron-free and manganese-free medium. After 21 days of culture, it can be obviously observed that the strains YT and JD grew in the three treatment media; compared with the culture solution without inoculation of bacteria, the culture solution with the inoculated strains became turbid obviously (Table 8 and FIG. 8), and FIG. 8 illustrated four cases from left to right in sequence: 1: JD strain + iron-free culture medium; 2: YT strain + iron-free and manganese-free culture medium; 3: YT strain + iron-free culture medium, 4: JD strain + iron-free and manganese-free culture medium. Compared with the iron-free culture medium and the iron-free and manganese-free culture medium, the $OD_{600}$ value in the basal medium containing iron and manganese was higher, an average of the $OD_{600}$ value of the strains JD and YT were 1.84 and 1.62 respectively.

The two strains JD and YT showed high nitrification activity in basal medium, the average concentration of $NO_2^--N$ in a culture solution after 24 days of growth reached 139.9mg/L and 96.31mg/L respectively, and the average contents of $NO_x^--N$ were 219.04 mg/L and 152.39 mg/L respectively; $NO_2^--N$ and $NO_x^--N$ were not produced in the control group with inoculation of bacteria, iron-free culture medium, and iron-free and manganese-free culture medium, it indicated that the test strains lacked nitrification activity in the absence of iron.

**Table 8 Comparison of growth and nitrification activity of test strains in Fe-containing culture medium and Fe-free culture medium**

| Treatment/medium | Tested strain | $OD_{600}$ value | Ammonia oxidation rate | Total nitrification rate |
|---|---|---|---|---|
| Basal medium (A+ pyruvic acid) | CK1 | 0 | 0 | 0 |
| | JD | 1.84 | 33.0% | 51.7% |
| | YT | 1.62 | 22.7% | 35.9% |
| Fe-free medium (B+ pyruvic acid) | CK2 | 0 | 0 | 0 |
| | JD | 0.707 | 0 | 0 |
| | YT | 0.823 | 0 | 0 |
| Fe-free and manganese-free medium (C+ pyruvic acid) | CK3 | 0 | 0 | 0 |
| | JD | 0.750 | 0 | 0 |
| | YT | 0.499 | 0 | 0 |

## 3. Conclusion:

(1) The test strains can grow in the Fe-free medium, but the tested stains were short of nitrification activity completely.
(2) The tested strains can grow in the Fe-free and manganese-free medium, but the tested stains were short of nitrification activity completely.
(3) Iron ions were important influencing factors for the occurrence of the nitrification activity of the bacteria, but manganese ions had no effect on the nitrification activity of the bacteria.

## Example 8

**Growth and nitrification activity of *Mycobacterium sp.* of the invention in pure culture with arginine as nitrogen source**

### 1. Materials and methods

**1.1 Culture:** PBS-JD/YT bacterial liquid

**1.2 Medium**

A. Modified Stephenson medium with arginine as nitrogen source

Firstly, a nitrogen-free modified Stephenson medium was prepared: the ammonium sulfate in the modified Stephenson medium was removed, and other ingredients were unchanged, the pH was within the range of 7.0-7.2, so that the modified Stephenson culture medium without nitrogen was obtained.

Then an L-arginine mother solution with a certain concentration was prepared: 5.28g L-arginine (with relative molecular mass of 174.20 and water solubility of 148.7g/L at 20°C) was weighed and dissolved in 50ml of deionized water, such that it was completely dissolved, the pH was adjusted to the range of 7.0-7.2, the volume was finally fixed to 80 ml. Since the amino acid L-arginine was prone to decompose under the high-pressure steam sterilization conditions, the sterilization was performed by using a filtration membrane sterilization method.

During culture, 2ml of prepared L-arginine solution was added into each 100ml of modified Stephenson medium without nitrogen, in the meanwhile, the Nitrogen content in the culture system was the same as the nitrogen content when the original nitrogen source was ammonium sulfate (the nitrogen content was 424 mg/L), namely the modified Stephenson culture medium with equal nitrogen content and arginine as the nitrogen source.

B: The carbon source was pyruvic acid. 1M pyruvic acid solutions were respectively prepared, sterilized separately, and finely adjusted the pH to 7.0-7.2 by using NaOH solution with a concentration of 1M before sterilization.

Before use, if it was necessary to add pyruvic acid as carbon source, 6ml of 1M pyruvic acid solution was added into about 100ml of the above-mentioned modified Stephenson culture medium using arginine as a nitrogen source, and the designed concentration of pyruvic acid in the culture system was about 0.06M.

### 1.3 Culture system and indicator determination

2 kinds of treatments were set:

(1) Treatment with arginine as the only carbon-nitrogen source (arginine medium): i.e., only the above-mentioned medium A.

(2) Treatment with arginine as carbon-nitrogen source and pyruvic acid as an additional carbon source (arginine + pyruvic acid medium): i.e., the above-mentioned mediums A + B.

1ml of PBS-JD/YT bacterial liquid were inoculated into the 2 kinds of mediums, each treatment used the medium without inoculation of bacterial liquid as a control group. The medium were placed in a shaking table with a rotating speed of 180rpm at a constant temperature of 28°C for culture.

After a certain period of culture, the $OD_{600}$ value and the pH of the bacterial liquid were measured, and the contents of $NO_2^-$-N, $NH_4^+$-N and $NO_x^-$-N in the supernatant were measured.

### 1.4 NOS gene detection

The bacterial liquid of strains YT and JD in pure culture of L-arginine + pyruvic acid were subjected to PCR amplification, and Nitric Oxide Synthase (NOS) gene was detected.

PCR was performed by using TransStart Fastpfu DNA Polymerase, $20\mu l$ reaction system as follows:

| | |
|---|---|
| 5×FastPfu Buffer ..................... | 4 $\mu l$ |
| 2.5 mM dNTPs........................ | 2 $\mu l$ |
| Forward Primer(5 $\mu M$)............ | 0.8$\mu l$ |
| Reverse Primer(5 $\mu M$)............. | 0.8$\mu l$ |
| FastPfu Polymerase................. | 0.4 $\mu l$ |
| Template DNA........................ | 10ng |
| Supplemented ddH$_2$O to.......... | 20 $\mu l$ |

PCR instrument: ABI GeneAmp® 9700 type
PCR reaction parameters:

a. 1× (5 min at 95°C)
b. cycles× (30 s at 95°C; 30 s at Tm°C; 45 s at 72°C)

c. 10 min at 72°C, 10°C and unity halted by user

The primer sequences were as follows:

nosF: 5'-CGM(C/T) TGT TCN(A/C) TCG ACA GCC AG-3' (SEQ ID NO: 9)
nosR: 5'-CAT GTG CAG D(A/C/G/T)GC H(A/G)TG GCA GAA-3' (SEQ ID NO: 10)

## 2. Results

### 2.1 Growth and nitrification activity

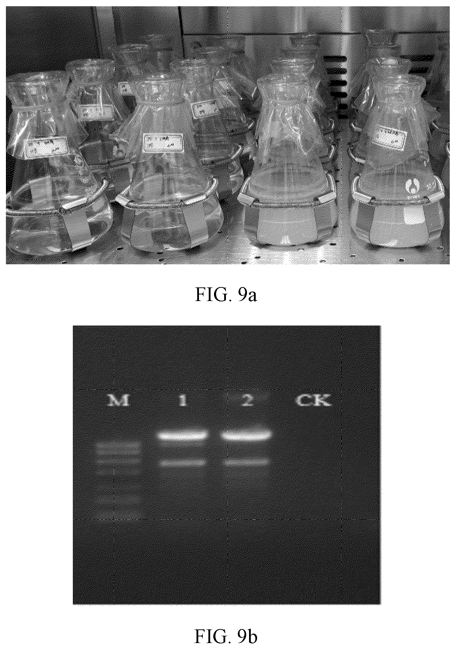

The experimental result of the culture with L-arginine as the only carbon-nitrogen source showed that both of the strains JD and YT did not grow, the pH of the culture solution was unchanged, the qualitative detection of the $NO_2^-$-N, $NH_4^+$-N and $NO_x^-$-N contents in the culture solution indicated negative results and had no any change. The color and morphology of the culture solution were not changed when observed from the outside (shown in the left two columns of FIG. 9a).

As shown in the following table and the two columns on the right side of FIG. 9a, the strains JD and YT grew rapidly when inoculated in the L-arginine + pyruvic acid culture medium, and the pH increased significantly during the growth process. After the two strains JD and YT grew for 18 days in a culture medium with pyruvic acid as a carbon source, the $NH_4^+$-N concentrations of the culture solutions were 59.27mg/L and 56.88mg/L respectively, which were obviously increased compared with the concentration in the control group.

When pyruvic acid was used as a carbon source, the strains JD and YT had strong nitrification effect, $NO_2^-$-N and $NO_x^-$-N in the culture solution accumulated in a large quantity: after 18 days of culture, the concentrations of $NO_2^-$-N in the culture solutions were 122.0mg/L and 136.55mg/L respectively, the concentrations of $NO_x^-$-N in the culture solutions were 198.67mg/L and 221.0mg/L, respectively.

**Table 9 Growth and nitrification activity of strains JD and YT inoculated in arginine + pyruvic acid medium (18 days of culture)**

| Strain | $OD_{600}$ | $NH_4^+$-N(mg/L) | $NO_2^-$-N(mg/L) | $NO_x^-$-N(mg/L) |
|---|---|---|---|---|
| CK | 0 | 1.9 | 0.03 | -0.02 |
| JD | 1.67 | 59.27 | 122.0 | 198.67 |
| YT | 1.83 | 56.88 | 136.55 | 221.0 |
| Note: The data in the table are averages, the number of replicates n =4 | | | | |

### 2.2 Nitric oxide synthase PCR amplification

Through gene detection analysis on YT and JD bacterial liquid in the culture system, both of the strains JD and JT displayed strong target bands according to PCR amplification results, as shown in FIG. 9b (M denoted standard, 1 denoted JD, 2 denoted YT, and CK denoted a control group), it demonstrated that nitric oxide synthase (NOS) existed in both of the strains JD and YT.

### 3. Conclusion

(1) The *Mycobacterium sp.* of the invention did not grow in a culture medium with L-arginine as the only carbon-nitrogen source, and the nitrification activity was not shown, which indicated that an arginine hydrolysis path did not exist.

(2) When culturing by using L-arginine as a carbon source and pyruvic acid as a nitrogen source, the *Mycobacterium sp.* of the invention grew vigorously, a large amount of nitrite nitrogen and nitrate nitrogen were accumulated, the nitrification of test strains was strong, thus the L-arginine oxidation pathway existed really.

(3) The *Mycobacterium sp.* of the invention utilized arginine and had strong nitrification, and nitric oxide synthase existed, it demonstrated that the arginine biosynthetic pathway is one of the pathways of the nitrogen metabolism of the strains.

**Example 9**

**The aerobic nitrite oxidation and assimilation nitrate reduction of *Mycobacterium sp.* of the invention**

**1. Materials and methods**

**1.1 Medium**

On the basis of the modified Stephenson medium, the nitrogen source ammonium sulfate was replaced by sodium nitrite with equal nitrogen amount (the content was 0.25 g/L), and other ingredients and treatments were unchanged, so that the modified Stephenson culture medium with the sodium nitrite as a nitrogen source was obtained.
The carbon source was sodium pyruvate, 0.4M sodium pyruvate solution was prepared first, and then sterilized separately, before use, 10ml of 0.4M sodium pyruvate solution was added into 100ml of modified Stephenson medium with sodium nitrite as a nitrogen source, the concentration of pyruvic acid in the medium was about 0.04M.

**1.2 Culture:** PBS-JD/YT bacterial liquid

**1.3 Culture system and conditions**

1ml of PBS-JD/YT bacterial liquid was inoculated to the modified Stevenson culture medium with sodium nitrite as a nitrogen source; the culture medium without inoculation of a bacterial liquid was regarded as a control group, 1ml of sterile water was added. The experiment was designed with a repetition number of 3 for each measurement.
The culture medium was placed in a shaking table with a rotation speed of 180rpm under the conditions including a temperature of 28°C, the $CO_2$ content of 5%, and the RH of 50%.

**1.4 Measurement of indicators**

Samples were taken for analysis every 3 days, starting from the 8 days of culture, 3 parallel replicates were taken for each time. The thallus growth amount ($OD_{600}$), pH value and contents of $NH_4^+$-N, $NO_2^-$-N, $NO_x^-$-N, and TN of the culture solution were measured. The difference between the $NO_x^-$-N content and the $NO_2^-$-N content was $NO_3^-$-N content.
After completion of the culture, the dry weight of the thallus, the nitrogen content (N%) and the carbon content (C%) were measured.

**2. Results**

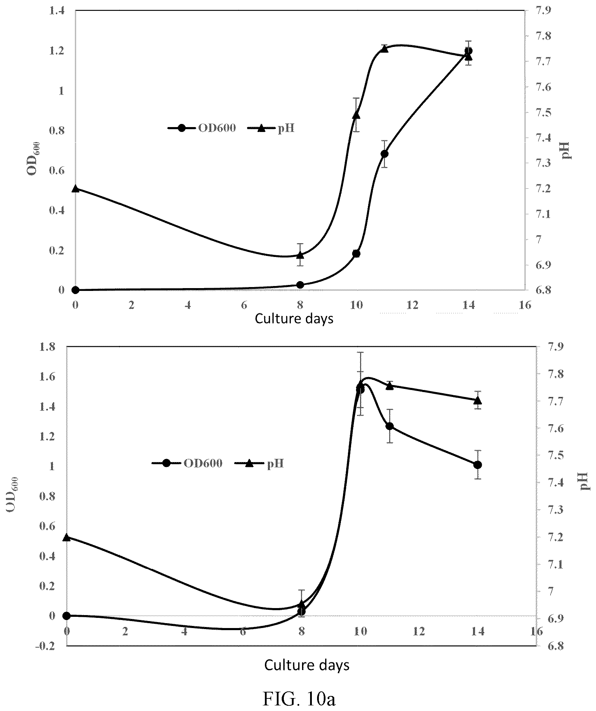

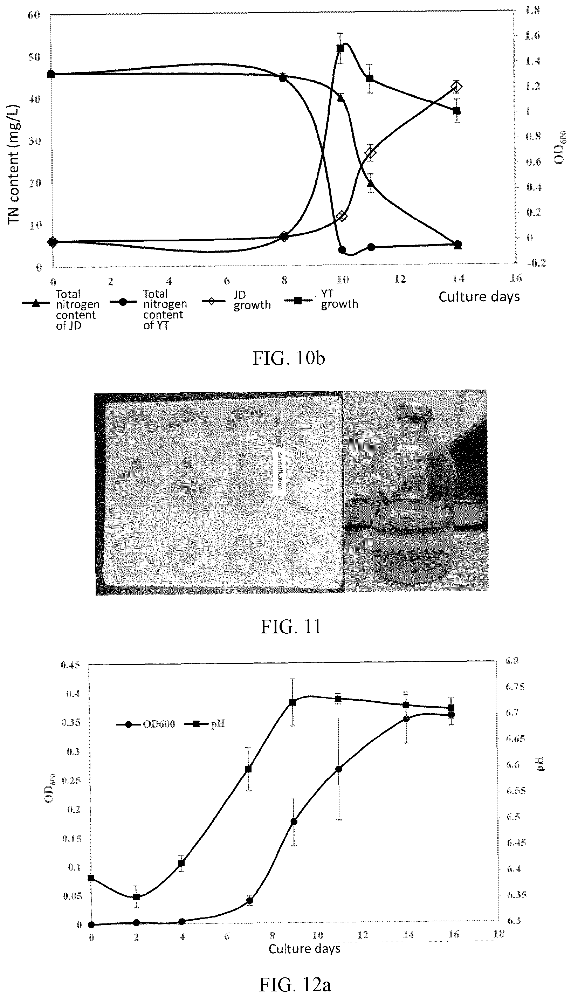

FIG. 10a showed the growth of strain JD (left) and strain YT (right) and the pH change of the medium under aerobic culture conditions with sodium nitrite as a nitrogen source, both of the strains JD and YT can grow, and the pH of the medium varied with the strain growth. In the initial culture period, both strains grew extremely slowly, and particularly, the $OD_{600}$ value had very small change after about 8 days of culture.
Although the strains JD and YT grew very slowly in the initial culture stage, the $NO_2^-$-N content in the medium was obviously reduced, and the $NO_3^-$-N content in the medium was clearly increased. As can be seen from Table 10a, after 8 days of culture, the average content of $NO_2^-$-N in the medium of the culture system in which the strains JD and YT resided was reduced from about 50mg/L in the initial stage to 15mg/L, and the content of $NO_3^-$-N appeared and reached 50mg/L, which resulted in the oxidation phenomenon of nitrous acid to nitrate.
Until 14 days of culture, the concentrations of $NO_2^-$-N and $NO_3^-$-N in JD medium were 0.36mg/L and 1.51mg/L respectively, and the concentrations of $NO_2^-$-N and $NO_3^-$-N in YT medium were 0.30mg/L and 1.54mg/L, respectively, the two forms of nitrogen basically disappeared; at the same time, a certain amount of was present in the medium but the concentrations were not high. The measured TN content of the two culture systems had no obvious change in the initial culture stage (namely the oxidation stage of nitrite to nitrate), the TN concentration was also rapidly reduced along with the fast growth of thallus and the rapid reduction of concentrations of $NO_2^-$-N and $NO_3^-$-N (FIG. 10b), and the concentrations of TN in the medium after 14 days of culture were 4.29mg/L and 4.58 mg/L respectively. As can be seen, the nitrate reduction occurred at the above stage.

**Table 10a Concentration change of $NH_4^+$-N, $NO_2^-$-N, $NO_3^-$-N during pure culture of strains JD and YT with sodium nitrite as a nitrogen source**

| Strain | Culture days | $NH_4^+$-N(mg/L) | | $NO_2^-$-N (mg/L) | | $NO_3^-$-N (mg/L) | |
|---|---|---|---|---|---|---|---|
| | | Average | SD | Average | SD | Average | SD |
| CK | 0 | 0 | | 50 | | 0 | |
| JD | 8 | 1.14 | 0.27 | 15.22 | 0.004 | 51.50 | 0.42 |
| | 10 | 1.04 | 0.16 | 15.21 | 0.004 | 48.51 | 0.75 |
| | 11 | 1.02 | 0.03 | 15.20 | 0.003 | 21.49 | 2.59 |
| | 14 | 1.11 | 0.02 | 0.36 | 0.076 | 1.51 | 0.05 |
| YT | 8 | 1.18 | 0.13 | 15.17 | 0.003 | 52.75 | 0.83 |
| | 10 | 0.85 | 0.20 | 0.25 | 0.147 | 1.45 | 0.07 |
| | 11 | 0.67 | 0.14 | 0.27 | 0.005 | 1.51 | 0.04 |
| | 14 | 0.39 | 0.08 | 0.30 | 0.072 | 1.54 | 0.07 |

JD and YT thallus grew during the nitrate reduction process. After 14 days of cultivation, the culture was centrifuged, the obtained thallus were weighed after being frozen and vacuum-dried, and the nitrogen content and the carbon content were measured, the nitrogen content in JD and YT thallus were about 6.5% and 7.4% respectively (Table 10b), the nitrogen content converted into thallus cells can be calculated according to the dry weight and the nitrogen content, and the result indicated that the organic nitrogen content of the thallus after completion of the culture was consistent with the addition amount of nitrogen source in a culture medium, and the nitrate reduction pathway was an assimilation pathway.

**Table 10b Weight and nitrogen contents of JD and YT thallus**

| Strain | Nitrogen content (%) | | Carbone content (%) | | Dry weight of thallus (g) | |
|---|---|---|---|---|---|---|
| | Average | SD | Average | SD | Average | SD |
| JD | 6.53 | 0.88 | 50.46 | 0.13 | 0.083 | 0.006 |
| YT | 7.38 | 0.31 | 50.29 | 0.77 | 0.075 | 0.009 |

### 3. Conclusion

The *Mycobacterium sp.* of the invention had the phenomenon that nitrite was oxidized into nitrate under the conditions of using sodium nitrite as a nitrogen source and shaking table cultivation; during the cultivation process, thallus grew, inorganic nitrogen in a medium basically synthesized into cells, and an assimilation pathway of nitrate reduction existed.

### Example 10

**Anaerobic denitrification (dissimilatory nitrate reduction) of *Mycobacterium sp.* of the invention**

### 1. Materials and methods

**1.1 Culture:** PBS-JD bacterial liquid

**1.2 Medium:**

On the basis of the modified Stephenson medium, the nitrogen source ammonium sulfate was replaced by sodium nitrite with equal nitrogen amount (the content was 0.25 g/L), and other ingredients and treatments were unchanged, so that the modified Stephenson medium with the sodium nitrite as a nitrogen source was obtained.
The carbon source was sodium pyruvate, 0.4M sodium pyruvate solution was prepared first, and then sterilized separately, before use, 10ml of 0.4M sodium pyruvate solution was added into 100ml of modified Stephenson medium with sodium nitrite as a nitrogen source, the concentration of pyruvic acid in the medium was about 0.04M.

**1.3 Culture system**

1ml of PBS-JD/YT bacterial liquid was inoculated to the modified Stevenson medium with sodium nitrite as a nitrogen source; the medium without inoculation of a bacterial liquid was regarded as a control group, 1ml of sterile water was added. The experiment was designed with a repetition number of 3 for each measurement.

**1.4 Anaerobic environment treatment and culture conditions:** the experimental culture system required to ensure that $O_2$ and $N_2$ did not exist in a bottle, the specific operations were as follows: the culture bottle containing the culture system was placed in an air extractor and vacuumized for 10min, helium gas was then introduced for 15s, the above operations were repeated for 6 times; in order to ensure pure culture, a $0.25\mu m$ filter membrane must be added during the air extraction process to prevent contamination by hybrid bacterium.

After the air pressure was balanced, a sealant was coated on the air suction holes, and the culture bottle was placed in a shaking table with a rotating speed of 180rpm at the temperature of 28°C.

**1.5 Measurement of indicators**

Samples were taken for analysis every 3 days, starting from 8 days of culture, 3 parallel replicates were taken for each time. The thallus growth amount ($OD_{600}$), pH value and contents of $NH_4^+$-N, $NO_2^-$-N, $NO_3^-$-N (the difference between the $NO_x$-N content and the $NO_2^-$-N content), and TN of the culture solution were measured; the contents of $N_2$, $CO_2$ and $N_2O$ were measured.

**2. Results**

The initial content of $NO_2^-$-N in the culture system was about 50mg/L, the concentration of $NO_2^-$-N in the culture medium was reduced to 0.5mg/L after 6 days of culture of strain JD, the contents of and $NO_x^-$-N were both lower than 0.5mg/L, and the TN content was about 2.0 mg/L; at this time, the culture medium had a pH of 7.18 and an $OD_{600}$ value of 0.005, and the thallus hardly grew (FIG. 11). Based on analysis on the gas content of the culture system, the accumulation of three gases $CO_2$, $N_2$ and $N_2O$ appeared in the culture flask (see the following table). As can be seen, the anaerobic denitrification phenomenon occurred in the strain JD, and the dissimilatory nitrate reduction existed.

**Table 11 Changes of three gases in the culture system varying with the culture time**

| Culture days | $CO_2$ content/ppm | | $N_2$ content/ppm | | $N_2O$ content/ppm | |
|---|---|---|---|---|---|---|
| | Average | SD | Average | SD | Average | SD |
| 0 | 0 | 0 | 0 | 0 | 0 | 0 |
| 3 | 4082.1 | 347.5 | 4326.6 | 409.6 | 0.082 | 0.004 |
| 6 | 4129.0 | 257.2 | 4769.9 | 296.3 | 0.116 | 0.016 |
| 9 | 4438.3 | 84.2 | 5032.4 | 133.6 | 0.195 | 0.027 |

**3. Conclusion**

Nitrite was used as a nitrogen source, under an anaerobic environment, $NO_2^-$-N and $NO_3^-$-N in the culture medium almost disappeared, gases $N_2$, $N_2O$ and $CO_2$ were generated, the *Mycobacterium sp.* of the invention generates an anaerobic denitrification phenomenon, and the thallus did not grow, and the dissimilatory nitrate reduction existed.

**Example 11**

**Ammoxidation direct nitrogen removal of *Mycobacterium sp.* of the invention**

**1. Materials and methods**

**1.1 Culture:** PBS-JD bacterial liquid

**1.2 Medium:** the modified Stephenson culture formula, the pH was adjusted to 6.4, the volume of each bottle of culture medium was 50ml, and the culture medium was sterilized at high temperature and high pressure for 20 minutes for later use.

The carbon source was sodium pyruvate, 0.2M sodium pyruvate solution (concentration was calculated in terms of pyruvic acid) was initially prepared, pH was adjusted to 6.4, the solution was sterilized separately for later use, 2.5ml of 0.2M sodium pyruvate solution was added into 50ml of modified Stephenson medium during culture, so that the designed concentration of sodium pyruvate was 0.01M.

**1.3 Culture System**

1ml of PBS-JD bacterial liquid was inoculated into the above-mentioned modified Stevenson medium with a pH of 6.4 and using sodium pyruvate as carbon source. The medium without inoculation of a bacterial liquid was regarded as a control group, 1ml of sterile water was added. The experiment was designed with a repetition number of 3 for each measurement.

The culture flask shall be covered with a sterilized rubber plug and pressed tightly to keep a closed environment.

**1.4 Culture conditions in closed environment**

After the culture systems were placed in an air extractor and vacuumized for 10min, a mixed gas containing 20% of oxygen gas and 80% of helium gas was introduced for 15s, the operations were repeated for 6 times. In order to ensure pure culture, a 0.25$\mu$m filter membrane must be added during the air extraction process to prevent contamination by hybrid bacterium.

After the air pressure was balanced, a sealant was coated on the air suction holes, and the culture bottle was placed in a shaking table with a rotating speed of 180rpm at the temperature of 28°C.

**1.5 Measurement of indicators**

Samples were taken for analysis every 3 days, 3 parallel replicates were taken for each time. The thallus growth amount ($OD_{600}$) and pH value were measured, the supernatant of the culture medium was extracted, contents of $NH_4^+$-N, $NO_2^-$-N, $NO_x^-$-N, and TN of the culture solution were measured; the contents of four gases $N_2$, $CO_2$, $O_2$ and $N_2O$ were measured, and the concentration of pyruvic acid in the culture medium was determined.

**2. Results**

FIG. 12a showed the growth condition and pH change of the liquid culture medium during the strain JD culture process. The strain JD grew rapidly after 7 days of culture, and the pH also exhibited an increase tendency, the pH was 6.73 after 11 days of culture.

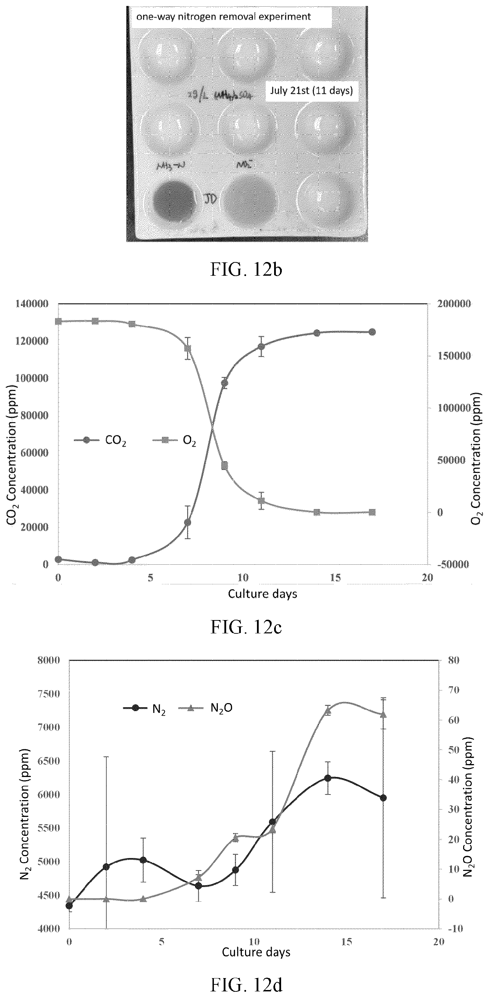

As shown in FIG. 12b, the content change of $NO_2^-$-N was qualitatively observed during the culture process, and no color was developed when titrated with the Griess reagent, indicated that $NO_2^-$-N was not accumulated in the culture solution; meanwhile, the contents of $NO_2^-$-N and $NO_x^-$-N were quantitatively measured, the contents did not increase compared with a control group, the concentrations of and TN in a liquid culture medium were reduced. It demonstrated that that ammonia oxidation of the strain JD was generated, but the nitrification did not occur under the culture conditions.

At the same time, the concentration changes of four gases $CO_2$, $O_2$, $N_2$ and $N_2O$ in the culture system were measured. Table 12 illustrated the statistics of the concentration changes of the four gases $CO_2$, $O_2$, $N_2$ and $N_2O$ in the culture system during JD culture process compared with the concentrations of control group; FIGs. 12c and 12d showed the concentration changes of the gases in the JD culture system varying with the culture time. Based on the analysis results, the concentration of $O_2$ in the JD culture system was reduced and the concentration of $CO_2$ was gradually increased along with an increase of the culture time; $N_2$ and $N_2O$ were obvious present in the JD culture system, in particular, the gas $N_2O$ exhibited a trend of increased accumulation along with an increase of the culture days. It indicated that under the culture condition, during JD metabolism process, pyruvic acid was oxidized to $CO_2$ and ammonia was oxidized to $N_2$ and $N_2O$.

**Table 12 Changes of gas concentration in the culture system**

| Culture days | $CO_2$/ppm | $O_2$/ppm | $N_2$/ppm | $N_2O$/ppm |
|---|---|---|---|---|
| 2 | -1728.1 | +296.5 | +578.7 | +0.08 |
| 4 | -380.7 | -2723.6 | +681.7 | +0.08 |
| 7 | +19818.2 | -25924.1 | +295.3 | +7.29 |
| 9 | +94661.6 | -138415.0 | +533.5 | +20.57 |
| 11 | +114245.6 | -172013.1 | + 1250.2 | +23.51 |

(continued)

| Culture days | $CO_2$/ppm | $O_2$/ppm | $N_2$/ppm | $N_2O$/ppm |
|---|---|---|---|---|
| 14 | +121486.9 | -182888.4 | +1901.6 | +63.24 |
| 17 | +121999.0 | -182913.9 | +1608.6 | +61.9 |

Note: (1) + represents an increase compared to a control group; - represents a decrease compared to a control group. (2) The data in the table are averages, n=3.

## 3. Conclusion

Nitrogen metabolism of the *Mycobacterium sp.* of the invention had a direct nitrogen removal pathway for ammonia oxidation.

## Example 12

**Inhibition of nitrification inhibitors on nitrification activity of *Mycobacterium sp.* of the invention**

## 1. Materials and methods

**1.1 Culture:** PBS-YT bacterial liquid

## 1.2 Medium

The nitrogen contents of the two nitrogen sources were equal in a modified Stephenson medium with $(NH_4)_2SO_4$ as a nitrogen source ($(NH_4)_2SO_4$ content was 2.0g/L) and a modified Stephenson medium with L-arginine hydrochloride ($C_6H_{14}N_4O_2 \cdot HCl$) as a nitrogen source ($C_6H_{14}N_4O_2 \cdot HCl$ content was 1.6 g/L).

When sodium pyruvate was added as a carbon source in the design of a culture medium, 1.0M sodium pyruvate solution (the concentration was calculated in terms of pyruvic acid) was prepared firstly, the pH was adjusted to the range of 7.0-7.2, and the solution was sterilized separately for later use. Before use, 1ml of 1.0M sodium pyruvate solution was added to about 100ml of modified Stevenson culture medium with $(NH_4)_2SO_4$ as a nitrogen source or L-arginine hydrochloride ($C_6H_{14}N_4O_2 \cdot HCl$) as a nitrogen source, and the pyruvic acid concentration of the culture system was designed to be 0.01M.

## 1.3 Nitrification inhibitors

Four kinds of nitrification inhibitors 4-amino-1,2,4-triazole (ATC), dicyandiamide (DCD, $C_2H_4N_4$), aminoguanidine hydrochloride ($CH_6N_4 \cdot HCl$), and guanylthiourea ($C_2H_6N_4S$) were selected. The four nitrification inhibitors were prepared with a concentration of 500mM, respectively, after the solutions were filtered and sterilized, 1ml of the four nitrification inhibitors with a concentration of 500mM was added into the two medium with different nitrogen sources, such that the corresponding concentration of each of the nitrification inhibitors in the systems was 5 mM.

## 1.4 Culture system setup

Two mediums with different nitrogen sources were respectively subjected to 5 treatments, namely without addition of an inhibitor, adding ATC, adding DCD, adding aminoguanidine hydrochloride, and adding guanylthiourea as shown in the table below; the YT system without addition of an inhibitor and the system without inoculation of strain YT and only addition of an inhibitor were respectively used as a control group; 1ml of PBS-YT bacterial liquid was inoculated, and 1ml of sterile water was used as a substitute when the inoculation of a bacterial strain was not performed. The total volume of each culture system was equal, it was about 100 ml.

Table 13a

| Medium | PBS-YT inoculation condition | Addition condition of nitrification inhibitor | | | | |
|---|---|---|---|---|---|---|
| Ammonium sulfate + pyruvic acid modified ST | Bacteria were non-inoculated | Not added | +ATC | +DCD | + Aminoguanidine hydrochloride | + Guanylthiourea |
| | Bacteria were inoculated | Not added | +ATC | +DCD | + Aminoguanidine hydrochloride | + Guanvlthiourea |
| L-arginine hydrochloride + pyruvic acid-modified ST | Bacteria were non-inoculated | Not added | +ATC | +DCD | + Aminoguanidine hydrochloride | + Guanvlthiourea |
| | Bacteria were inoculated | Not added | +ATC | DCD | + Aminoguanidine hydrochloride | + Guanylthiourea |

Meanwhile, the invention further designed with the treatments of medium, which were not added with any nitrogen source, 4 inhibitors were used as the nitrogen source respectively, and the pyruvic acid was used as the carbon source; the growth and nitrogen metabolism conditions of the culture medium were observed.

**1.5 Culture conditions:** the culture was performed in a shaking table with a rotational speed of 180rpm at 28°C.

**1.6 Measurement and calculation of indicator:** the growth $OD_{600}$ and pH value of thallus in the culture solution were measured every week, the contents of $NH_4^+$-N, $NO_2^-$-N and $NO_x^-$-N were measured, the inhibition effect of the nitric oxide synthase (nitrification) activity of different culture systems were calculated and compared.

Nitric oxide synthase (nitrification) inhibition rate (%) = ($NO_2^-$-$N_{YT}$ concentration - $NO_2^-$-$N_{culture}$ system added with an inhibitor concentration) $\times 100$/ $NO_2^-$-$N_{YT}$ concentration

## 2. Research results

### 2.1 Effects of aminoguanidine hydrochloride and guanylthiourea on YT growth and nitrification activity

Pure culture was carried out by using ammonium sulfate and L-arginine hydrochloride as a nitrogen sources respectively, the strain YT can perform nitrification, and the accumulation amount of $NO_2^-$-N was gradually increased along with an increase of culture time. Pure culture was implemented for 35 days by using ammonium sulfate as a nitrogen source, the concentration of $NO_2^-$-N in a culture system without adding any nitrification inhibitor was 6.88 mg/L; after the aminoguanidine hydrochloride was added, the concentrations of $NO_2^-$-N in the system after the culture was carried out for 7 days, 14 days, 21 days, 28 days and 35 days were lower than the $NO_2^-$-N concentration in the system without adding the inhibitor; after the addition of guanylthiourea, the concentrations of $NO_2^-$-N in the system after the culture was carried out for 7 days, 14 days, 21 days, 28 days and 35 days were lower than the $NO_2^-$-N concentration in the system without adding the inhibitor; as can be seen, aminoguanidine hydrochloride had an inhibitory effect on the nitrification activity of the strain YT, and guanylthiourea also exhibited inhibitory effect on the nitrification activity, but the nitrification inhibitory effect was gradually weakened along with an increase of culture time. For details, see the Table below.

Pure culture was carried out by using L-arginine hydrochloride as a nitrogen source, the concentrations of $NO_2^-$-N in a culture system without adding any nitrification inhibitor after 7 days, 14 days, 21 days and 35 days were 0, 101.59, 113.72 and 113.65mg/L respectively; the concentrations of $NO_2^-$-N in the system added with aminoguanidine hydrochloride after the culture was performed for 14 days, 21 days, 28 days and 35 days were lower than the $NO_2^-$-N concentration in the system without adding the inhibitor; the concentrations of $NO_2^-$-N in the system added with guanylthiourea after the culture was performed for 14 days, 21 days, 28 days and 35 days were lower than the $NO_2^-$-N concentration in the system without adding the inhibitor. Therefore, aminoguanidine hydrochloride and guanylthiourea both had an inhibitory effect on the nitrification activity of the strain YT, the effect of nitration inhibitor aminoguanidine hydrochloride was always obvious within 35 days of culture, and the nitration inhibition effect of guanylthiourea was reduced along with an extension of the culture time. For details, see the Table below.

**Table 13b Effects of aminoguanidine hydrochloride and guanylthiourea on nitrification activity of the strain YT**

| Growth time | Nitrification inhibitor | Ammonium sulfate as nitrogen source | | | L-arginine hydrochloride as nitrogen source | | |
|---|---|---|---|---|---|---|---|
| | | pH | $NH_4^+$-N (mg/L) | $NO_2^-$-N (mg/L) | pH | $NH_4^+$-N (mg/L) | $NO_2^-$-N (mg/L) |
| 7 days | Without addition of inhibitor YT | 6.7 | 391.90 | 0.41 | 7.7 | 55.35 | 0 |
| | Aminoguanidine hydrochloride | 6.7 | 418.92 | 0 | 7.5 | 12.86 | 0 |
| | Amidinothiourea | 6.5 | 395.07 | 0.02 | 7.2 | 8.13 | 0 |
| 14 days | Without addition of inhibitor YT | 7.2 | 406.85 | 2.44 | 7.5 | 234.09 | 101.59 |
| | Aminoguanidine hydrochloride | 6.7 | 419.06 | 0.01 | 7.2 | 38.07 | 0.24 |
| | Amidinothiourea | 7.0 | 418.86 | 0.13 | 7.7 | 175.90 | 1.72 |
| 21 days | Without addition of inhibitor YT | 7.0 | 408.84 | 4.09 | 7.2 | 206.15 | 113.72 |
| | Aminoguanidine hydrochloride | 6.7 | 438.98 | 0.03 | 7.2 | 100.12 | 0.28 |
| | Amidinothiourea | 7.0 | 407.33 | 1.64 | 7.7 | 154.73 | 31.10 |
| 28 days | Without addition of inhibitor YT | 7.0 | 426.55 | 6.76 | 7.2 | 194.32 | 109.86 |
| | Aminoguanidine hydrochloride | 7.0 | 448.65 | 0.19 | 7.5 | 145.55 | 0.58 |
| | Amidinothiourea | 7.0 | 419.92 | 4.57 | 7.7 | 138.43 | 25.70 |
| 35 days | Without addition of inhibitor YT | 7.0 | 426.55 | 6.88 | 7.2 | 194.32 | 113.65 |
| | Aminoguanidine hydrochloride | 6.7 | 468.07 | 0.14 | 7.5 | 58.70 | 1.00 |
| | Amidinothiourea | 7.0 | 422.98 | 6.12 | 8.0 | 165.02 | 32.26 |

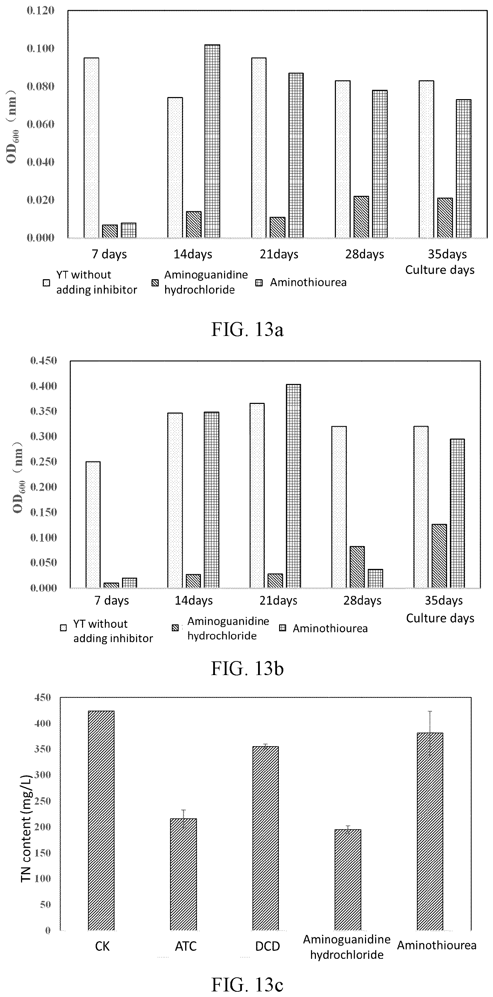

FIGs. 13a and 13b showed that the strain YT was subjected to a pure culture by using ammonium sulfate as a nitrogen source (FIG. 13a) and using L-arginine hydrochloride as a nitrogen source (FIG. 13b) respectively; when the pure culture was performed by using L-arginine hydrochloride as a nitrogen source, the strain YT grew faster than that in pure culture with ammonium sulfate as a nitrogen source; the addition of aminoguanidine hydrochloride can inhibit the growth of the strain YT under pure culture with two nitrogen sources, and the inhibition effect was always obvious during the culture process; when guanylthiourea was added, it can obviously inhibit the growth of stain YT under pure culture of two nitrogen sources in the initial culture stage, after 14 days of culture, the growth inhibition of strain YT under pure culture with ammonium sulfate as a nitrogen source was weakened.

**2.2 Effects of ATC and DCD on YT growth and nitrification activity**

Firstly, as shown in the following table, when the L-arginine hydrochloride was used as a nitrogen source for pure culture, the growth and the nitrification activity of strain YT were stronger than those of strain YT when ammonia sulfate was used as a nitrogen source for pure culture; through the analysis on the concentration change of the $NO_2^-$-N in different culture times, ATC always exhibited the inhibiting effect on the nitrification activity of strain YT under pure culture with two nitrogen sources, but DCD did not impose an inhibitory effect on the nitrification activity. On the other hand, both of the nitrification inhibitors ATC and DCD did not inhibit the growth of strain YT.

**Table 13c Effects of ATC and DCD on growth and nitrification activity of the strain YT in** pure culture with two nitrogen sources

| Culture days | Nitrification inhibitor | Ammonium sulfate as nitrogen source | | | | L-arginine hydrochloride as nitrogen source | | | |
|---|---|---|---|---|---|---|---|---|---|
| | | $OD_{600}$ | pH | $NH_4^+$-N | $NO_2^-$-N | $OD_{600}$ | pH | $NH_4^+$-N | $NO_2^-$-N |
| 7 days | YT without adding inhibitor | 0.254 | 7.0 | 423 | 1.0 | 0.318 | 7.5 | 58 | 0.50 |
| | ATC | 0.214 | 7.0 | 419 | 0 | 0.403 | 7.5 | 15 | 0 |
| | DCD | 0.207 | 7.0 | 421 | 1.00 | 0.397 | 7.5 | 18 | 0.50 |
| 14 days | YT without adding inhibitor | 0.105 | 7.0 | 405 | 2.50 | 0.449 | 7.7 | 132 | 35.0 |
| | ATC | 0.127 | 7.0 | 410 | 0.50 | 0.394 | 7.7 | 89 | 0.5 |
| | DCD | 0.121 | 7.0 | 408 | 2.50 | 0.438 | 7.7 | 121 | 32.5 |
| 20 days | YT without adding inhibitor | 0.115 | 7.0 | 375 | 4.73 | 0.468 | 7.5 | 186 | 153.87 |
| | ATC | 0.133 | 7.2 | 366 | 0.5 | 0.329 | 8 | 130 | 0.37 |
| | DCD | 0.127 | 7.2 | 355 | 3.20 | 0.415 | 7.7 | 191 | 152.77 |
| 31 days | YT without adding inhibitor | 0.136 | 7.0 | 344 | 5.59 | 0.346 | 7.5 | 177 | 151.66 |
| | ATC | 0.146 | 7.0 | 382 | 1.17 | 0.252 | 7.7 | 111 | 0.33 |
| | DCD | 0.128 | 7.0 | 382 | 5.11 | 0.378 | 7.5 | 173 | 153.87 |

**2.4 Comparison of the inhibitory effect of different inhibitors on nitric oxide synthase (nitrification) activity of stain YT**

As shown in the following Table, the inhibition rate of aminoguanidine hydrochloride on nitrification activity of strain YT using L-arginine hydrochloride as a nitrogen source was 99% or more, and its inhibition rate on nitrification activity of strain YT using ammonium sulfate as a nitrogen source more than 97%. Before 14 days of culture, the guanylthiourea had an inhibition rate of 95% or more on YT nitric oxide synthase (nitrification) activities of two nitrogen sources, and the inhibition rate was then decreased, after 35 days of culture with ammonium sulfate as a nitrogen source, the inhibition rate of the nitric oxide synthase (nitrification) activity was greatly reduced to 11%; after 35 days of culture with L-arginine hydrochloride as a nitrogen source, the inhibition rate of the nitric oxide synthase (nitrification) activity was reduced to 72%.

**Table 13d The inhibitory effect of aminoguanidine hydrochloride and guanylthiourea on nitric oxide synthase (nitrification) activity of strain YT**

| Nitrification inhibitor types | Nitrogen source types | Nitric oxide synthase (nitrification) activity inhibition rate (%) | | | | |
|---|---|---|---|---|---|---|
| | | Culture 7 days | Culture 14 days | Culture 21 days | Culture 28 days | Culture 35 days |
| Aminoguanidine hydrochloride | Ammonium sulfate | 99.19 | 99.66 | 99.36 | 97.23 | 97.96 |
| | L-arginine hydrochloride | * | 99.76 | 99.75 | 99.49 | 99.12 |
| Amidinothiourea | Ammonium sulfate | 95.64 | 94.80 | 59.82 | 33.52 | 11.07 |
| | L-arginine hydrochloride | * | 98.31 | 72.65 | 77.39 | 71.62 |
| Note: * in the table indicates that both the control group in which the inhibitor was not added and the treat group in which the inhibitor was added did not perform nitrification. | | | | | | |

ATC had obvious inhibition effect on nitric oxide synthase (nitrification) activity of strain YT, it almost completely inhibited the nitric oxide synthase (nitrification) activity when L-arginine hydrochloride was used as a nitrogen source, and had a weaker inhibition effect when ammonium sulfate was used as the nitrogen source, and the inhibition effect showed a decreasing trend along with the culture time. DCD had little inhibition effect on the nitric oxide synthase (nitrification) activity of strain YT, as shown in Table 13e.

**Table 13e The inhibitory Effect of ATC and DCD on nitric oxide synthase (nitrification) activity of strain YT**

| Nitrification inhibitor types | Nitrogen source types | Nitric oxide synthase (nitrification) activity inhibition rate (%) | | | |
|---|---|---|---|---|---|
| | | Culture 7 days | Culture 14 days | Culture 20 days | Culture 31 days |
| ATC | Ammonium sulfate | 100 | 80.0 | 89.40 | 25.42 |
| | L-arginine hydrochloride | 100 | 98.57 | 99.76 | 99.78 |
| DCD | Ammonium sulfate | 0 | 0 | 32.42 | 8.62 |
| | L-arginine hydrochloride | 0 | 7.14 | 0.72 | 0 |

**2.5 Growth and metabolism of YT when four nitrification inhibitors were used as the nitrogen sources for pure culture**

The strain YT was able to grow in pure culture when four nitrification inhibitors were used as the nitrogen sources and pyruvic acid was used as a carbon source, and the content of TN in the culture solution was reduced compared to the initial content of the culture medium before inoculation, it indicated that the nitrification inhibitors were involved in the nitrogen metabolism (FIG. 13c).

**3. Conclusion**

(1) Each of the 4 selected nitrification inhibitors can be used as alternative nitrogen source substrate, so that the strain YT with arginine as the unique nitrogen source grew.

(2) Aminoguanidine hydrochloride had an inhibitory effect on growth and nitrification activity of strain YT; guanylthiourea exhibited an inhibiting effect on nitrification activity of strain YT, the inhibiting effect at the initial stage of culture was obvious, and the inhibition effect on the nitrification activity was weakened along with the prolonging of the culture time; ATC had an obvious inhibitory effect on nitrification activity of strain YT, but did not inhibit the growth of strain YT; DCD had no significant inhibitory effect on growth and nitrification activity of strain YT.

(3) The growth and nitrification activity of strain YT with L-arginine hydrochloride as a nitrogen source were stronger than those when ammonium sulfate was used as a nitrogen source.

(4) The nitrification inhibitor substantially inhibited activity of the nitric oxide synthase.

**Example 13**

**The antagonistic effect of *Mycobacterium sp.* in the invention in regard to other *Mycobacterium sp.***

**1. Materials and methods**

1.1 Test strains: Strain YT obtained in Preparation Example 1; standard strains *Mycobacterium austroafricanum* (deposit number CMCC (B) 95044), *Mycobacterium vaccae* (ATCC No. 15483™), and *Mycobacterium vanbaalenii* (DSM 7251).

The aforementioned strains were prepared into a PBS bacterial liquid.

**1.2 LB plate:** 10g/L of peptone, 5g/L of yeast powder, 10g/L of NaCl, 20g/L of agar powder, and the pH of 7.2.

**1.3 Experimental methods**

Two types of experimental methods were adopted, namely

The first type of experimental method: LB plate lineation method. The specific operations were as follows: the same LB plate was uniformly lineated and then inoculated two or three test standard strains, the lineation between two test standard strains was inoculated with the strain YT, the LB plate was placed in an incubator at 28°C for 1 week, the growth condition of the test strains on each plate was observed.

The second type of experimental method: the K-B method, i.e., the filtering paper method. The specific steps were as follows: (1) the filter paper with uniform texture was selected, a plurality of wafers (with a diameter of 6mm) with the same diameter were produced by using a punching machine, the wafers were sterilized and dried, a certain amount of wafers were soaked in a PBS-YT bacterial liquid for later use; (2) a ring of three standard strains were picked and added into three triangular flasks of PBS with a volume of 100 ml, 200μL of the standard strains were then respectively taken and uniformly coated on different LB plates to form the test plates; (3) the filter paper soaked with PBS-YT bacterial liquid was placed in test plates, 3 filter paper sheets were placed on each test plate, and inverted after 5min; (4) the test plate attached with a filter paper soaked with physiological saline was used as a control group; (5) the treated plates were placed in an incubator, and cultured at 28°C for 1 week, and then taken out, the size of an inhibition zone was measured, the morphology of the inhibition zone was shot by using the Nikon AZ100 stereoscope.

## 2. Results

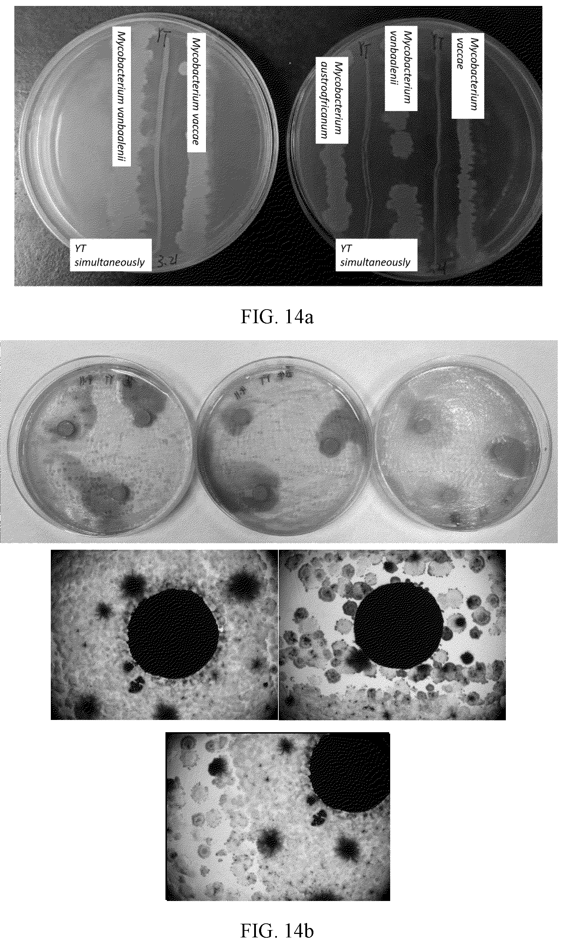

The *Mycobacterium vaccae* and *Mycobacterium vanbaalenii* were inoculated on the LB plate, when the strain YT was lineated between two standard strains, it was obviously observed that the two strains *Mycobacterium vaccae* and *Mycobacterium vanbaalenii* expanded and grew toward the side which was not inoculated with the strain YT; when three standard strains were lineated on the LB plate, YT was inoculated between *Mycobacterium austroafricanum* and *Mycobacterium vanbaalenii* and between *Mycobacterium vanbaalenii* and *Mycobacterium vaccae,* both of the strains *Mycobacterium austroafricanum* and *Mycobacterium vaccae* on both sides of LB plate spreaded and grew toward the side which was not inoculated with the strain YT, the strain *Mycobacterium vanbaalenii* in the middle did not exhibit the sprawl and growth phenomenon, as shown in FIG. 14a.

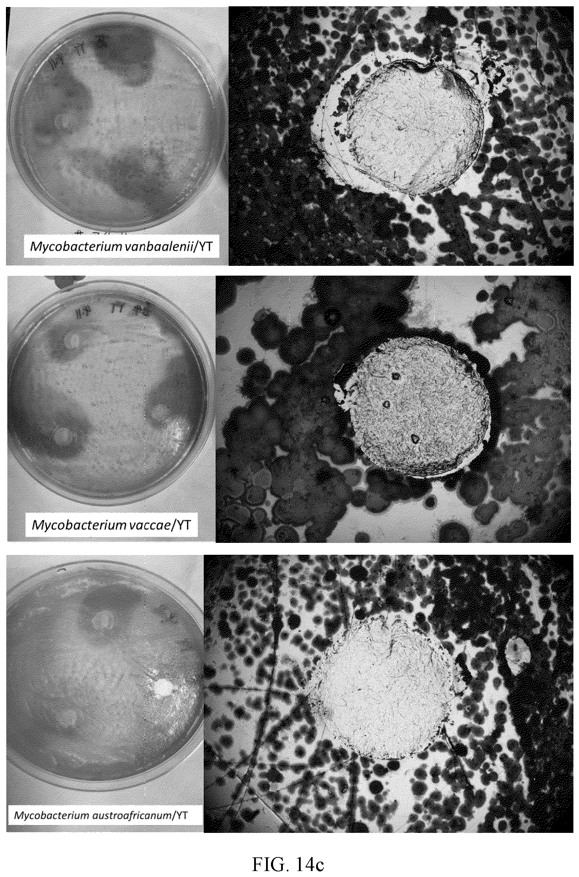

After cultivation with the K-B method for 1 week, it was observed by combining an electron microscope, an inhibition zone was formed at the position where the LB plate was attached with a filter paper soaked with the YT bacterial liquid, wherein the diameter of the inhibition zone exceeded 6mm, the three standard strains *Mycobacterium austroafricanum, Mycobacterium vanbaalenii* and *Mycobacterium vaccae* did not grow at all; the filter paper was further dripped with Griess reagent, it showed pink color, which indicated an occurrence of nitrification, as shown in FIG. 14b (the filter paper soaked with the YT bacterial liquid was not removed, and 3 LB plates were sequentially coated with *Mycobacterium vanbaalenii, Mycobacterium vaccae,* and *Mycobacterium austroafricanum* from left to right) and FIG. 14c (the filter paper soaked with the YT bacterial liquid was removed). However, the three standard strains in the control group attached with the filter paper which was not soaked with YT bacterial liquid grew uniformly. As can be seen, the strain YT had an antagonistic effect on *other Mycobacterium sp.*

## 3. Conclusion

The *Mycobacterium sp.* of the invention had an antagonistic effect on the mycobacteria including *Mycobacterium vanbaalenii, Mycobacterium vaccae,* and *Mycobacterium austroafricanum.*

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

**References**

Markus Ribbe, Dilip Gadkari, Ortwin Meyer. N2 Fixation by Streptomyces thermoautotrophicus Involves a Molybdenum-Dinitrogenase and a Manganese-Superoxide Oxidoreductase That Couple N2 Reduction to the Oxidation of Superoxide Produced from O2 by a Molybdenum-CO Dehydrogenase. The Journal of Biological Chemistry, 1997, 272(42):26627-26633.

Marcel M. M. Kuypers, Hannah K. Marchant and Boran Karta. The microbial nitrogen-cycling network. Nature, 2018. doi:10.1038/nrmicro.2018.9, Published online 5 Feb 2018.

Pertti J.M. Heterotrophic nitrification -- An eternal mystery in the nitrogen cycle. *Soil Biology and Biochemistry,* 2022, 168. https://doi.org/10.1016/j.soilbio.2022.108611.

Aina Soler-Jofra, Julio Pérez, Mark C.M. van Loosdrecht. Hydroxylamine and the nitrogen cycle: A review. Water Research, 2021, 190, 116723. https://doi.org/10.1016/j.watres.2020.116723.

Holger Daims, Elena V. Lebedeva, Petra Pjevac, Ping Han, Craig Herbold, et al. Complete nitrification by Nitrospira bacteria. Nature, 2015, 528. doi: 10.1038/nature 16461.

ZHANG Miao-miao, SHEN Ju-pei, HE Ji-zheng, ZHANG Li-mei, "Microbial Mechanisms of Nitrification Inhibitors and Their Application", Journal of Agro-Environment Science, 2014, 33 (11): 2077-2083.

ZHANG Hao, ZAN Jin-xing, LIU Pei-xun, "Research Progress on Inducible Nitric Oxide Synthase Inhibitors", CHINESE JOURNAL OF NEW DRUGS, 2012, 22 (6): 665-669.

LUO Hui-ting, KONG Bao-hua, LI Pei-jun, LI Mu-zi, "Bacterial Nitric Oxide Synthase and Its Application in Food Science: a Review", FOOD SCIENCE, 2014, 35 (17): 266-271.

ZHANG Yang, LI Ya-ying, ZHENG Ning-guo, YAO Huai-ying, "The Inhibition Principle of Biological Nitrification Inhibitors and the Research Progress", JIANGSU AGRICULTURE SCIENCES, 2019, 47 (1): 21-26.

ZHU Zhao-liang, WEN Qi-xiao, "Nitrogen in Chinese Soil", Nanjing: Jiangsu Science and Technology Press, 1992.

SHENG Tong, WANG Jing-yan, "Biochemistry", Beijing: Higher Education Press, 2000.

HAN Bin, KONG Ji-jun, ZOU Xiao-ming, KONG He-de, "Current Status and Prospect of Biological Nitrogen Fixation Research", Journal of Shanxi Agriculture Sciences, 2009, 37 (10): 86-89, 85.

## Claims

1.  *Mycobacterium sp.*, **characterized in that** the deposit number of the *Mycobacterium sp.* is CGMCC No.21272 or CGMCC No. 21273.

2.  A microbial agent, **characterized in that** the microbial agent comprises the *Mycobacterium sp.* according to claim 1.

3.  A nitrogen removal method, **characterized in that** the method comprises: contacting the *Mycobacterium sp.* according to claim 1 or the microbial agent according to claim 2 with a system for nitrogen removal.

4.  The method according to claim 3, wherein N in the system for nitrogen removal is at least one of molecular nitrogen, ammoniacal nitrogen, nitrate nitrogen and nitrite nitrogen;
    and/or, the system for nitrogen removal is waste water, waste gas or waste residue.

5.  A method for treating nitrogen pollution, **characterized in that** the method comprises: contacting the *Mycobacterium sp.* according to claim 1 or the microbial agent according to claim 2 with a nitrogen-containing pollutant.

6.  A method for capturing $CO_2$, **characterized in that** the method comprises: contacting the *Mycobacterium sp.* according to claim 1 or the microbial agent according to claim 2 with a $CO_2$-containing system.

7.  A method for screening agricultural chemicals, **characterized in that** the method comprises: inoculating the *Mycobacterium sp.* according to claim 1 into an ammonia nitrogen medium containing agricultural chemicals to be screened, and culturing the *Mycobacterium sp.*, and determining whether the agricultural chemicals to be screened have a function of inhibiting the ammonia nitrogen loss based on the change of N during the culturing process and/or the growth condition of the *Mycobacterium sp.*

8.  Use of the *Mycobacterium sp.* according to claim 1 or the microbial agent according to claim 2 in treating nitrogen pollution, carbon capture, screening agricultural chemicals, the treatment and/or prevention of skin diseases, the treatment and/or prevention of diseases associated with low nitrite level, the treatment and/or prevention of body odour, the treatment of supplying nitric oxide to a subject, or inhibiting growth of microorganisms.

9.  Use according to claim 8, wherein the agricultural chemical is a nitrification inhibitor and/or a nitric oxide synthase inhibitor;

    and/or, the inhibition is *in vitro* inhibition;
    and/or, the use is a use in the manufacture of a medicament or formulation for the treatment and/or prevention of skin diseases, the treatment and/or prevention of diseases associated with low nitrite level, the treatment and/or prevention of body odour, the treatment of supplying nitric oxide to a subject, and inhibiting growth of microorganisms;
    and/or, the microorganisms are *Mycobacterium* genus microorganisms, more preferably *Mycobacterium vaccae* and/or *Mycobacterium vanbaalenii.*

**10.** A method for isolating the *Mycobacterium sp.* with nitrification activity, **characterized in that** the method comprises: (1) preliminary screening the *Mycobacterium sp.* having a nitrification activity; (2) using a medium added with antibiotics for further enriching the *Mycobacterium sp.* with a high nitrification activity.

FIG. 1a

Non-coding RNA categories

■ tRNA
■ 5S RNA
■ 16S rRNA
■ 23S rRNA

RNA processing and modification
Chromatin structure and dynamics
Energy production and conversion
Cell cycle control, cell division, chromosome partitioning
Amino acid transport and metabolism
Nucleotide transport and metabolism
Carbohydrate transport and metabolism
Coenzyme transport and metabolism
Lipid transport and metabolism
Translation, ribosomal structure and biogenesis
Transcription
Replication, recombination and repair
Cell wall/membrane/envelope biogenesis

COG Functional categories

Cell motility
Posttranslational modification, protein turnover, chaperones
Inorganic ion transport and metabolism
Secondary metabolites biosynthesis, transport and catabolism
General function prediction only
Function unknown
Signal transduction mechanisms
Intracellular trafficking, secretion, and vesicular transport
Defense mechanisms
Extracellular structures
Nuclear structure
Cytoskeleton
No assigned COG

FIG. 1b

FIG. 1c

FIG. 1d

FIG. 2a

FIG. 2b

FIG. 2c

FIG. 2d

FIG. 2e

FIG. 2f

FIG. 3a

FIG. 3b

FIG. 3c

FIG. 3d

FIG. 3e

FIG. 3f

FIG. 3g

FIG. 4a

FIG. 4b

FIG. 5

FIG. 6

FIG. 7a

FIG. 7b

FIG. 7c

FIG. 8

FIG. 9a

FIG. 9b

FIG. 10a

FIG. 10b

FIG. 11

FIG. 12a

FIG. 12b

FIG. 12c

FIG. 12d

FIG. 13a

FIG. 13b

FIG. 13c

FIG. 14a

FIG. 14b

*Mycobacterium vanbaalenii*/YT

*Mycobacterium vaccae*/YT

*Mycobacterium austroafricanum*/YT

FIG. 14c

## INTERNATIONAL SEARCH REPORT

| International application No. |
| --- |
| **PCT/CN2023/113637** |

### A. CLASSIFICATION OF SUBJECT MATTER

C12N 1/20(2006.01)i; C12R 1/32(2006.01)i

According to International Patent Classification (IPC) or to both national classification and IPC

### B. FIELDS SEARCHED

Minimum documentation searched (classification system followed by classification symbols)

IPC: C12N,C12R

Documentation searched other than minimum documentation to the extent that such documents are included in the fields searched

Electronic data base consulted during the international search (name of data base and, where practicable, search terms used)

Databases: CNABS, WPABSC, VEN, CNTXT, ENTXT, CNKI, 百度学术, Baidu Scholar, PubMed, ISI-WEB OF SCIENCE, Genbank+EMBL, 中国专利生物序列检索系统, China Patent Biological Sequence Search System; search terms: 分枝杆菌, 脱氮, 氮污染, 硝化, 碳捕获, 捕获CO2, 捕获二氧化碳, mycobacterium, denitrification, nitrogen pollution, nitrification, carbon capture, capture CO2, capture carbon dioxide, 对序列SEQ ID NOs: 1-2的检索, search for SEQ ID NOs: 1-2

### C. DOCUMENTS CONSIDERED TO BE RELEVANT

| Category* | Citation of document, with indication, where appropriate, of the relevant passages | Relevant to claim No. |
| --- | --- | --- |
| A | CN 108285881 A (GUANGZHOU UNIVERSITY; CHONGQING INSTITUTE OF GREEN AND INTELLIGENT TECHNOLOGY, CHINESE ACADEMY OF SCIENCES) 17 July 2018 (2018-07-17)<br>see description, paragraphs [0006]-[0025], [0035]-[0045] | 1-10 |
| A | CN 108220186 A (WUXI BAIWOTE ENVIRONMENTAL PROTECTION TECHNOLOGY CO., LTD.; YIXING ZHONGYI TOWN WATER EQUIPMENT DEVELOPMENT CO., LTD.) 29 June 2018 (2018-06-29)<br>see description, paragraphs [0005]-[0019] | 1-10 |
| A | KR 20070111916 A (SAMSUNG ENG CO., LTD.) 22 November 2007 (2007-11-22)<br>see abstract | 1-10 |
| A | 李全霞 等人 (LI, Quanxia et al.). "降解芘的分枝杆菌M11的分离鉴定和降解特性 (Isolation, Identification of a Pyrene-Degrading Strain Mycobacterium sp. M11 and Its Degrading Characteristics)"<br>环境科学 (Environmental Science), Vol. 29, No. (3), 15 March 2008 (2008-03-15),<br>see pages 763-767 | 1-10 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **17 November 2023** | **24 November 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/113637** |

### C. DOCUMENTS CONSIDERED TO BE RELEVANT

| Category* | Citation of document, with indication, where appropriate, of the relevant passages | Relevant to claim No. |
| --- | --- | --- |
| A | JIN, Xiaojun et al. "Enhancing the Electricity Generation and Nitrate Removal of Microbial Fuel Cells With a Novel Denitrifying Exoelectrogenic Strain EB-1" *Frontiers in Microbiology,* Vol. vol. 9, 09 November 2018 (2018-11-09), see article no. 2633, pages 1-8 | 1-10 |

Form PCT/ISA/210 (second sheet) (July 2022)

## INTERNATIONAL SEARCH REPORT

| International application No. |
| --- |
| **PCT/CN2023/113637** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
|---|
| **PCT/CN2023/113637** |

**Box No. II    Observations where certain claims were found unsearchable (Continuation of item 2 of first sheet)**

This international search report has not been established in respect of certain claims under Article 17(2)(a) for the following reasons:

1. ☑  Claims Nos.: **8-9**
   because they relate to subject matter not required to be searched by this Authority, namely:

   Claims 8-9 relate to a method for surgery or therapy of a living human or animal body. The subject matter of claims 8-9 is reasonably expected to be "the use of the mycobacterium of claim 1 or the microbial agent of claim 2 in nitrogen pollution treatment, carbon capture, screening of agricultural chemicals, and preparation of a drug or preparation for treating and/or preventing skin diseases, a drug or preparation for treating and/or preventing a disease associated with a low nitrite level, a drug or preparation for treating and/or preventing body odor, a drug or preparation for the treatment of supplying nitric oxide to a subject, or a drug or preparation for inhibiting microbial growth.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

### INTERNATIONAL SEARCH REPORT
Information on patent family members

International application No.

**PCT/CN2023/113637**

| Patent document cited in search report | | | Publication date (day/month/year) | Patent family member(s) | | | Publication date (day/month/year) |
|---|---|---|---|---|---|---|---|
| CN | 108285881 | A | 17 July 2018 | None | | | |
| CN | 108220186 | A | 29 June 2018 | None | | | |
| KR | 20070111916 | A | 22 November 2007 | KR | 101195900 | B1 | 30 October 2012 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210989249
- CN 03118598

- WO 2009018686 A1

**Non-patent literature cited in the description**

- **MARKUS RIBBE** ; **DILIP GADKARI** ; **ORTWIN MEYER**. N2 Fixation by Streptomyces thermoautotrophicus Involves a Molybdenum-Dinitrogenase and a Manganese-Superoxide Oxidoreductase That Couple N2 Reduction to the Oxidation of Superoxide Produced from O2 by a Molybdenum-CO Dehydrogenase. *The Journal of Biological Chemistry*, 1997, vol. 272 (42), 26627-26633
- **MARCEL M. M. KUYPERS** ; **HANNAH K. MARCHANT** ; **BORAN KARTA**. The microbial nitrogen-cycling network. *Nature*, 05 February 2018
- **AINA SOLER-JOFRA** ; **JULIO PÉREZ** ; **MARK C.M. VAN LOOSDRECHT**. Hydroxylamine and the nitrogen cycle: A review. *Water Research*, 2021, vol. 190, 116723, https://doi.org/10.1016/j.watres.2020.116723
- **HOLGER DAIMS** ; **ELENA V. LEBEDEVA** ; **PETRA PJEVAC** ; **PING HAN** ; **CRAIG HERBOLD et al.** Complete nitrification by Nitrospira bacteria. *Nature*, 2015, vol. 528
- **ZHANG MIAO-MIAO** ; **SHEN JU-PEI** ; **HE JI-ZHENG** ; **ZHANG LI-MEI**. Microbial Mechanisms of Nitrification Inhibitors and Their Application. *Journal of Agro-Environment Science*, 2014, vol. 33 (11), 2077-2083

- **ZHANG HAO** ; **ZAN JIN-XING** ; **LIU PEI-XUN**. Research Progress on Inducible Nitric Oxide Synthase Inhibitors. *CHINESE JOURNAL OF NEW DRUGS*, 2012, vol. 22 (6), 665-669
- **LUO HUI-TING** ; **KONG BAO-HUA** ; **LI PEI-JUN** ; **LI MU-ZI**. Bacterial Nitric Oxide Synthase and Its Application in Food Science: a Review. *FOOD SCIENCE*, 2014, vol. 35 (17), 266-271
- **ZHANG YANG** ; **LI YA-YING** ; **ZHENG NING-GUO** ; **YAO HUAI-YING**. The Inhibition Principle of Biological Nitrification Inhibitors and the Research Progress. *JIANGSU AGRICULTURE SCIENCES*, 2019, vol. 47 (1), 21-26
- **ZHU ZHAO-LIANG** ; **WEN QI-XIAO**. Nitrogen in Chinese Soil. Nanjing: Jiangsu Science and Technology Press, 1992
- **SHENG TONG** ; **WANG JING-YAN**. Biochemistry. Beijing: Higher Education Press, 2000
- **HAN BIN** ; **KONG JI-JUN** ; **ZOU XIAO-MING** ; **KONG HE-DE**. Current Status and Prospect of Biological Nitrogen Fixation Research. *Journal of Shanxi Agriculture Sciences*, 2009, vol. 37 (10), 86-89, 85